# EUROPEAN PATENT APPLICATION

(11) **EP 4 223 302 A1**
(43) Date of publication of application: **09.08.2023**
(21) Application number: 22155744.0
(22) Date of filing: 08.02.2022
(51) Int. Cl.: A61K 35/768, A61P 35/00, C07K 16/28, C07K 16/30, C12N 15/86, A61K 39/395

(54) **ONCOLYTIC MEASLES VIRUS ENCODING A BIKE AND METHODS OF USE THEREOF IN TREATING CANCER**

(71) Applicant: Universität Heidelberg, 69117 Heidelberg (DE)
(72) Inventor: ENGELAND, Christine, Heidelberg (DE); FLOERCHINGER, Alessia, 67374 Hanhofen (DE); KLEIN, Jessica, Heidelberg (DE); UNGERECHTS, Guy, Heidelberg (DE)
(74) Representative: Thomann, William John

(57) **Abstract**

The present invention relates to a bispecific killer engager (BiKE) which binds to a tumor antigen and binds to immune cells, such as NK cells, and its use in promoting anti-tumor cytotoxicity for treating cancer/tumors. In particular, the BiKE can be encoded within the genome of an oncolytic virus of the family *Paramyxoviridae.*

SEQUENCE LISTING

<110>  Universität Heidelberg

<120>  Oncolytic Measles Virus Encoding a BiKE and Methods of Use
       Thereof in Treating Cancer

<130>  789-39 EP

<160>  8

<170>  PatentIn version 3.5

<210>  1
<211>  254
<212>  PRT
<213>  Homo sapiens

<400>  1

Met Trp Gln Leu Leu Leu Pro Thr Ala Leu Leu Leu Leu Val Ser Ala
1               5                   10                  15

Gly Met Arg Thr Glu Asp Leu Pro Lys Ala Val Val Phe Leu Glu Pro
            20                  25                  30

Gln Trp Tyr Arg Val Leu Glu Lys Asp Ser Val Thr Leu Lys Cys Gln
        35                  40                  45

Gly Ala Tyr Ser Pro Glu Asp Asn Ser Thr Gln Trp Phe His Asn Glu
    50                  55                  60

Ser Leu Ile Ser Ser Gln Ala Ser Ser Tyr Phe Ile Asp Ala Ala Thr
65                  70                  75                  80

Val Asp Asp Ser Gly Glu Tyr Arg Cys Gln Thr Asn Leu Ser Thr Leu
                85                  90                  95

Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
        115                 120                 125

His Ser Trp Lys Asn Thr Ala Leu His Lys Val Thr Tyr Leu Gln Asn
    130                 135                 140

Gly Lys Gly Arg Lys Tyr Phe His His Asn Ser Asp Phe Tyr Ile Pro
145                 150                 155                 160

Lys Ala Thr Leu Lys Asp Ser Gly Ser Tyr Phe Cys Arg Gly Leu Phe
                165                 170                 175

Gly Ser Lys Asn Val Ser Ser Glu Thr Val Asn Ile Thr Ile Thr Gln
            180                 185                 190

Gly Leu Ala Val Ser Thr Ile Ser Ser Phe Phe Pro Pro Gly Tyr Gln
        195                 200                 205

Val Ser Phe Cys Leu Val Met Val Leu Leu Phe Ala Val Asp Thr Gly
    210                 215                 220

Leu Tyr Phe Ser Val Lys Thr Asn Ile Arg Ser Ser Thr Arg Asp Trp
225                 230                 235                 240

Lys Asp His Lys Phe Lys Trp Arg Lys Asp Pro Gln Asp Lys
                245                 250

<210> 2
<211> 702
<212> PRT
<213> Homo sapiens

<400> 2

Met Glu Ser Pro Ser Ala Pro Pro His Arg Trp Cys Ile Pro Trp Gln
1               5                   10                  15

Arg Leu Leu Leu Thr Ala Ser Leu Leu Thr Phe Trp Asn Pro Pro Thr
            20                  25                  30

Thr Ala Lys Leu Thr Ile Glu Ser Thr Pro Phe Asn Val Ala Glu Gly
        35                  40                  45

Lys Glu Val Leu Leu Leu Val His Asn Leu Pro Gln His Leu Phe Gly
    50                  55                  60

Tyr Ser Trp Tyr Lys Gly Glu Arg Val Asp Gly Asn Arg Gln Ile Ile
65                  70                  75                  80

Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240

Thr Ile Ser Pro Leu Asn Thr Ser Tyr Arg Ser Gly Glu Asn Leu Asn
                245                 250                 255

Leu Ser Cys His Ala Ala Ser Asn Pro Pro Ala Gln Tyr Ser Trp Phe
            260                 265                 270

Val Asn Gly Thr Phe Gln Gln Ser Thr Gln Glu Leu Phe Ile Pro Asn
        275                 280                 285

Ile Thr Val Asn Asn Ser Gly Ser Tyr Thr Cys Gln Ala His Asn Ser
    290                 295                 300

Asp Thr Gly Leu Asn Arg Thr Thr Val Thr Thr Ile Thr Val Tyr Ala
305                 310                 315                 320

Glu Pro Pro Lys Pro Phe Ile Thr Ser Asn Asn Ser Asn Pro Val Glu
                325                 330                 335

Asp Glu Asp Ala Val Ala Leu Thr Cys Glu Pro Glu Ile Gln Asn Thr
            340                 345                 350

Thr Tyr Leu Trp Trp Val Asn Asn Gln Ser Leu Pro Val Ser Pro Arg
        355                 360                 365

Leu Gln Leu Ser Asn Asp Asn Arg Thr Leu Thr Leu Leu Ser Val Thr
    370                 375                 380

Arg Asn Asp Val Gly Pro Tyr Glu Cys Gly Ile Gln Asn Lys Leu Ser
385                 390                 395                 400

Val Asp His Ser Asp Pro Val Ile Leu Asn Val Leu Tyr Gly Pro Asp
                405                 410                 415

Asp Pro Thr Ile Ser Pro Ser Tyr Thr Tyr Tyr Arg Pro Gly Val Asn
            420                 425                 430

Leu Ser Leu Ser Cys His Ala Ala Ser Asn Pro Pro Ala Gln Tyr Ser
        435                 440                 445

Trp Leu Ile Asp Gly Asn Ile Gln Gln His Thr Gln Glu Leu Phe Ile
    450                 455                 460

Ser Asn Ile Thr Glu Lys Asn Ser Gly Leu Tyr Thr Cys Gln Ala Asn
465                 470                 475                 480

Asn Ser Ala Ser Gly His Ser Arg Thr Thr Val Lys Thr Ile Thr Val
                485                 490                 495

Ser Ala Glu Leu Pro Lys Pro Ser Ile Ser Ser Asn Asn Ser Lys Pro
            500                 505                 510

Val Glu Asp Lys Asp Ala Val Ala Phe Thr Cys Glu Pro Glu Ala Gln
        515                 520                 525

Asn Thr Thr Tyr Leu Trp Trp Val Asn Gly Gln Ser Leu Pro Val Ser
    530                 535                 540

Pro Arg Leu Gln Leu Ser Asn Gly Asn Arg Thr Leu Thr Leu Phe Asn
545                 550                 555                 560

Val Thr Arg Asn Asp Ala Arg Ala Tyr Val Cys Gly Ile Gln Asn Ser
                565                 570                 575

Val Ser Ala Asn Arg Ser Asp Pro Val Thr Leu Asp Val Leu Tyr Gly
            580                 585                 590

Pro Asp Thr Pro Ile Ile Ser Pro Pro Asp Ser Ser Tyr Leu Ser Gly
        595                 600                 605

Ala Asn Leu Asn Leu Ser Cys His Ser Ala Ser Asn Pro Ser Pro Gln
    610                 615                 620

Tyr Ser Trp Arg Ile Asn Gly Ile Pro Gln Gln His Thr Gln Val Leu
625                 630                 635                 640

Phe Ile Ala Lys Ile Thr Pro Asn Asn Asn Gly Thr Tyr Ala Cys Phe

645                 650                 655

Val Ser Asn Leu Ala Thr Gly Arg Asn Asn Ser Ile Val Lys Ser Ile
            660                 665                 670

Thr Val Ser Ala Ser Gly Thr Ser Pro Gly Leu Ser Ala Gly Ala Thr
        675                 680                 685

Val Gly Ile Met Ile Gly Val Leu Val Gly Val Ala Leu Ile
    690                 695                 700

<210>  3
<211>  543
<212>  PRT
<213>  Artificial Sequence

<220>
<223>  CEAxhCD16a

<400>  3

Met Glu Thr Asp Thr Leu Leu Leu Trp Val Leu Leu Leu Trp Val Pro
1               5                   10                  15

Gly Ser Thr Gly Asp Tyr Pro Tyr Asp Val Pro Asp Tyr Ala Gly Ala
            20                  25                  30

Gln Val Lys Leu Glu Gln Ser Gly Ala Glu Val Val Lys Pro Gly Ala
        35                  40                  45

Ser Val Lys Leu Ser Cys Lys Ala Ser Gly Phe Asn Ile Lys Asp Ser
    50                  55                  60

Tyr Met His Trp Leu Arg Gln Gly Pro Gly Gln Cys Leu Glu Trp Ile
65                  70                  75                  80

Gly Trp Ile Asp Pro Glu Asn Gly Asp Thr Glu Tyr Ala Pro Lys Phe
                85                  90                  95

Gln Gly Lys Ala Thr Phe Thr Thr Asp Thr Ser Ala Asn Thr Ala Tyr
            100                 105                 110

Leu Gly Leu Ser Ser Leu Arg Pro Glu Asp Thr Ala Val Tyr Tyr Cys
        115                 120                 125

Asn Glu Gly Thr Pro Thr Gly Pro Tyr Tyr Phe Asp Tyr Trp Gly Gln
    130                 135                 140

Gly Thr Leu Val Thr Val Ser Ser Gly Gly Gly Gly Ser Gly Gly Gly
145                 150                 155                 160

Gly Ser Gly Gly Gly Gly Ser Glu Asn Val Leu Thr Gln Ser Pro Ser
                165                 170                 175

Ser Met Ser Val Ser Val Gly Asp Arg Val Thr Ile Ala Cys Ser Ala
            180                 185                 190

Ser Ser Ser Val Pro Tyr Met His Trp Leu Gln Gln Lys Pro Gly Lys
        195                 200                 205

Ser Pro Lys Leu Leu Ile Tyr Leu Thr Ser Asn Leu Ala Ser Gly Val
    210                 215                 220

Pro Ser Arg Phe Ser Gly Ser Gly Ser Gly Thr Asp Tyr Ser Leu Thr
225                 230                 235                 240

Ile Ser Ser Val Gln Pro Glu Asp Ala Ala Thr Tyr Tyr Cys Gln Gln
                245                 250                 255

Arg Ser Ser Tyr Pro Leu Thr Phe Gly Cys Gly Thr Lys Leu Glu Ile
            260                 265                 270

Lys Ala Ala Ala Pro Ser Gly Gln Ala Gly Ala Ala Ala Ser Glu Ser
        275                 280                 285

Leu Phe Val Ser Asn His Ala Tyr Gln Val Gln Leu Val Gln Ser Gly
    290                 295                 300

Ala Glu Val Lys Lys Pro Gly Glu Ser Leu Lys Val Ser Cys Lys Ala
305                 310                 315                 320

Ser Gly Tyr Thr Phe Thr Ser Tyr Tyr Met His Trp Val Arg Gln Ala
                325                 330                 335

Pro Gly Gln Gly Leu Glu Trp Met Gly Ile Ile Asn Pro Ser Gly Gly
            340                 345                 350

Ser Thr Ser Tyr Ala Gln Lys Phe Gln Gly Arg Val Thr Met Thr Arg
        355                 360                 365

Asp Thr Ser Thr Ser Thr Val Tyr Met Glu Leu Ser Ser Leu Arg Ser
    370                 375                 380

Glu Asp Thr Ala Val Tyr Tyr Cys Ala Arg Gly Ser Ala Tyr Tyr Tyr
385                 390                 395                 400

Asp Phe Ala Asp Tyr Trp Gly Gln Gly Thr Leu Val Thr Val Ser Ser

405                 410                 415

Gly Gly Gly Gly Ser Gly Gly Gly Gly Ser Gly Gly Gly Gly Ser Ser
            420                 425                 430

Tyr Val Leu Thr Gln Pro Ser Ser Val Ser Val Ala Pro Gly Gln Thr
        435                 440                 445

Ala Thr Ile Ser Cys Gly Gly His Asn Ile Gly Ser Lys Asn Val His
    450                 455                 460

Trp Tyr Gln Gln Arg Pro Gly Gln Ser Pro Val Leu Val Ile Tyr Gln
465                 470                 475                 480

Asp Asn Lys Arg Pro Ser Gly Ile Pro Glu Arg Phe Ser Gly Ser Asn
                485                 490                 495

Ser Gly Asn Thr Ala Thr Leu Thr Ile Ser Gly Thr Gln Ala Met Asp
            500                 505                 510

Glu Ala Asp Tyr Tyr Cys Gln Val Trp Asp Asn Tyr Ser Val Leu Phe
        515                 520                 525

Gly Gly Gly Thr Lys Leu Thr Val Leu His His His His His His
    530                 535                 540

<210>  4
<211>  1632
<212>  DNA
<213>  Artificial Sequence

<220>
<223>  CEAxhCD16a nt

<400>  4
atggagacag acacactcct gctatgggta ctgctgctct gggttccagg ttccactggt      60

gactatccat atgatgttcc agattatgct ggcgcccagg ttaagctgga acagagtggc     120

gcagaagtgg tcaaacctgg agcaagcgtc aagctgtcct gcaaagcctc tggcttcaac     180

atcaaggaca gctacatgca ctggcttagg caaggaccag gtcagtgtct ggagtggatt     240

gggtggatag accctgagaa tggcgatacc gagtatgctc ccaagtttca ggggaaagcc     300

acgtttacaa ccgatactag cgctaacacc gcgtatctgg gcttgtcctc tctcagaccc     360

gaagatacag ccgtgtatta ctgcaatgag gggactccaa caggaccgta ctacttcgac     420

tattggggtc aaggcactct cgtaaccgtg tcatcaggtg gaggcggttc aggcggaggt     480

ggctctggcg gtggcggatc agagaatgtg ctgacacagt ctccttccag catgtcagtg     540

tccgtagggg acagagtcac gattgcgtgc tcagcaagca gctctgtgcc ctatatgcac     600

tggttgcagc agaaaccagg caagagtccg aaactcctga tctacctgac ttccaacctt 660

gcctcaggtg ttccatctcg gttcagtgga agcggaagtg gcactgacta ttccctcaca 720

atcagctctg tccaacccga agatgctgcc acctactact gccaacagag gagctcctat 780

cctctgacct ttggctgtgg gaccaagctg gagataaagg cggccgcacc ttctggtcaa 840

gctggtgctg ctgcttctga atctttattt gtttctaatc atgcttatca ggtacagctt 900

gtgcagagtg gggcagaagt gaagaaaccc ggtgagagcc tcaaggtttc atgcaaagcc 960

tctgggtaca cattcacctc atactacatg cactgggtta ggcaagcacc aggccaaggc 1020

ttggagtgga tgggcattat caatcccagc ggagggtcaa ccagttatgc ccagaagttc 1080

cagggaagag tcacaatgac tcgcgatacg tctaccagta ccgtgtacat ggaactgagc 1140

tccttgagga gcgaggatac cgctgtgtac tattgtgcca gaggtagtgc ctactactac 1200

gattttgccg actattgggg tcaaggaaca ctggtgactg tctcctctgg aggcggaggg 1260

tctggcggcg gaggtagcgg aggtggcggg tcatcctatg tgcttacaca gccgagtagc 1320

gtgagcgtag ctcctggcca aactgcgact atctcctgtg gcgggcataa cattggaagc 1380

aagaatgtcc actggtatca gcagcgtcct ggccagtctc cagtgctggt catctaccag 1440

gacaacaaac gacctagcgg gatacccgaa cggttctcag ggtccaactc cggaaatacg 1500

gcaactctga ccatttctgg cacacaggct atggatgagg ctgactatta ttgccaggtt 1560

tgggacaact attccgtcct gtttggtggc gggacaaagc tgaccgtgct ccaccaccat 1620

catcaccact ag 1632

<210> 5
<211> 21333
<212> DNA
<213> Artificial Sequence

<220>
<223> pcMeVac P-CEAxhCD16a BiKE

<400> 5
accaaacaaa gttgggtaag gatagttcaa tcaatgatca tcttctagtg cacttaggat 60

tcaagatcct attatcaggg acaagagcag gattagggat atccgagatg gccacacttt 120

taaggagctt agcattgttc aaaagaaaca aggacaaacc acccattaca tcaggatccg 180

gtggagccat cagaggaatc aaacacatta ttatagtacc aatccctgga gattcctcaa 240

ttaccactcg atccagactt ctggaccggt tggtgaggtt aattggaaac ccggatgtga 300

gcgggcccaa actaacaggg gcactaatag gtatattatc cttatttgtg gagtctccag 360

gtcaattgat tcagaggatc accgatgacc ctgacgttag cataaggctg ttagaggttg 420

tccagagtga ccagtcacaa tctggcctta ccttcgcatc aagaggtacc aacatggagg 480

atgaggcgga ccaatacttt tcacatgatg atccaattag tagtgatcaa tccaggttcg 540
gatggttcgg gaacaaggaa atctcagata ttgaagtgca agaccctgag ggattcaaca 600
tgattctggg taccatccta gcccaaattt gggtcttgct cgcaaaggcg gttacggccc 660
cagacacggc agctgattcg gagctaagaa ggtggataaa gtacacccaa caaagaaggg 720
tagttggtga atttagattg gagagaaaat ggttggatgt ggtgaggaac aggattgccg 780
aggacctctc cttacgccga ttcatggtcg ctctaatcct ggatatcaag agaacacccg 840
gaaacaaacc caggattgct gaaatgatat gtgacattga tacatatatc gtagaggcag 900
gattagccag ttttatcctg actattaagt ttgggataga aactatgtat cctgctcttg 960
gactgcatga atttgctggt gagttatcca cacttgagtc cttgatgaac ctttaccagc 1020
aaatggggga aactgcaccc tacatggtaa tcctggagaa ctcaattcag aacaagttca 1080
gtgcaggatc ataccctctg ctctggagct atgccatggg agtaggagtg gaacttgaaa 1140
actccatggg aggtttgaac tttggccgat cttactttga tccagcatat tttagattag 1200
ggcaagagat ggtaaggagg tcagctggaa aggtcagttc cacattggca tctgaactcg 1260
gtatcactgc cgaggatgca aggcttgttt cagagattgc aatgcatact actgaggaca 1320
agatcagtag agcggttgga cccagacaag cccaagtatc atttctacac ggtgatcaaa 1380
gtgagaatga gctaccgaga ttggggggca aggaagatag gagggtcaaa cagagtcgag 1440
gagaagccag ggagagctac agagaaaccg ggcccagcag agcaagtgat gcgagagctg 1500
cccatcttcc aaccggcaca cccctagaca ttgacactgc aacggagtcc agccaagatc 1560
cgcaggacag tcgaaggtca gctgacgccc tgcttaggct gcaagccatg gcaggaatct 1620
cggaagaaca aggctcagac acggacaccc ctatagtgta caatgacaga aatcttctag 1680
actaggtgcg agaggccgag ggccagaaca acatccgcct accatccatc attgttataa 1740
aaaacttagg aaccaggtcc acacagccgc cagcccatca accatccact cccacgattg 1800
gagccaatgg cagaagagca ggcacgccat gtcaaaaacg gactggaatg catccgggct 1860
ctcaaggccg agcccatcgg ctcactggcc atcgaggaag ctatggcagc atggtcagaa 1920
atatcagaca acccaggaca ggagcgagcc acctgcaggg aagagaaggc aggcagttcg 1980
ggtctcagca aaccatgcct ctcagcaatt ggatcaactg aaggcggtgc acctcgcatc 2040
cgcggtcagg gacctggaga gagcgatgac gacgctgaaa ctttgggaat ccccccaaga 2100
aatctccagg catcaagcac tgggttacag tgttattacg tttatgatca cagcggtgaa 2160
gcggttaagg gaatccaaga tgctgactct atcatggttc aatcaggcct tgatggtgat 2220
agcaccctct caggaggaga caatgaatct gaaaacagcg atgtggatat tggcgaacct 2280
gataccgagg gatatgctat cactgaccgg ggatctgctc ccatctctat ggggttcagg 2340
gcttctgatg ttgaaactgc agaaggaggg gagatccacg agctcctgag actccaatcc 2400

agaggcaaca actttccgaa gcttgggaaa actctcaatg ttcctccgcc cccggacccc 2460

ggtagggcca gcacttccgg gacacccatt aaaaagggca cagacgcgag attagcctca 2520

tttggaacgg agatcgcgtc tttattgaca ggtggtgcaa cccaatgtgc tcgaaagtca 2580

ccctcggaac catcagggcc aggtgcacct gcggggaatg tccccgagtg tgtgagcaat 2640

gccgcactga tacaggagtg gacacccgaa tctggtacca caatctcccc gagatcccag 2700

aataatgaag aagggggaga ctattatgat gatgagctgt tctctgatgt ccaagatatt 2760

aaaacagcct tggccaaaat acacgaggat aatcagaaga taatctccaa gctagaatca 2820

ctgctgttat tgaagggaga agttgagtca attaagaagc agatcaacag gcaaaatatc 2880

agcatatcca ccctggaagg acacctctca agcatcatga tcgccattcc tggacttggg 2940

aaggatccca acgaccccac tgcagatgtc gaaatcaatc ccgacttgaa acccatcata 3000

ggcagagatt caggccgagc actggccgaa gttctcaaga aacccgttgc cagccgacaa 3060

ctccaaggaa tgacaaatgg acggaccagt tccagaggac agctgctgaa ggaatttcag 3120

ctaaagccga tcgggaaaaa gatgagctca gccgtcgggt ttgttcctga caccggccct 3180

gcatcacgca gtgtaatccg ctccattata aaatccagcc ggctagagga ggatcggaag 3240

cgttacctga tgactctcct tgatgatatc aaaggagcca atgatcttgc caagttccac 3300

cagatgctga tgaagataat aatgaagtag ctacagctca acttacctgc caaccccatg 3360

ccagtcgacc caactagcaa cctaaatcca ttataaaaaa cttaggaacc aggtccacac 3420

agctcgagtc gcgcgtgcca ccatggagac agacacactc ctgctatggg tactgctgct 3480

ctgggttcca ggttccactg gtgactatcc atatgatgtt ccagattatg ctggcgccca 3540

ggttaagctg gaacagagtg gcgcagaagt ggtcaaacct ggagcaagcg tcaagctgtc 3600

ctgcaaagcc tctggcttca acatcaagga cagctacatg cactggctta ggcaaggacc 3660

aggtcagtgt ctggagtgga ttgggtggat agaccctgag aatggcgata ccgagtatgc 3720

tcccaagttt caggggaaag ccacgtttac aaccgatact agcgctaaca ccgcgtatct 3780

gggcttgtcc tctctcagac ccgaagatac agccgtgtat tactgcaatg aggggactcc 3840

aacaggaccg tactacttcg actattgggg tcaaggcact ctcgtaaccg tgtcatcagg 3900

tggaggcggt tcaggcggag gtggctctgg cggtggcgga tcagagaatg tgctgacaca 3960

gtctccttcc agcatgtcag tgtccgtagg ggacagagtc acgattgcgt gctcagcaag 4020

cagctctgtg ccctatatgc actggttgca gcagaaacca ggcaagagtc cgaaactcct 4080

gatctacctg acttccaacc ttgcctcagg tgttccatct cggttcagtg gaagcggaag 4140

tggcactgac tattccctca caatcagctc tgtccaaccc gaagatgctg ccacctacta 4200

ctgccaacag aggagctcct atcctctgac ctttggctgt gggaccaagc tggagataaa 4260

ggcggccgca ccttctggtc aagctggtgc tgctgcttct gaatctttat ttgtttctaa 4320

tcatgcttat caggtacagc ttgtgcagag tggggcagaa gtgaagaaac ccggtgagag 4380

cctcaaggtt tcatgcaaag cctctgggta cacattcacc tcatactaca tgcactgggt 4440

taggcaagca ccaggccaag gcttggagtg gatgggcatt atcaatccca gcggagggtc 4500

aaccagttat gcccagaagt tccaggaaag agtcacaatg actcgcgata cgtctaccag 4560

taccgtgtac atggaactga gctccttgag gagcgaggat accgctgtgt actattgtgc 4620

cagaggtagt gcctactact acgattttgc cgactattgg ggtcaaggaa cactggtgac 4680

tgtctcctct ggaggcggag ggtctggcgg cggaggtagc ggaggtggcg ggtcatccta 4740

tgtgcttaca cagccgagta gcgtgagcgt agctcctggc caaactgcga ctatctcctg 4800

tggcgggcat aacattggaa gcaagaatgt ccactggtat cagcagcgtc ctggccagtc 4860

tccagtgctg gtcatctacc aggacaacaa acgacctagc gggatacccg aacggttctc 4920

agggtccaac tccggaaata cggcaactct gaccatttct ggcacacagg ctatggatga 4980

ggctgactat tattgccagg tttgggacaa ctattccgtc ctgtttggtg gcgggacaaa 5040

gctgaccgtg ctccaccacc atcatcacca ctaggcgcgc gtgctagtac aacctaaatc 5100

cattataaaa aacttaggag caaagtgatt gcctcccaag gtccacaatg acagagacct 5160

acgacttcga caagtcggca tgggacatca aagggtcgat cgctccgata caacccacca 5220

cctacagtga tggcaggctg gtgccccagg tcagagtcat agatcctggt ctaggcgaca 5280

ggaaggatga atgctttatg tacatgtttc tgctgggggt tgttgaggac agcgattccc 5340

tagggcctcc aatcgggcga gcatttgggt tcctgccctt aggtgttggc agatccacag 5400

caaagcccga aaaactcctc aaagaggcca ctgagcttga catagttgtt agacgtacag 5460

cagggctcaa tgaaaaactg gtgttctaca acaacacccc actaactctc ctcacacctt 5520

ggagaaaggt cctaacaaca gggagtgtct tcaacgcaaa ccaagtgtgc aatgcggtta 5580

atctgatacc gctcgatacc ccgcagaggt tccgtgttgt ttatatgagc atcacccgtc 5640

tttcggataa cgggtattac accgttccta gaagaatgct ggaattcaga tcggtcaatg 5700

cagtggcctt caacctgctg gtgaccctta ggattgacaa ggcgataggc cctgggaaga 5760

tcatcgacaa tacagagcaa cttcctgagg caacatttat ggtccacatc gggaacttca 5820

ggagaaagaa gagtgaagtc tactctgccg attattgcaa aatgaaaatc gaaaagatgg 5880

gcctggtttt tgcacttggt gggatagggg gcaccagtct tcacattaga agcacaggca 5940

aaatgagcaa gactctccat gcacaactcg ggttcaagaa gaccttatgt tacccgctga 6000

tggatatcaa tgaagacctt aatcgattac tctggaggag cagatgcaag atagtaagaa 6060

tccaggcagt tttgcagcca tcagttcctc aagaattccg catttacgac gacgtgatca 6120

taaatgatga ccaaggacta ttcaaagttc tgtagaccgt agtgcccagc aatgcccgaa 6180

aacgaccccc ctcacaatga cagccagaag gcccggacaa aaaagccccc tccgaaagac 6240

tccacggacc aagcgagagg ccagccagca gccgacggca agcgcgaaca ccaggcggcc 6300

ccagcacaga acagccctga cacaaggcca ccaccagcca ccccaatctg catcctcctc 6360

gtgggacccc cgaggaccaa cccccaaggc tgcccccgat ccaaaccacc aaccgcatcc 6420

ccaccacccc cgggaaagaa acccccagca attggaaggc ccctccccct cttcctcaac 6480

acaagaactc cacaaccgaa ccgcacaagc gaccgaggtg acccaaccgc aggcatccga 6540

ctccctagac agatcctctc tccccggcaa actaaacaaa acttagggcc aaggaacata 6600

cacacccaac agaacccaga ccccggccca cggcgccgcg cccccaaccc ccgacaacca 6660

gagggagccc ccaaccaatc ccgccggctc ccccggtgcc cacaggcagg gacaccaacc 6720

cccgaacaga cccagcaccc aaccatcgac aatccaagac gggggggccc ccccaaaaaa 6780

aggcccccag gggccgacag ccagcaccgc gaggaagccc acccacccca cacacgacca 6840

cggcaaccaa accagaaccc agaccaccct gggccaccag ctcccagact cggccatcac 6900

cccgcagaaa ggaaaggcca caacccgcgc accccagccc cgatccggcg gggagccacc 6960

caacccgaac cagcacccaa gagcgatccc cgaaggaccc ccgaaccgca aaggacatca 7020

gtatcccaca gcctctccaa gtcccccggt ctcctcctct tctcgaaggg accaaaagat 7080

caatccacca cacccgacga cactcaactc cccaccccta aaggagacac cgggaatccc 7140

agaatcaaga ctcatccaat gtccatcatg ggtctcaagg tgaacgtctc tgccatattc 7200

atggcagtac tgttaactct ccaaacaccc accggtcaaa tccattgggg caatctctct 7260

aagatagggg tggtaggaat aggaagtgca agctacaaag ttatgactcg ttccagccat 7320

caatcattag tcataaaatt aatgcccaat ataactctcc tcaataactg cacgaggtta 7380

gagattgcag aatacaggag actactgaga acagttttgg aaccaattag agatgcactt 7440

aatgcaatga cccagaatat aagaccggtt cagagtgtag cttcaagtag gagacacaag 7500

agatttgcgg gagtagtcct ggcaggtgcg gccctaggcg ttgccacagc tgctcagata 7560

acagccggca ttgcacttca ccagtccatg ctgaactctc aagccatcga caatctgaga 7620

gcgagcctgg aaactactaa tcaggcaatt gagacaatca gacaagcagg gcaggagatg 7680

atattggctg ttcagggtgt ccaagactac atcaataatg agctgatacc gtctatgaac 7740

caactatctt gtgatttaat cggccagaag ctcgggctca aattgctcag atactataca 7800

gaaatcctgt cattatttgg ccccagttta cgggacccca tatctgcgga gatatctatc 7860

caggctttga gctatgcgct tggaggagac atcaataagg tgttagaaaa gctcggatac 7920

agtggaggtg atttactggg catcttagag agcggaggaa taaaggcccg gataactcac 7980

gtcgacacag agtcctactt cattgtcctc agtatagcct atccgacgct gtccgagatt 8040

aagggggtga ttgtccaccg gctagagggg gtctcgtaca acataggctc tcaagagtgg 8100

tataccactg tgcccaagta tgttgcaacc caagggtacc ttatctcgaa ttttgatgag 8160

tcatcgtgta ctttcatgcc agaggggact gtgtgcagcc aaaatgcctt gtacccgatg 8220

agtcctctgc tccaagaatg cctccggggg tacaccaagt cctgtgctcg tacactcgta 8280

tccgggtctt ttgggaaccg gttcatttta tcacaaggga acctaatagc caattgtgca 8340

tcaatccttt gcaagtgtta cacaacagga acgatcatta atcaagaccc tgacaagatc 8400

ctaacataca ttgctgccga tcactgcccg gtagtcgagg tgaacggcgt gaccatccaa 8460

gtcgggagca ggaggtatcc agacgctgtg tacttgcaca gaattgacct cggtcctccc 8520

atatcattgg agaggttgga cgtagggaca aatctgggga atgcaattgc taagttggag 8580

gatgccaagg aattgttgga gtcatcggac cagatattga ggagtatgaa aggtttatcg 8640

agcactagca tagtctacat cctgattgca gtgtgtcttg gagggttgat agggatcccc 8700

gctttaatat gttgctgcag ggggcgttgt aacaaaaagg gagaacaagt tggtatgtca 8760

agaccaggcc taaagcctga tcttacggga acatcaaaat cctatgtaag gtcgctctga 8820

tcctctacaa ctcttgaaac acaaatgtcc cacaagtctc ctcttcgtca tcaagcaacc 8880

accgcaccca gcatcaagcc cacctgaaat tatctccggc ttccctctgg ccgaacaata 8940

tcggtagtta atcaaaactt agggtgcaag atcatccaca atgtcaccac aacgagaccg 9000

gataaatgcc ttctacaaag ataaccccca tcccaaggga agtaggatag tcattaacag 9060

agaacatctt atgattgata gaccttatgt tttgctggct gttctgtttg tcatgtttct 9120

gagcttgatc gggttgctag ccattgcagg cattagactt catcgggcag ccatctacac 9180

cgcagagatc cataaaagcc tcagcaccaa tctagatgta actaactcaa tcgagcatca 9240

ggtcaaggac gtgctgacac cactcttcaa aatcatcggt gatgaagtgg gcctgaggac 9300

acctcagaga ttcactgacc tagtgaaatt aatctctgac aagattaaat tccttaatcc 9360

ggatagggag tacgacttca gagatctcac ttggtgtatc aacccgccag agagaatcaa 9420

attggattat gatcaatact gtgcagatgt ggctgctgaa gagctcatga atgcattggt 9480

gaactcaact ctactggaga ccagaacaac caatcagttc ctagctgtct caaagggaaa 9540

ctgctcaggg cccactacaa tcagaggtca attctcaaac atgtcgctgt ccctgttaga 9600

cttgtattta ggtcgaggtt acaatgtgtc atctatagtc actatgacat cccagggaat 9660

gtatggggga acttacctag tggaaaagcc taatctgagc agcaaaaggt cagagttgtc 9720

acaactgagc atgtaccgag tgtttgaagt aggtgttatc agaaatccgg gtttgggggc 9780

tccggtgttc catatgacaa actatcttga gcaaccagtc agtaatgatc tcagcaactg 9840

tatggtggct ttgggggagc tcaaactcgc agccctttgt cacggggaag attctatcac 9900

aattccctat cagggatcag ggaaaggtgt cagcttccag ctcgtcaagc taggtgtctg 9960

gaaatcccca accgacatgc aatcctgggt ccccttatca acggatgatc cagtgataga 10020

caggctttac ctctcatctc acagaggtgt tatcgctgac aatcaagcaa aatgggctgt 10080

cccgacaaca cgaacagatg acaagttgcg aatggagaca tgcttccaac aggcgtgtaa 10140

gggtaaaatc caagcactct gcgagaatcc cgagtgggca ccattgaagg ataacaggat 10200

tccttcatac ggggtcttgt ctgttgatct gagtctgaca gttgagctta aaatcaaaat 10260

tgcttcggga ttcgggccat tgatcacaca cggttcaggg atggacctat acaaatccaa 10320

ccacaacaat gtgtattggc tgactatccc gccaatgaag aacctagcct taggtgtaat 10380

caacacattg gagtggatac cgagattcaa ggttagtccc tacctcttca ctgtcccaat 10440

taaggaagca ggcgaagact gccatgcccc aacataccta cctgcggagg tggatggtga 10500

tgtcaaactc agttccaatc tggtgattct acctggtcaa gatctccaat atgttttggc 10560

aacctacgat acttccaggg ttgaacatgc tgtggtttat tacgtttaca gcccaagccg 10620

ctcattttct tacttttatc cttttaggtt gcctataaag ggggtcccca tcgaattaca 10680

agtggaatgc ttcacatggg accaaaaact ctggtgccgt cacttctgtg tgcttgcgga 10740

ctcagaatct ggtggacata tcactcactc tgggatggtg ggcatgggag tcagctgcac 10800

agtcacccgg gaagatggaa ccaatcgcag atagggctgc tagtgaacca atcacatgat 10860

gtcacccaga catcaggcat acccactagt gtgaaataga catcagaatt aagaaaaacg 10920

tagggtccaa gtggttcccc gttatggact cgctatctgt caaccagatc ttataccctg 10980

aagttcacct agatagcccg atagttacca ataagatagt agccatcctg gagtatgctc 11040

gagtccctca cgcttacagc ctggaggacc ctacactgtg tcagaacatc aagcaccgcc 11100

taaaaaacgg attttccaac caaatgatta taaacaatgt ggaagttggg aatgtcatca 11160

agtccaagct taggagttat ccggcccact ctcatattcc atatccaaat tgtaatcagg 11220

atttatttaa catagaagac aaagagtcaa cgaggaagat ccgtgaactc ctcaaaaagg 11280

ggaattcgct gtactccaaa gtcagtgata aggttttcca atgcttaagg gacactaact 11340

cacggcttgg cctaggctcc gaattgaggg aggacatcaa ggagaaagtt attaacttgg 11400

gagtttacat gcacagctcc cagtggtttg agccctttct gttttggttt acagtcaaga 11460

ctgagatgag gtcagtgatt aaatcacaaa cccatacttg ccataggagg agacacacac 11520

ctgtattctt cactggtagt tcagttgagt tgctaatctc tcgtgacctt gttgctataa 11580

tcagtaaaga gtctcaacat gtatattacc tgacatttga actggttttg atgtattgtg 11640

atgtcataga ggggaggtta atgacagaga ccgctatgac tattgatgct aggtatacag 11700

agcttctagg aagagtcaga tacatgtgga aactgataga tggtttcttc cctgcactcg 11760

ggaatccaac ttatcaaatt gtagccatgc tggagcctct ttcacttgct tacctgcagc 11820

tgagggatat aacagtagaa ctcagaggtg ctttccttaa ccactgcttt actgaaatac 11880
atgatgttct tgaccaaaac gggttttctg atgaaggtac ttatcatgag ttaactgaag 11940
ctctagatta cattttcata actgatgaca tacatctgac aggggagatt ttctcatttt 12000
tcagaagttt cggccacccc agacttgaag cagtaacggc tgctgaaaat gttaggaaat 12060
acatgaatca gcctaaagtc attgtgtatg agactctgat gaaaggtcat gccatatttt 12120
gtggaatcat aatcaacggc tatcgtgaca ggcacggagg cagttggcca ccgctgaccc 12180
tccccctgca tgctgcagac acaatccgga atgctcaagc ttcaggtgaa gggttaacac 12240
atgagcagtg cgttgataac tggaaatctt ttgctggagt gaaatttggc tgctttatgc 12300
ctcttagcct ggatagtgat ctgacaatgt acctaaagga caaggcactt gctgctctcc 12360
aaagggaatg ggattcagtt tacccgaaag agttcctgcg ttacgaccct cccaagggaa 12420
ccgggtcacg gaggcttgta gatgttttcc ttaatgattc gagctttgac ccatatgatg 12480
tgataatgta tgttgtaagt ggagcttacc tccatgaccc tgagttcaac ctgtcttaca 12540
gcctgaaaga aaaggagatc aaggaaacag gtagactttt tgctaaaatg acttacaaaa 12600
tgagggcatg ccaagtgatt gctgaaaatc taatctcaaa cgggattggc aaatatttta 12660
aggacaatgg gatggccaag gatgagcacg atttgactaa ggcactccac actctagctg 12720
tctcaggagt ccccaaagat ctcaaagaaa gtcacagggg ggggccagtc ttaaaaacct 12780
actcccgaag cccagtccac acaagtacca ggaacgtgag agcagcaaaa gggtttatag 12840
ggttccctca agtaattcgg caggaccaag acactgatca tccggagaat atggaagctt 12900
acgagacagt cagtgcattt atcacgactg atctcaagaa gtactgcctt aattggagat 12960
atgagaccat cagcttgttt gcacagaggc taaatgagat ttacggattg ccctcatttt 13020
tccagtggct gcataagagg cttgagacct ctgtcctgta tgtaagtgac cctcattgcc 13080
cccccgacct tgacgcccat atcccgttat ataaagtccc caatgatcaa atcttcatta 13140
agtaccctat gggaggtata gaagggtatt gtcagaagct gtggaccatc agcaccattc 13200
cctatctata cctggctgct tatgagagcg gagtaaggat tgcttcgtta gtgcaagggg 13260
acaatcagac catagccgta acaaaaaggg tacccagcac atggccctac aaccttaaga 13320
aacgggaagc tgctagagta actagagatt actttgtaat tcttaggcaa aggctacatg 13380
atattggcca tcacctcaag gcaaatgaga caattgtttc atcacatttt tttgtctatt 13440
caaaaggaat atattatgat gggctacttg tgtcccaatc actcaagagc atcgcaagat 13500
gtgtattctg gtcagagact atagttgatg aaacaagggc agcatgcagt aatattgcta 13560
caacaatggc taaaagcatc gagagaggtt atgaccgtta ccttgcatat tccctgaacg 13620
tcctaaaagt gatacagcaa attctgatct ctcttggctt cacaatcaat tcaaccatga 13680
cccgggatgt agtcataccc ctcctcacaa acaacgacct cttaataagg atggcactgt 13740

tgcccgctcc tattgggggg atgaattatc tgaatatgag caggctgttt gtcagaaaca 13800

tcggtgatcc agtaacatca tcaattgctg atctcaagag aatgattctc gcctcactaa 13860

tgcctgaaga gaccctccat caagtaatga cacaacaacc gggggactct tcattcctag 13920

actgggctag cgacccttac tcagcaaatc ttgtatgtgt ccagagcatc actagactcc 13980

tcaagaacat aactgcaagg tttgtcctga tccatagtcc aaacccaatg ttaaaaggat 14040

tattccatga tgacagtaaa gaagaggacg agggactggc ggcattcctc atggacaggc 14100

atattatagt acctagggca gctcatgaaa tcctggatca tagtgtcaca ggggcaagag 14160

agtctattgc aggcatgctg gataccacaa aaggcttgat tcgagccagc atgaggaagg 14220

gggggttaac ctctcgagtg ataaccagat tgtccaatta tgactatgaa caattcagag 14280

cagggatggt gctattgaca ggaagaaaga gaaatgtcct cattgacaaa gagtcatgtt 14340

cagtgcagct ggcgagagct ctaagaagcc atatgtgggc gaggctagct cgaggacggc 14400

ctatttacgg ccttgaggtc cctgatgtac tagaatctat gcgaggccac cttattcggc 14460

gtcatgagac atgtgtcatc tgcgagtgtg gatcagtcaa ctacggatgg ttttttgtcc 14520

cctcgggttg ccaactggat gatattgaca aggaaacatc atccttgaga gtcccatata 14580

ttggttctac cactgatgag agaacagaca tgaagcttgc cttcgtaaga gccccaagtc 14640

gatccttgcg atctgctgtt agaatagcaa cagtgtactc atgggcttac ggtgatgatg 14700

atagctcttg gaacgaagcc tggttgttgg ctaggcaaag ggccaatgtg agcctggagg 14760

agctaagggt gatcactccc atctcaactt cgactaattt agcgcatagg ttgagggatc 14820

gtagcactca agtgaaatac tcaggtacat cccttgtccg agtggcgagg tataccacaa 14880

tctccaacga caatctctca tttgtcatat cagataagaa ggttgatact aactttatat 14940

accaacaagg aatgcttcta gggttgggtg ttttagaaac attgtttcga ctcgagaaag 15000

ataccggatc atctaacacg gtattacatc ttcacgtcga aacagattgt tgcgtgatcc 15060

cgatgataga tcatcccagg atacccagct cccgcaagct agagctgagg gcagagctat 15120

gtaccaaccc attgatatat gataatgcac ctttaattga cagagatgca acaaggctat 15180

acacccagag ccataggagg caccttgtgg aatttgttac atggtccaca ccccaactat 15240

atcacatttt agctaagtcc acagcactat ctatgattga cctggtaaca aaatttgaga 15300

aggaccatat gaatgaaatt tcagctctca taggggatga cgatatcaat agtttcataa 15360

ctgagtttct gctcatagag ccaagattat tcactatcta cttgggccag tgtgcggcca 15420

tcaattgggc atttgatgta cattatcata gaccatcagg gaaatatcag atgggtgagc 15480

tgttgtcatc gttcctttct agaatgagca aaggagtgtt taaggtgctt gtcaatgctc 15540

taagccaccc aaagatctac aagaaattct ggcattgtgg tattatagag cctatccatg 15600

gtccttcact tgatgctcaa aacttgcaca caactgtgtg caacatggtt tacacatgct 15660

atatgaccta cctcgacctg ttgttgaatg aagagttaga agagttcaca tttctcttgt 15720

gtgaaagcga cgaggatgta gtaccggaca gattcgacaa catccaggca aaacacttat 15780

gtgttctggc agatttgtac tgtcaaccag ggacctgccc accaattcga ggtctaagac 15840

cggtagagaa atgtgcagtt ctaaccgacc atatcaaggc agaggctatg ttatctccag 15900

caggatcttc gtggaacata aatccaatta ttgtagacca ttactcatgc tctctgactt 15960

atctccggcg aggatcgatc aaacagataa gattgagagt tgatccagga ttcattttcg 16020

acgccctcgc tgaggtaaat gtcagtcagc caaagatcgg cagcaacaac atctcaaata 16080

tgagcatcaa ggctttcaga cccccacacg atgatgttgc aaaattgctc aaagatatca 16140

acacaagcaa gcacaatctt cccatttcag ggggcaatct cgccaattat gaaatccatg 16200

ctttccgcag aatcgggttg aactcatctg cttgctacaa agctgttgag atatcaacat 16260

taattaggag atgccttgag ccaggggagg acggcttgtt cttgggtgag ggatcgggtt 16320

ctatgttgat cacttataaa gagatactta aactaaacaa gtgcttctat aatagtgggg 16380

tttccgccaa ttctagatct ggtcaaaggg aattagcacc ctatccctcc gaagttggcc 16440

ttgtcgaaca cagaatggga gtaggtaata ttgtcaaagt gctctttaac gggaggcccg 16500

aagtcacgtg ggtaggcagt gtagattgct tcaatttcat agttagtaat atccctacct 16560

ctagtgtggg gtttatccat tcagatatag agaccttgcc tgacaaagat actatagaga 16620

agctagagga attggcagcc atcttatcga tggctctgct cctgggcaaa ataggatcaa 16680

tactggtgat taagcttatg cctttcagcg gggattttgt tcagggattt ataagttatg 16740

tagggtctca ttatagagaa gtgaaccttg tataccctag atacagcaac ttcatctcta 16800

ctgaatctta tttggttatg acagatctca aggctaaccg gctaatgaat cctgaaaaga 16860

ttaagcagca gataattgaa tcatctgtga ggacttcacc tggacttata ggtcacatcc 16920

tatccattaa gcaactaagc tgcatacaag caattgtggg agacgcagtt agtagaggtg 16980

atatcaatcc tactctgaaa aaacttacac ctatagagca ggtgctgatc aattgcgggt 17040

tggcaattaa cggacctaag ctgtgcaaag aattgatcca ccatgatgtt gcctcagggc 17100

aagatggatt gcttaattct atactcatcc tctacaggga gttggcaaga ttcaaagaca 17160

accaaagaag tcaacaaggg atgttccacg cttaccccgt attggtaagt agcaggcaac 17220

gagaacttat atctaggatc acccgcaaat tctgggggca cattcttctt tactccggga 17280

acaaaaagtt gataaataag tttatccaga atctcaagtc cggctatctg atactagact 17340

tacaccagaa tatcttcgtt aagaatctat ccaagtcaga gaaacagatt attatgacgg 17400

ggggtttgaa acgtgagtgg gtttttaagg taacagtcaa ggagaccaaa gaatggtata 17460

agttagtcgg atacagtgcc ctgattaagg actaattggt tgaactccgg aaccctaatc 17520

ctgccctagg tggttaggca ttatttgcaa tatattaaag aaaactttga aaatacgaag 17580

tttctattcc cagctttgtc tggtggccgg catggtccca gcctcctcgc tggcgccggc 17640

tgggcaacat tccgagggga ccgtcccctc ggtaatggcg aatgggacgc ggccggtcga 17700

tcgacgatcc ggctgctaac aaagcccgaa aggaagctga gttggctgct gccaccgctg 17760

agcaataact agcataaccc cttggggcct ctaaacgggt cttgaggggt tttttgctga 17820

aaggaggaac tatatccgga tcgagatcaa ttctgtgagc gtatggcaaa cgaaggaaaa 17880

atagttatag tagccgcact cgatgggaca tttcaacgta aaccgtttaa taatattttg 17940

aatcttattc cattatctga aatggtggta aaactaactg ctgtgtgtat gaaatgcttt 18000

aaggaggctt ccttttctaa acgattgggt gaggaaaccg agatagaaat aataggaggt 18060

aatgatatgt atcaatcggt gtgtagaaag tgttacatcg actcataata ttatattttt 18120

tatctaaaaa actaaaaata aacattgatt aaattttaat ataatactta aaaatggatg 18180

ttgtgtcgtt agataaaccg tttatgtatt ttgaggaaat tgataatgag ttagattacg 18240

aaccagaaag tgcaaatgag gtcgcaaaaa aactgccgta tcaaggacag ttaaaactat 18300

tactaggaga attatttttt cttagtaagt tacagcgaca cggtatatta gatggtgcca 18360

ccgtagtgta tataggatct gctcccggta cacatatacg ttatttgaga gatcatttct 18420

ataatttagg agtgatcccg aaaggaagct gagttggctg ctgccaccgc tgagcaataa 18480

ctagcataac cccttggggc ctctaaacgg gtcttgaggg gttttttgct gaaaggagga 18540

acgcgcctga tgcggtattt tctccttacg catctgtgcg gtatttcaca ccgcatatat 18600

ggtgcactct cagtacaatc tgctctgatg ccgcatagtt aagccagccc cgacacccgc 18660

caacacccgc tgacgcgccc tgacgggctt gtctgctccc ggcatccgct tacagacaag 18720

ctgtgaccgt ctccgggagc tgcatgtgtc agaggttttc accgtcatca ccgaaacgcg 18780

cgagacgaaa gggcctcgtg atacgcctat ttttataggt taatgtcatg ataataatgg 18840

tttcttagac gtcaggtggc acttttcggg gaaatgtgcg cggaacccct atttgtttat 18900

ttttctaaat acattcaaat atgtatccgc tcatgagaca ataaccctga taaatgcttc 18960

aataatattg aaaaaggaag agtatgagta ttcaacattt ccgtgtcgcc cttattccct 19020

tttttgcggc attttgcctt cctgtttttg ctcacccaga aacgctggtg aaagtaaaag 19080

atgctgaaga tcagttgggt gcacgagtgg gttacatcga actggatctc aacagcggta 19140

agatccttga gagttttcgc cccgaagaac gttttccaat gatgagcact tttaaagttc 19200

tgctatgtgg cgcggtatta tcccgtattg acgccgggca agagcaactc ggtcgccgca 19260

tacactattc tcagaatgac ttggttgagt actcaccagt cacagaaaag catcttacgg 19320

atggcatgac agtaagagaa ttatgcagtg ctgccataac catgagtgat aacactgcgg 19380

ccaacttact tctgacaacg atcggaggac cgaaggagct aaccgctttt ttgcacaaca 19440

tgggggatca tgtaactcgc cttgatcgtt gggaaccgga gctgaatgaa gccataccaa 19500

acgacgagcg tgacaccacg atgcctgtag caatggcaac aacgttgcgc aaactattaa 19560

ctggcgaact acttactcta gcttcccggc aacaattaat agactggatg gaggcggata 19620

aagttgcagg accacttctg cgctcggccc ttccggctgg ctggtttatt gctgataaat 19680

ctggagccgg tgagcgtggg tctcgcggta tcattgcagc actggggcca gatggtaagc 19740

cctcccgtat cgtagttatc tacacgacgg ggagtcaggc aactatggat gaacgaaata 19800

gacagatcgc tgagataggt gcctcactga ttaagcattg gtaactgtca gaccaagttt 19860

actcatatat actttagatt gatttaaaac ttcattttta atttaaaagg atctaggtga 19920

agatcctttt tgataatctc atgaccaaaa tcccttaacg tgagttttcg ttccactgag 19980

cgtcagaccc cgtagaaaag atcaaaggat cttcttgaga tccttttttt ctgcgcgtaa 20040

tctgctgctt gcaaacaaaa aaaccaccgc taccagcggt ggtttgtttg ccggatcaag 20100

agctaccaac tctttttccg aaggtaactg gcttcagcag agcgcagata ccaaatactg 20160

tccttctagt gtagccgtag ttaggccacc acttcaagaa ctctgtagca ccgcctacat 20220

acctcgctct gctaatcctg ttaccagtgg ctgctgccag tggcgataag tcgtgtctta 20280

ccgggttgga ctcaagacga tagttaccgg ataaggcgca gcggtcgggc tgaacggggg 20340

gttcgtgcac acagcccagc ttggagcgaa cgacctacac cgaactgaga tacctacagc 20400

gtgagcattg agaaagcgcc acgcttcccg aagggagaaa ggcggacagg tatccggtaa 20460

gcggcagggt cggaacagga gagcgcacga gggagcttcc agggggaaac gcctggtatc 20520

tttatagtcc tgtcgggttt cgccacctct gacttgagcg tcgatttttg tgatgctcgt 20580

caggggggcg gagcctatgg aaaaacgcca gcaacgcggc ctttttacgg ttcctggcct 20640

tttgctggcc ttttgctcac atgttctttc ctgcgttatc ccctgattct gtggataacc 20700

gtattaccgc ctttgagtga gctgataccg ctcgccgcag ccgaacgacc gagcgcagcg 20760

agtcagtgag cgaggaagcg gaagagcgcc caatacgcaa accgcctctc cccgcgcgtt 20820

ggccgattca ttaatgcagc tggcacgaca ggtttcccga ctggaaagcg ggcagtgagc 20880

gcaacgcaat taatgtgagt tagctcactc attaggcacc ccaggcttta cactttatgc 20940

ttccggctcg tatgttgtgt ggaattgtga gcggataaca atttcacaca ggaaacagct 21000

atgaccatga ttacgccaag cttacgcgtc ctggcattat gcccagtaca tgaccttatg 21060

ggactttcct acttggcagt acatctacgt attagtcatc gctattacca tggtgatgcg 21120

gttttggcag tacatcaatg ggcgtggata gcggtttgac tcacggggat ttccaagtct 21180

ccaccccatt gacgtcaatg ggagtttgtt ttggcaccaa aatcaacggg actttccaaa 21240

atgtcgtaac aactccgccc cattgacgca aatgggcggt aggcgtgtac ggtgggaggt 21300

ctatataagc agagctcgtt tagtgaaccg tgg                                    21333

<210>  6
<211>  20
<212>  PRT
<213>  Artificial Sequence

<220>
<223>  HMA linker

<400>  6

Pro Ser Gly Gln Ala Gly Ala Ala Ala Ser Glu Ser Leu Phe Val Ser
1               5                   10                  15

Asn His Ala Tyr
            20

<210>  7
<211>  120
<212>  PRT
<213>  Artificial Sequence

<220>
<223>  SM3E VH

<400>  7

Gln Val Lys Leu Glu Gln Ser Gly Ala Glu Val Val Lys Pro Gly Ala
1               5                   10                  15

Ser Val Lys Leu Ser Cys Lys Ala Ser Gly Phe Asn Ile Lys Asp Ser
            20                  25                  30

Tyr Met His Trp Leu Arg Gln Gly Pro Gly Gln Cys Leu Glu Trp Ile
        35                  40                  45

Gly Trp Ile Asp Pro Glu Asn Gly Asp Thr Glu Tyr Ala Pro Lys Phe
    50                  55                  60

Gln Gly Lys Ala Thr Phe Thr Thr Asp Thr Ser Ala Asn Thr Ala Tyr
65                  70                  75                  80

Leu Gly Leu Ser Ser Leu Arg Pro Glu Asp Thr Ala Val Tyr Tyr Cys
                85                  90                  95

Asn Glu Gly Thr Pro Thr Gly Pro Tyr Tyr Phe Asp Tyr Trp Gly Gln
            100                 105                 110

Gly Thr Leu Val Thr Val Ser Ser
        115                 120

<210>  8
<211>  106
<212>  PRT
<213>  Artificial Sequence

<220>
<223>  SM3E VL

<400>  8

Glu Asn Val Leu Thr Gln Ser Pro Ser Ser Met Ser Val Ser Val Gly
1               5                   10                  15

Asp Arg Val Thr Ile Ala Cys Ser Ala Ser Ser Ser Val Pro Tyr Met
            20                  25                  30

His Trp Leu Gln Gln Lys Pro Gly Lys Ser Pro Lys Leu Leu Ile Tyr
        35                  40                  45

Leu Thr Ser Asn Leu Ala Ser Gly Val Pro Ser Arg Phe Ser Gly Ser
    50                  55                  60

Gly Ser Gly Thr Asp Tyr Ser Leu Thr Ile Ser Ser Val Gln Pro Glu
65                  70                  75                  80

Asp Ala Ala Thr Tyr Tyr Cys Gln Gln Arg Ser Ser Tyr Pro Leu Thr
                85                  90                  95

Phe Gly Cys Gly Thr Lys Leu Glu Ile Lys
            100                 105

## Description

### TECHNICAL FIELD

The present invention relates to a bispecific killer engager (BiKE) which binds to a tumor antigen and binds to immune cells and its use in promoting anti-tumor cytotoxicity for treating cancer/tumors. In particular, the BiKE can be encoded within the genome of an oncolytic virus of the family *Paramyxoviridae.*

### BACKGROUND

Oncolytic viruses (OV) which replicate selectively in tumor cells are an emerging modality of cancer treatment. Aside from direct cytopathic effects and lysis of tumor cells, interactions of OV with the immune system can trigger systemic anti-tumor immunity. OV have been modified to express immunomodulatory transgenes to further enhance these effects (Melcher et al., Mol Ther. 2011, 19: 1008-1016). The vaccinia virus JX-594 and herpesvirus *talimogene laherpavec* (TVEC), both harboring GM-CSF, have shown promising results in clinical phase II and III trials (Heo et al., Nat Med. 2013, 19: 329-336 and Andtbacka et al. J Clin Oncol. 2013, 31, suppl; abstr LBA9008).

RNA viruses, in particular members of the family *Paramyxoviridae* like, *e.g.,* measles virus, also have shown potential use in oncolysis. Viruses of the family *Paramyxoviridae* are negative-sense single-stranded RNA viruses and include human pathogens like, e.g., human parainfluenza viruses, mumps virus, human respiratory syncytial virus, and measles virus. From wild type measles virus, several non-pathogenic strains, including a vaccine strain, have been derived, which have been shown to remain oncolytic. The measles virus vaccine strain has been developed as a vector platform to target multiple tumor entities and several clinical trials are ongoing. Recently, the capacity of oncolytic MV encoding GM-CSF to support the induction of a specific anti-tumor immune response in terms of a tumor vaccination effect was demonstrated (Grossardt et al, Hum Gene Ther. 2013, 24: 644-654).

There is, however, still a need in the art for improved cancer therapies, in particular for improved oncolytic viruses.

### SUMMARY

In one aspect, the present invention is directed to a bispecific killer engager (BiKE) molecule comprising at least a first binding domain and a second binding domain, wherein the first binding domain is capable of binding to CD16a and wherein the second binding domain is capable of binding to a tumor antigen.

In an embodiment, the tumor antigen of the BiKE can be selected from the group consisting of androgen receptor (AR), BCL-1, calprotectin, carcinoembryonic antigen (CEA), EGFRs, epithelial cell adhesion molecule (Ep-CAM), epithelial sialomucin, membrane estrogen receptors (mER), FAP, HER2/neu, human high molecular weight melanoma-associated antigen (HMW-MAA), IL-6, MOC-1, MOC-21, MOC-52, melan-A/MART-1, melanoma-associated antigen, mucin, OKT9, progesterone receptor (PGR), prostate specific antigen (PSA), prostate stem cell antigen (PSCA), prostate-specific membrane antigen (PSMA), symaptophysin, VEGFRs, CD19, CD20, CD22, CD30 and CD33.

In a preferred embodiment, the second binding domain of the BiKE molecule can bind to the tumor antigen CEA.

In an embodiment, the first binding domain and the second binding domain are separated by a linker. In an embodiment, the first binding domain and/or the second binding domain is a single chain antibody (scFv). In an embodiment, the scFv is a humanized scFv. In an embodiment, wherein the first binding domain scFv and/or the second binding domain scFv comprises heavy and light chain variable domains derived from the same parental full-length antibody or from different parental full-length antibodies.

The BiKE molecule can comprise an amino acid sequence that can be at least 70%, 75%, 80%, 85%, 90%, 95%, 96%, 97%, 98%, 98% or 99% identical to the amino avid sequence depicted in SEQ ID NO:3 and is able to bind to CD16a and to the tumor antigen CEA. In an embodiment, the BiKE molecule comprises the amino acid sequence depicted in SEQ IN NO:3. The BiKE molecule of the present invention can be encoded by a nucleic acid. In an embodiment, the nucleic acid can comprise the nucleotide sequence depicted in SEQ ID NO:4. In an embodiment, the nucleic acid can be a DNA or RNA molecule. In an embodiment, the nucleic acid can be a viral vector, for example, a measles virus vector.

In an aspect, the present invention is directed to a recombinant virus of the family *Paramyxoviridae,* comprising at least one expressible polynucleotide encoding a bispecific killer engager (BiKE) molecule, said molecule comprising at least a first binding domain and a second binding domain, wherein the first binding domain is capable of binding to CD16a and wherein the second binding domain is capable of binding to a tumor antigen. In an embodiment, the tumor antigen is selected from the group consisting of androgen receptor (AR), BCL-1, calprotectin, carcinoembryonic antigen (CEA), EGFRs, epithelial cell adhesion molecule (Ep-CAM), epithelial sialomucin, membrane estrogen receptors (mER), FAP, HER2/neu, human high molecular weight melanoma-associated antigen (HMW-MAA), IL-6, MOC-1, MOC-21, MOC-52, melan-A/MART-1, melanoma-associated antigen, mucin, OKT9, progesterone receptor (PGR), prostate specific antigen (PSA), prostate stem cell antigen (PSCA), prostate-specific membrane antigen (PSMA), symaptophysin, VEGFRs, CD19, CD20, CD22, CD30 and CD33. In an embodiment, the second binding domain is capable of binding to the tumor antigen CEA. In an embodiment, the first binding domain and the second binding domain can be separated by a linker.

In an embodiment, the recombinant virus can be a recombinant Morbillivirus virus, preferably a recombinant measles virus (MV). In an embodiment, the strain of the measles virus can be Schwartz, Edmonston A or Edmonston B.

In an embodiment, the at least one expressible polynucleotide encoding the BiKE molecule can be comprised within a polynucleotide encoding the recombinant virus.

In an embodiment, the expressible polynucleotide encoding the BiKE molecule can further comprise a nucleotide sequence encoding a cytokine. In an embodiment, the expressible polynucleotide encoding the BiKE molecule can further comprise a nucleotide sequence encoding a signal peptide for secretion operably linked to the BiKE molecule.

In an aspect, the present invention provides a polynucleotide encoding the recombinant virus of the family *Paramyxoviridae* of the invention. In an embodiment, the polynucleotide can comprise the nucleotide sequence depicted in SEQ ID NO:5. In an embodiment, the polynucleotide can comprise the portion or fragment of the nucleotide sequence depicted in SEQ ID NO:5 corresponding to the sequence encoding the recombinant virus.

In an aspect, the present invention provides a kit comprising a BiKE molecule according to the invention and a virus of the family *Paramyxoviridae* in one or more containers. In an embodiment, the virus is a measles virus.

In an aspect, the present invention is directed to a BiKE molecule according to the invention which binds to CD16a and to a tumor antigen, such as CEA, or a nucleic acid encoding the BiKE molecule for use in a method for promoting anti-tumor cytotoxicity in a patient or for treating cancer in a patient, the method comprising administering the BiKE molecule or nucleic acid encoding the BiKE molecule to the patient in combination with administering a virus of the family *Paramyxoviridae* to the patient, wherein the BiKE molecule is administered prior to, simultaneously or after administration of the virus.

The present invention is also directed to a recombinant virus of the family *Paramyxoviridae* according to the invention or a nucleic acid encoding the recombinant virus for use in a method for promoting anti-tumor cytotoxicity in a patient or for treating cancer in a patient, the method comprising administering the recombinant virus or nucleic acid encoding the recombinant virus to the patient

In an embodiment, the cancer is malignant melanoma, head and neck cancer, hepatocellular carcinoma, pancreatic carcinoma, prostate cancer, renal cell carcinoma, gastric carcinoma, colorectal carcinoma, lymphomas or leukemias. In an embodiment, the cancer is a solid tumor cancer.

The present invention is directed to an *in vitro* method for activating immune cells to have anti-cancer activity, the method comprising contacting a sample comprising immune cells and cancer cells with a BiKE molecule or a recombinant virus according to the invention, thereby providing activated immune cells having anti-cancer activity. In an embodiment, the immune cells and the cancer cells are in a sample obtained from a cancer patient. In an embodiment, the activated immune cells are for use in a method for treating cancer in a patient, the method comprising administering activated immune cells to the patient.

In embodiments of the present invention, the amino acid and nucleotide sequences can be fragments or variants of the parental amino acid or nucleotide sequence, in which fragment or variant maintains the function of the parental sequence. For example, a fragment or variant of SEQ ID NO:3 is still capable of binding to CD16a and/or CEA with at least 50%, 60%, 65%, 79%, 75%, 80%, 85%, 90%, 95% of the binding affinity compared to the parental sequence.

### DETAILED DESCRIPTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to disclose and encompass embodiments which combine the explicitly described embodiments with any number of the disclosed and/or preferred elements. Furthermore, any permutations and combinations of all described elements in this application should be considered disclosed by this description unless the context indicates otherwise.

The term "about" means approximately or nearly, and in the context of a numerical value or range set forth herein preferably means +/- 10 % of the numerical value or range recited or claimed.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Unless expressly specified otherwise, the term "comprising" is used in the context of the present document to indicate that further members may optionally be present in addition to the members of the list introduced by "comprising". It is, however, contemplated as a specific embodiment of the present invention that the term "comprising" encompasses the possibility of no further members being present, *i.e.,* for the purpose of this embodiment "comprising" is to be understood as having the meaning of "consisting of'.

Indications of relative amounts of a component characterized by a generic term are meant to refer to the total amount of all specific variants or members covered by said generic term. If a certain component defined by a generic term is specified to be present in a certain relative amount, and if this component is further characterized to be a specific variant or member covered by the generic term, it is meant that no other variants or members covered by the generic term are additionally present such that the total relative amount of components covered by the generic term exceeds the specified relative amount; more preferably no other variants or members covered by the generic term are present at all.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the present invention was not entitled to antedate such disclosure.

Terms such as "reduce" or "inhibit" as used herein means the ability to cause an overall decrease, preferably of 5% or greater, 10% or greater, 20% or greater, more preferably of 50% or greater, and most preferably 75% or greater, in the level. The term "inhibit" or similar phrases includes a complete or essentially complete inhibition, *i.e.,* a reduction to zero or essentially to zero.

Terms such as "increase" or "enhance" preferably relate to an increase or enhancement by about at least 10%, preferably at least 20%, preferably at least 30%, more preferably at least 40%, more preferably at least 50%, even more preferably at least 80%, and most preferably at least 100%.

The term "nucleic acid" according to the invention also comprises a chemical derivatization of a nucleic acid on a nucleotide base, on the sugar or on the phosphate, and nucleic acids containing non-natural nucleotides and nucleotide analogs. In some embodiments, the nucleic acid is a deoxyribonucleic acid (DNA) or a ribonucleic acid (RNA). In general, a nucleic acid molecule or a nucleic acid sequence refers to a nucleic acid which is preferably deoxyribonucleic acid (DNA) or ribonucleic acid (RNA). According to the invention, nucleic acids comprise genomic DNA, cDNA, mRNA, viral RNA, recombinantly prepared and chemically synthesized molecules. According to the invention, a nucleic acid may be in the form of a single-stranded or double-stranded and linear or covalently closed circular molecule.

According to the invention "nucleic acid sequence" refers to the sequence of nucleotides in a nucleic acid, *e.g*.; a ribonucleic acid (RNA) or a deoxyribonucleic acid (DNA). The term may refer to an entire nucleic acid molecule (such as to the single strand of an entire nucleic acid molecule) or to a part (*e.g.* a fragment) thereof.

"Fragment", with reference to a nucleic acid sequence, relates to a part of a nucleic acid sequence, *i.e*.; a sequence which represents the nucleic acid sequence shortened at the 5'- and/or 3'-end(s). Preferably, a fragment of a nucleic acid sequence comprises at least 80%, preferably at least 90%, 95%, 96%, 97%, 98%, or 99% of the nucleotide residues from said nucleic acid sequence. In the present invention those fragments of RNA molecules are preferred which retain RNA stability and/or translational efficiency.

"Fragment", with reference to an amino acid sequence (peptide or protein), relates to a part of an amino acid sequence, *i.e.,* a sequence which represents the amino acid sequence shortened at the N-terminus and/or C-terminus. A fragment shortened at the C-terminus (N-terminal fragment) is obtainable, e.g., by translation of a truncated open reading frame that lacks the 3'-end of the open reading frame. A fragment shortened at the N-terminus (C-terminal fragment) is obtainable, e.g., by translation of a truncated open reading frame that lacks the 5'-end of the open reading frame, as long as the truncated open reading frame comprises a start codon that serves to initiate translation. A fragment of an amino acid sequence comprises e.g. at least 1 %, at least 2 %, at least 3 %, at least 4 %, at least 5 %, at least 10 %, at least 20 %, at least 30 %, at least 40 %, at least 50 %, at least 60 %, at least 70 %, at least 80%, at least 90% of the amino acid residues from an amino acid sequence.

The term "variant" with respect to, for example, nucleic acid and amino acid sequences, according to the invention includes any variants, in particular mutants, viral strain variants, splice variants, conformations, isoforms, allelic variants, species variants and species homologs, in particular those which are naturally present. An allelic variant relates to an alteration in the normal sequence of a gene, the significance of which is often unclear. Complete gene sequencing often identifies numerous allelic variants for a given gene. With respect to nucleic acid molecules, the term "variant" includes degenerate nucleic acid sequences, wherein a degenerate nucleic acid according to the invention is a nucleic acid that differs from a reference nucleic acid in codon sequence due to the degeneracy of the genetic code. A species homolog is a nucleic acid or amino acid sequence with a different species of origin from that of a given nucleic acid or amino acid sequence. A virus homolog is a nucleic acid or amino acid sequence with a different virus of origin from that of a given nucleic acid or amino acid sequence.

According to the invention, nucleic acid variants include single or multiple nucleotide deletions, additions, mutations, substitutions and/or insertions in comparison with the reference nucleic acid. Deletions include removal of one or more nucleotides from the reference nucleic acid. Addition variants comprise 5'- and/or 3'-terminal fusions of one or more nucleotides, such as 1, 2, 3, 5, 10, 20, 30, 50, or more nucleotides. In the case of substitutions, at least one nucleotide in the sequence is removed and at least one other nucleotide is inserted in its place (such as transversions and transitions). Mutations include abasic sites, crosslinked sites, and chemically altered or modified bases. Insertions include the addition of at least one nucleotide into the reference nucleic acid.

According to the invention, "nucleotide change" can refer to single or multiple nucleotide deletions, additions, mutations, substitutions and/or insertions in comparison with the reference nucleic acid. In some embodiments, a "nucleotide change" is selected from the group consisting of a deletion of a single nucleotide, the addition of a single nucleotide, the mutation of a single nucleotide, the substitution of a single nucleotide and/or the insertion of a single nucleotide, in comparison with the reference nucleic acid. According to the invention, a nucleic acid variant can comprise one or more nucleotide changes in comparison with the reference nucleic acid.

Variants of specific nucleic acid sequences preferably have at least one functional property of said specific sequences and preferably are functionally equivalent to said specific sequences, e.g., nucleic acid sequences exhibiting properties identical or similar to those of the specific nucleic acid sequences.

As described below, some embodiments of the present invention are characterized, *inter alia,* by nucleic acid sequences that are homologous to other nucleic acid sequences. These homologous sequences are variants of other nucleic acid sequences.

Preferably the degree of identity between a given nucleic acid sequence and a nucleic acid sequence which is a variant of said given nucleic acid sequence will be at least 70%, preferably at least 75%, preferably at least 80%, more preferably at least 85%, even more preferably at least 90% or most preferably at least 95%, 96%, 97%, 98% or 99%. The degree of identity is preferably given for a region of at least about 30, at least about 50, at least about 70, at least about 90, at least about 100, at least about 150, at least about 200, at least about 250, at least about 300, or at least about 400 nucleotides. In preferred embodiments, the degree of identity is given for the entire length of the reference nucleic acid sequence.

"Sequence similarity" indicates the percentage of amino acids that either are identical or that represent conservative amino acid substitutions. "Sequence identity" between two polypeptide or nucleic acid sequences indicates the percentage of amino acids or nucleotides that are identical between the sequences.

The term "% identical" is intended to refer, in particular, to a percentage of nucleotides which are identical in an optimal alignment between two sequences to be compared, with said percentage being purely statistical, and the differences between the two sequences may be randomly distributed over the entire length of the sequence and the sequence to be compared may comprise additions or deletions in comparison with the reference sequence, in order to obtain optimal alignment between two sequences. Comparisons of two sequences are usually carried out by comparing said sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2:482, with the aid of the local homology algorithm by Needleman and Wunsch, 1970, J. Mol. Biol. 48:443, and with the aid of the similarity search algorithm by Pearson and Lipman, 1988, Proc. Natl Acad. Sci. USA 85:2444 or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.).

Percentage identity is obtained by determining the number of identical positions in which the sequences to be compared correspond, dividing this number by the number of positions compared and multiplying this result by 100.

For example, the BLAST program "BLAST 2 sequences" which is available on the website http://www.ncbi.nlm.nih.gov/blast/bl2seq/wblast2.cgi may be used.

A nucleic acid is "capable of hybridizing" or "hybridizes" to another nucleic acid if the two sequences are complementary with one another. A nucleic acid is "complementary" to another nucleic acid if the two sequences are capable of forming a stable duplex with one another. According to the invention, hybridization is preferably carried out under conditions which allow specific hybridization between polynucleotides (stringent conditions). Stringent conditions are described, for example, in Molecular Cloning: A Laboratory Manual, J. Sambrook et al., Editors, 2nd Edition, Cold Spring Harbor Laboratory press, Cold Spring Harbor, New York, 1989 or Current Protocols in Molecular Biology, F.M. Ausubel et al., Editors, John Wiley & Sons, Inc., New York and refer, for example, to hybridization at 65°C in hybridization buffer (3.5 x SSC, 0.02% Ficoll, 0.02% polyvinylpyrrolidone, 0.02% bovine serum albumin, 2.5 mM NaH2PO4 (pH 7), 0.5% SDS, 2 mM EDTA). SSC is 0.15 M sodium chloride/0.15 M sodium citrate, pH 7. After hybridization, the membrane to which the DNA has been transferred is washed, for example, in 2 × SSC at room temperature and then in 0.1-0.5 × SSC/0.1 × SDS at temperatures of up to 68°C.

A percent complementarity indicates the percentage of contiguous residues in a nucleic acid molecule that can form hydrogen bonds (e.g., Watson-Crick base pairing) with a second nucleic acid sequence *(e.g.,* 5, 6, 7, 8, 9, 10 out of 10 being 50%, 60%, 70%, 80%, 90%, and 100% complementary). "Perfectly complementary" or "fully complementary" means that all the contiguous residues of a nucleic acid sequence will hydrogen bond with the same number of contiguous residues in a second nucleic acid sequence. Preferably, the degree of complementarity according to the invention is at least 70%, preferably at least 75%, preferably at least 80%, more preferably at least 85%, even more preferably at least 90% or most preferably at least 95%, 96%, 97%, 98% or 99%. Most preferably, the degree of complementarity according to the invention is 100%.

The term "derivative" comprises any chemical derivatization of a nucleic acid on a nucleotide base, on the sugar or on the phosphate. The term "derivative" also comprises nucleic acids which contain nucleotides and nucleotide analogs not occurring naturally. Preferably, a derivatization of a nucleic acid increases its stability.

A "nucleic acid sequence which is derived from a nucleic acid sequence" refers to a nucleic acid which is a variant of the nucleic acid from which it is derived. Preferably, a sequence which is a variant with respect to a specific sequence, when it replaces the specific sequence in an RNA molecule retains RNA stability and/or translational efficiency.

"nt" is an abbreviation for nucleotide; or for nucleotides, preferably consecutive nucleotides in a nucleic acid molecule.

According to the invention, the term "codon" refers to a base triplet in a coding nucleic acid that specifies which amino acid will be added next during protein synthesis at the ribosome.

The terms "transcription" and "transcribing" relate to a process during which a nucleic acid molecule with a particular nucleic acid sequence (the "nucleic acid template") is read by an RNA polymerase so that the RNA polymerase produces a single-stranded RNA molecule. During transcription, the genetic information in a nucleic acid template is transcribed. The nucleic acid template may be DNA; however, *e.g*., in the case of transcription from an alphaviral nucleic acid template, the template is typically RNA. Subsequently, the transcribed RNA may be translated into protein. According to the present invention, the term "transcription" comprises *"in vitro* transcription", wherein the term *"in vitro* transcription" relates to a process wherein RNA, in particular mRNA, is *in vitro* synthesized in a cell-free system. Preferably, cloning vectors are applied for the generation of transcripts. These cloning vectors are generally designated as transcription vectors and are according to the present invention encompassed by the term "vector". The cloning vectors are preferably plasmids. According to the present invention, RNA preferably is *in vitro* transcribed RNA (IVT-RNA) and may be obtained by *in vitro* transcription of an appropriate DNA template. The promoter for controlling transcription can be any promoter for any RNA polymerase. A DNA template for *in vitro* transcription may be obtained by cloning of a nucleic acid, in particular cDNA, and introducing it into an appropriate vector for *in vitro* transcription. The cDNA may be obtained by reverse transcription of RNA.

The single-stranded nucleic acid molecule produced during transcription typically has a nucleic acid sequence that is the complementary sequence of the template.

According to the invention, the terms "template" or "nucleic acid template" or "template nucleic acid" generally refer to a nucleic acid sequence that may be replicated or transcribed.

"Nucleic acid sequence transcribed from a nucleic acid sequence" and similar terms refer to a nucleic acid sequence, where appropriate as part of a complete RNA molecule, which is a transcription product of a template nucleic acid sequence. Typically, the transcribed nucleic acid sequence is a single-stranded RNA molecule.

According to the invention, "functional linkage" or "functionally linked" relates to a connection within a functional relationship. A nucleic acid is "functionally linked" if it is functionally related to another nucleic acid sequence. For example, a promoter is functionally linked to a coding sequence if it influences transcription of said coding sequence. Functionally linked nucleic acids are typically adjacent to one another, where appropriate separated by further nucleic acid sequences, and, in particular embodiments, are transcribed by RNA polymerase to give a single RNA molecule (common transcript).

In particular embodiments, a nucleic acid is functionally linked according to the invention to expression control sequences which may be homologous or heterologous with respect to the nucleic acid.

The term "nucleic acid encoding a peptide or protein" means that the nucleic acid, if present in the appropriate environment, preferably within a cell, can direct the assembly of amino acids to produce the peptide or protein during the process of translation. Preferably, coding RNA according to the invention is able to interact with the cellular translation machinery allowing translation of the coding RNA to yield a peptide or protein.

The term "peptide" comprises oligo- and polypeptides and refers to substances which comprise two or more, preferably 3 or more, preferably 4 or more, preferably 6 or more, preferably 8 or more, preferably 10 or more, preferably 13 or more, preferably 16 or more, preferably 20 or more, and up to preferably 50, preferably 100 or preferably 150, consecutive amino acids linked to one another via peptide bonds. The term "protein" refers to large peptides, preferably peptides having at least 151 amino acids, but the terms "peptide" and "protein" are used herein usually as synonyms.

The terms "peptide" and "protein" comprise, according to the invention, substances which contain not only amino acid components but also non-amino acid components such as sugars and phosphate structures, and also comprise substances containing bonds such as ester, thioether or disulfide bonds.

The terms "virus" and "virus of the family *Paramyxoviridae*" are known to the skilled person. Preferably, the virus of the family *Paramyxoviridae* is a member of the genus Morbillivirus. More preferably, the virus of the family *Paramyxoviridae* is a measles virus (MV), such as the MV strain Schwarz, or the MV strain Edmonston A or B.

The term "recombinant virus", as used herein, relates to a virus comprising a genome modified by biotechnological means as compared to known, naturally occurring, viral genomes. Preferably, the recombinant virus is a virus comprising a genome modified as compared to its naturally occurring genome. Preferred biotechnological means for modifying a viral genome are known to the skilled person and include any of the methods of molecular cloning, in particular recombinant DNA techniques including, without limitation, cleavage of DNA by restriction enzymes, ligation of DNA, polymerase chain reaction (PCR), cloning of viral genomes, and the like. It is understood by the skilled person that viruses of the family *Paramyxoviridae* have a single-stranded (-)-RNA as a genome. Accordingly, the genome of the recombinant virus of the present invention, preferably, is obtained by cloning an expression vector as described herein below comprising an expressible nucleotide sequence encoding said recombinant virus genome. The genome can then be transferred into a host cell for expression. The host cell can be a naturally permissive host cell for replication of the virus on which the recombinant virus is based, or the host cell can be a non-permissive host cell, *i.e*., a host cell that is not normally infected by the virus on which the recombinant virus is based.

As used herein, the term "bispecific killer engager molecule" (BiKE) relates to a polypeptide or variant or derivative thereof, preferably specifically binding, to at least two non-identical epitopes, wherein said epitopes, preferably, are epitopes of non-identical molecules of interest. The bispecific killer engager polypeptide comprises at least a first binding domain binding to a surface molecule of an immune cell with antitumor activity, preferably binding to CD16a. Such CD16a-positive cells include NK cells, monocytes, macrophages and neutrophils. The term "immune cell with antitumor activity", as used herein relates to a lymphocyte, preferably a lymphocyte with the capacity to inactivate cancer cells and/or to activate cells that can inactivate cancer cells. An immune cell with antitumor activity can be a T cell, a dendritic cell or a natural killer cell. Thus, preferably, the BiKE comprises a first binding domain binding, preferably specifically binding, to CD16a on the surface of a T cell, preferably on the surface of a natural killer cell, and a second binding domain binding to a tumor antigen. The BiKE can further comprise a transport signal or signal sequence, in particular a peptide export signal. Preferably, the constituent parts of the BiKE, *i.e*., in particular the first binding domain and the second binding domain, are contiguous in amino acid sequence. The constituent parts optionally can be separated by a peptide linker. The BiKE can be expressed from a single open reading frame comprised within a polynucleotide. Thus, the BiKE as described herein can be a polypeptide expressed from a single transcription unit. Accordingly, the BiKE can be a fusion polypeptide. Preferably, the BiKE comprises at least one, more preferably two, single-chain antibodies, single-chain Fab polypeptides, or nanobodies which are able to bind CD16a and to the tumor antigen, respectively. In an embodiment, the BiKE can be a secreted polypeptide.

The term "binding domain" is known to the skilled person and relates to a contiguous or non-contiguous subpart of a polypeptide having the activity of binding, preferably specifically binding, to a molecule of interest (cognate binding partner, for example, an antibody binding domain and its cognate antigen). Preferably, the binding domain binds to its cognate partner with sufficient affinity to allow detection of a binding domain/antigen complex. Preferably, the dissociation constant (Kd) of the binding domain/partner complex is at most 10⁻⁶ mol/L, more preferably at most 10⁻⁷ mol/L, even more preferably, at most 10⁻⁸ mol/L, most preferably, at most 10⁻⁹ mol/L. The term "specifically binding" is understood by the skilled person and can relate to binding in which the affinity of the binding domain to the partner is at least threefold, more preferably at least fivefold, still more preferably at least tenfold, even more preferably at least 100-fold, most preferably at least 1000-fold higher than for any other molecule/non-cognate partner. Accordingly, preferred dissociation constants (Kd) of the binding domain in a BiKE of the present invention to its binding partner, e.g., CD16a and/or CEA, is at least 10⁻⁶ mol/L, at least 10⁻⁷ mol/L, at least 10⁻⁸ mol/L or at least 10⁻⁹ mol/L.

Preferably, the binding domains of the BiKE can be independently selected from the list of molecule types consisting of a peptide aptamer, an anticalin, a Designed Ankyrin Repeat Protein (DARPin), or an antibody or fragment thereof. Preferably, at least one, more preferably two binding domains are antibodies or fragments or portions thereof as specified herein below. In a particular embodiment, the binding domains of the BiKE are each single-chain antibodies (scFv) or nanobodies.

In the context of the present invention, a "peptide aptamer" is a peptide that can specifically bind to a molecule of interest. Peptide aptamers are peptides, preferably, comprising 8-80 amino acids, more preferably 10-50 amino acids, and most preferably 15-30 amino acids. They can be, *e.g.,* isolated from randomized peptide expression libraries in a suitable host system like baker's yeast (see, for example, Klevenz et al., Cell Mol Life Sci. 2002, 59: 1993-1998). The peptide aptamer can be present in a BiKE of the invention as a binding domain of the BiKE. As used herein, the term "anticalin" relates to an artificial polypeptide derived from a lipocalin which can specifically bind to a molecule of interest. Similarly, a "Designed Ankyrin Repeat Protein" or "DARPin", as used herein, is an artificial polypeptide comprising several ankyrin repeat motifs and which can specifically bind a molecule of interest.

As used herein, the term "antibody" relates to an immunoglobulin from any of the classes of antibodies, e.g., IgA, IgD, IgE, IgG, or IgM, having the activity of specifically binding to a molecule of interest, e.g., CD16a or CEA. Antibodies against antigens can be prepared by well-known methods using, *e.g.,* a purified molecule of interest or a suitable fragment derived therefrom as an antigen. A fragment which is suitable as an antigen may be identified by antigenicity determining algorithms well known in the art. Such fragments may be obtained either from one of the molecules of interest by proteolytic digestion, may be a synthetic peptide, or may be obtained by recombinant expression. Suitability of an antibody generated as a binding domain of the BiKE can be tested by the assay as described herein in the Examples. Preferably, the antibody of the present invention is a monoclonal antibody, a human or humanized antibody or primatized, chimerized or fragment thereof so long as the desired binding activity is exhibited. Also comprised as antibodies of the present invention are a bispecific antibody, a synthetic antibody, or a chemically modified derivative of any of these. Preferably, the antibody of the present invention shall specifically bind (*i.e.* does not cross react with other polypeptides or peptides) to a molecule of interest as specified above. Specific binding can be tested by various well known techniques. Antibodies or fragments thereof can be obtained by using methods which are described, e.g., in Harlow and Lane "Antibodies, A Laboratory Manual", CSH Press, Cold Spring Harbor, 1988. Monoclonal antibodies can be prepared by the techniques originally described in Kohler and Milstein, Nature. 1975. 256: 495; and Galfré, Meth. Enzymol. 1981, 73:3, which comprise the fusion of mouse myeloma cells to spleen cells derived from immunized mammals.

"Antibody fragments" comprise a portion of an intact antibody, in an embodiment, comprising the antigen-binding region thereof. Examples of antibody fragments and fusion proteins of variable regions include Fab, Fab', F(ab')2, and Fv fragments; diabodies; linear antibodies; single-chain antibody molecules; single-domain-antibodies (VHH), also known as nanobodies, and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen. "Fv" is the minimum antibody fragment which contains a complete antigen-binding site. Preferably, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three hypervariable regions (HVRs, also referred to as complementarity determining regions (CDRs)) of each variable domain interact to define an antigen-binding site. Collectively, the six HVRs of one scFv confer antigen-binding specificity to the antibody. However, even a single variable domain (or half of an Fv comprising only three HVRs specific for an antigen) has the ability to recognize and bind antigen, although at a lower affinity than the entire binding site. The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 0 404 097; WO 1993/01161; Hudson et al., Nat. Med. 9 (2003) 129-134; and Hollinger et al., PNAS USA 90 (1993) 6444-6448. Triabodies and tetrabodies are also described in Hudson et al., Nat. Med. 9 (2003) 129-134.

In an embodiment, the BiKE comprises further amino acids which may serve, *e.g.,* as immunogenic antigens, as a tag for purification or detection or as a linker. In an embodiment of the BiKE of the invention, the BiKE further comprises a detectable tag. The term "detectable tag" refers to a stretch of amino acids which are added to or introduced into the BiKE of the invention. Preferably, the tag shall be added C- or N-terminally to the BiKE. The stretch of amino acids can allow for detection of the BiKE by an antibody which specifically recognizes the tag or it can allow for the formation of a functional conformation, such as a chelator or allow for visualization by fluorescent tags. Preferred tags are the Myc-tag, FLAG-tag, poly-His-tag, HA-tag, GST-tag or GFP-tag. These tags are all well known in the art. In an embodiment, the BiKE can further comprise a cytokine as specified herein.

The second binding domain of the BiKE binds to a tumor antigen. In an embodiment, the tumor antigen is an antigen exposed on the surface of a tumor/cancer cell. The tumor antigen can be selected from the group consisting of androgen receptor (AR), BCL-1, calprotectin, carcinoembryonic antigen (CEA), EGFRs, epithelial cell adhesion molecule (Ep-CAM), epithelial sialomucin, membrane estrogen receptor (mERs), FAP, HER2/neu, human high molecular weight-melanoma-associated antigen (HMW-MAA), IL-6, MOC-1, MOC-21, MOC-52, melan-A/MART-1, melanoma-associated antigen, mucin, OKT9, progesterone receptor (PGR), prostate specific antigen (PSA), prostate stem cell antigen (PSCA), prostate-specific membrane antigen (PSMA), synaptophysin, VEGFRs, CD19, CD22, CD30 and CD33.

Preferably, the tumor antigen is carcinoembryonic antigen (CEA). Also preferably, the second binding domain comprises a single-chain antibody against CEA.

The term "secreted", as used herein, relates to a compound being transferred from the interior of a host cell to the exterior of said host cell by a mechanism intrinsic to said host cell. Where the BiKE is a peptide or polypeptide, said secretion is mediated by a signal peptide mediating import of said peptide or polypeptide into the lumen of the endoplasmic reticulum. Optionally, the BiKE does not comprise an endoplasmic reticulum retention signal. Signal peptides causing secretion of peptides or polypeptides are known in the art. In an embodiment, the signal peptide is or comprises an Ig leader sequence or comprises a human Ig leader sequence. In an embodiment, the signal peptide is or comprises a matching leader sequence, *i.e*., a leader sequence selected from the same Ig kappa subgroup as the variable light chain of the antibody, or single-chain antibody.

The term "cytokine" is known to the skilled person and relates to any one of a group of peptides released by cells and affecting the state or behavior of other cells or the secreting cells themselves. In an embodiment, the cytokine can be a chemokine, an interferon, an interleukin, a lymphokine, or a tumor necrosis factor. In an embodiment, the cytokine can be GM-CSF (Genbank Acc NO: AAA52121.1 GI:181146, encoded by Genbank Acc NO: M10663.1 GI:181145) or Interleukin-12 (p35 subunit, Genbank Acc NO: AAD16432.1 GI:4323579; p40 subunit, Genbank Acc NO: AAG32620.1 GI:11192035.)

Moreover, also encompassed are variants of the BiKEs. Such variants have at least the same essential biological activity as the parental BiKE. Moreover, it is to be understood that a variant as referred to in accordance with the present invention shall have an amino acid sequence which differs due to at least one amino acid substitution, deletion and/or addition, wherein the amino acid sequence of the variant is still, preferably, at least 50%, 60%, 70%, 80%, 85%, 90%, 92%, 95%, 97%, 98%, or 99% identical with the amino sequence of the specific inhibitory peptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining, preferably over the whole length of the peptide, the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, Wis.), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the parental BiKE or the aforementioned types of variants as long as these fragments and/or variants have the essential biological activity as referred to above. Such fragments may be or be derived from, e.g., degradation products or splice variants of the parental BiKEs. Further included are variants which differ due to posttranslational modifications such as phosphorylation, glycosylation, ubiquitinylation, sumoylation or myristylation.

The nucleic acid molecule encoding a BiKE of the present invention; in addition to the BiKE coding sequence, can have further sequences such as expression control sequences or regulatory elements for controlling transcription of the BiKE coding sequence. Regulatory elements ensuring expression in eukaryotic cells, preferably mammalian cells, are well known in the art. They can comprise regulatory sequences ensuring initiation of transcription and, optionally, poly-A signals ensuring termination of transcription and stabilization of the transcript. Additional regulatory elements may include transcriptional as well as translational enhancers. In an embodiment, the aforesaid at least one expression control sequence is an expression control sequence of a (-)strand RNA virus, such as a Paramyxovirus as described herein, e.g., a measles virus (MV). In an embodiment, the at least one expression control sequence comprises a (-)strand RNA viral regulatory sequence ensuring initiation of transcription (consensus "gene start signal", for example, a consensus MV "gene start signal") and termination signals (consensus "gene stop signal", for example, consensus MV "gene stop signal") ensuring termination of transcription and stabilization of the transcript. It is known in the art that production of Paramyxovirus viral particles in permissive host cells can be initiated by transfecting into said permissive host cells one or more expressible DNA constructs encoding (i) a recombinant viral anti-genome, (ii) the viral L gene, (iii) the viral P gene and (iv) the viral N gene. It is also understood by the skilled person that, once a viral genome and the aforesaid viral genes were expressed in said host cell, replication and assembly of viral particles occurs in the cytoplasm of the host cell and is, therefore, solely dependent on viral regulatory signals.

The term "polynucleotide encoding a recombinant virus", as used herein, relates to a polynucleotide comprising a nucleic acid sequence or nucleic acid sequences required for generating a virus particle or a virus-like particle in a host cell. It is understood by the skilled person that a virus is constituted by a polynucleotide genome and at least one kind of capsid polypeptide. Accordingly, the polynucleotide encoding a recombinant virus of the present invention, preferably, comprises a recombinant virus genome. As will be understood by the skilled person, in case the polynucleotide encoding a recombinant virus is comprised in a virus according to the present invention, the polynucleotide is (-) strand RNA. It is also understood by the skilled person that in case the polynucleotide is DNA comprised in a host cell, at least an RNA-dependent RNA polymerase activity will additionally be required to produce viral particles from said DNA polynucleotide. The sequence of the DNA copy of negative-strand (- ) RNA viruses is annotated in the usual 5'→3-orientation; this corresponds to the viral sequence in antigenomic (+) RNA orientation with respect to the natural 3'→5'-orientation of negative-strand (-) RNA viruses.

As used herein, the term "host cell" relates to a vertebrate cell. Preferably, the cell is a mammalian cell, more preferably, a mouse, rat, cat, dog, hamster, guinea pig, sheep, goat, pig, cattle, or horse cell. Still more preferably, the host cell is a primate cell. Most preferably, the host cell is a human cell. The host cell can be a tumor cell.

Advantageously, it was found in the work underlying the present invention that measles virus can be engineered to express BiKEs destined for secretion and that these can be are efficiently secreted during viral replication in the cell. Moreover, it was found that by administering measles virus expressing a BiKE according to the invention, immune cells such as T cells or NK cells can be effectively tethered to tumor cells.

The present invention further relates to a polynucleotide encoding the BiKE or recombinant virus of the family *Paramyxoviridae* according to the present invention.

The present invention also relates to a medicament or a pharmaceutical composition comprising the BiKE or recombinant virus of the family *Paramyxoviridae* of the present invention and at least one pharmacologically acceptable carrier or excipient.

The terms "medicament" and "pharmaceutical composition", as used herein, relate to the BiKEs, encoding nucleic acids and recombinant viruses ("compounds") of the invention and optionally one or more pharmaceutically acceptable carrier or excipient. The compounds of the invention can be formulated as pharmaceutically acceptable salts. Acceptable salts comprise acetate, methyl ester, HCl, sulfate, chloride and the like. The pharmaceutical compositions can be administered locally, topically or systemically. Suitable routes of administration conventionally used for drug administration are oral, intravenous, or parenteral administration as well as inhalation. A preferred route of administration is intra-tumoral administration. In an embodiment, the nucleic acid compounds of the invention can be administered in a gene therapy approach by using viral vectors or viruses or liposomes.

Moreover, the compounds of the invention can be administered in combination with other drugs either in a common pharmaceutical composition or as separated pharmaceutical compositions wherein said separated pharmaceutical compositions may be provided in form of a kit of parts. The compounds can be administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The excipient(s) must be acceptable in the sense of being compatible with the other ingredients of the formulation and being not deleterious to the recipient thereof. The excipient employed may be, for example, a solid, a gel or a liquid carrier. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pa. The diluent(s) is/are selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, non-immunogenic stabilizers and the like.

A therapeutically effective dose refers to an amount of the compounds to be used in a pharmaceutical composition of the present invention which prevents, ameliorates or treats the symptoms accompanying a disease or condition referred to herein, e.g., cancer. Therapeutic efficacy and toxicity of such compounds can be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, LD₅₀/ED₅₀.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg for a polypeptide or polynucleotide, or 10⁴⁻10⁸ viral particles for a virus or a virus-like particle; however, doses below or above this exemplary range are envisioned, especially considering the aforementioned factors. Progress can be monitored by periodic assessment. The pharmaceutical compositions and formulations referred to herein are administered at least once in order to treat or ameliorate or prevent a disease or condition recited in this specification. However, the pharmaceutical compositions may be administered more than once, for example from one to four times daily up to a non-limited number of days. Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adapted to the mode of administration, *i.e*. in the forms of tablets, capsules, suppositories, solutions, suspensions or the like. Dosage recommendations shall be indicated in the instructions for use in order to anticipate dose adjustments depending on the considered recipient.

The present invention also relates to a method of promoting anti-tumor cytotoxicity in a patient or for treating cancer in a patient or for treating inappropriate cell proliferation in a patient, the method comprising administering a BiKE molecule or nucleic acid encoding the BiKE molecule of the invention to the patient in combination with administering a virus of the family *Paramyxoviridae* to the patient, wherein the BiKE molecule is administered prior to, simultaneously or after administration of the virus.

The present invention also relates to a method of promoting anti-tumor cytotoxicity in a patient or for treating cancer in a patient or for treating inappropriate cell proliferation in a patient, the method comprising administering a recombinant virus of the family *Paramyxoviridae* of the invention or a nucleic acid encoding the recombinant virus to the patient.

The methods of treatment of the present invention can comprise steps in addition to those explicitly mentioned above. For example, further steps may relate, *e.g.,* to localizing a tumor and/or diagnosing cancer or administration of additional medication.

The term "treatment" refers to an amelioration of the diseases or disorders referred to herein or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99%. The p-values are, preferably, 0.1, 0.05, 0.01, 0.005, or 0.0001. Preferably, the treatment shall be effective for at least 10%, at least 20% at least 50% at least 60%, at least 70%, at least 80%, or at least 90% of the subjects of a given cohort or population. Preferably, treating cancer is reducing tumor burden in a subject. As will be understood by the skilled person, effectiveness of treatment of cancer is dependent on a variety of factors including cancer stage and cancer type.

As used herein, the terms "patient" or "subject" are used interchangeably and relate to a vertebrate. In an embodiment, the vertebrate is a mammal, such as a mouse, rat, cat, dog, hamster, guinea pig, sheep, goat, pig, cattle, or horse. In an embodiment, the mammal is a primate, such as a human.

The term "cancer", as used herein, relates to a disease of an animal, including humans, which is characterized by uncontrolled growth by a group of body cells ("cancer cells"). This uncontrolled growth may be accompanied by intrusion into and destruction of surrounding tissue and possibly spread of cancer cells to other locations in the body. Also included by the term cancer is a relapse of the cancer.

Exemplary cancers include but are not limited to acute lymphoblastic leukemia, acute myeloid leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, Burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, fibrosarcoma, gallbladder cancer, gastric cancer, gastrointestinal stromal tumor, gestational trophoblastic tumor, hairy cell leukemia, head and neck cancer, hepatocellular cancer, Hodgkin lymphoma, hypopharyngeal cancer, hypothalamic and visual pathway glioma, intraocular melanoma, Kaposi sarcoma, laryngeal cancer, medulloblastoma, medulloepithelioma, melanoma, Merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-Hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, Sezary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, Waldenström macroglobulinemia, and Wilm's tumor. In an embodiment, the cancer is a solid cancer, a metastasis, or a relapse thereof. In an embodiment, the cancer is a tumor derived from malignant melanoma, head and neck cancer, hepatocellular carcinoma, pancreatic carcinoma, prostate cancer, renal cell carcinoma, gastric carcinoma, colorectal carcinoma, lymphoma or leukemia. The present invention also relates to an *in vitro* method for activating immune cells to have anti-cancer activity, the method comprising contacting a sample comprising immune cells and cancer cells with a BiKE molecule according to the invention or a recombinant virus according to the invention, thereby providing activated immune cells having anti-cancer activity. In an embodiment, the immune cells and the cancer cells are in a sample obtained from a cancer patient. The present invention also relates to a method for treating cancer in a patient, the method comprising administering activated immune cells produced by the *in vitro* method of the invention to the patient.

The present invention is described in detail and is illustrated by the figures and examples, which are used only for illustration purposes and are not meant to be limiting. Owing to the description and the examples, further embodiments which are likewise included in the invention are accessible to the skilled worker.

### DESCRIPTION OF THE FIGURES

### Figure 1

### NK cell activation in oncolytic measles virotherapy

NK cell activation upon exposure to colorectal cancer (KM12, HT 29) or pancreatic ductal adenocarcinoma (HPAC) cell lines infected with recombinant measles vaccine strain virus (MV) was assessed *in vitro.* (A) Schematic outline. Tumor cells were inoculated with MeVac at a multiplicity of infection (MOI) of 1 or subjected to mock infection one day prior to coculture with NK cells isolated from healthy donor PBMCs. After 12 h co incubation, *i.e*., 36 h post inoculation, expression of the early activation marker CD69 was analyzed by flow cytometry. NK cell only control and IL 2 stimulated positive control were included for comparison. (B) Exemplary histograms depict expression levels of CD69 on live CD45⁺ CD56⁺ NK cells after coculture with infected cells compared to control conditions. (C) CD69 mean fluorescence intensity (MFI) is summarized for n ≥ 4 donors for each cell line with different symbols representing individual donors. (D) Overview of mechanism of action.

### Figure 2

### NK cell effector functions in MV therapy combined with bispecific killer engagers

NK cell degranulation and cytokine expression were monitored in response to MV therapy with subsequent BiKE treatment. (A) Schematic outline of combination therapy experiments. Colorectal cancer (KM 12, HT-29) or pancreatic ductal adenocarcinoma (HPAC) cell lines were inoculated with MV at MOI1. NK cells were added 24h post inoculation and co-incubated for 12 h, unless indicated otherwise. BiKEs were purified from supernatants of infected Vero producer cells (vBiKEs). Non infected target cells and vBiKEs were added to the coculture and incubated for 4 h. NK cell degranulation and cytokine expression were analyzed by flow cytometry. (B) NK cell degranulation (CD107a) in response to MeVac and vBiKE monotherapy or combination therapy is shown in exemplary histograms for one donor. (C) Percentages of CD107a⁺ NK cells, (D) IFNγ⁺ and TNFα⁺ NK cells in different conditions are summarized for n ≥ 4 donors per cell line with different symbols representing individual donors. (E) - (G) NK cell response to tumor cells infected with lab grade virus preparations and highly purified MV NIS. (E) CD69 expression on NK cells in coculture with infected HT 29 and KM12 cells was analyzed for lab grade MeVac and NSe and commercial MV NIS at designated time points post inoculation. MFI is shown for n = 2 donors (12 h) or n = 3 donors (36 h, 60 h). (F) After 36 h coculture with MeVac, NSe, or MV NIS infected tumor cells, *i.e.,* 60 h post inoculation, vBiKE treatment was performed, and NK cells were analyzed 4 h after treatment. Exemplary histograms and (G) percentages of NK cells positive for the degranulation marker CD107a are depicted. For MV NIS and mock infection, a second independent experiment is shown in Figure S3C-F, including cytokine expression analysis after vBiKE treatment.

### Figure 3:

### Oncolytic measles virus encoding BiKEs.

(A) Schematic of recombinant MV BiKE. (i) The BiKE transgene is encoded downstream of the P open reading frame within the MV genome. Hemagglutinin (HA) and hexa histidine (His6) tags enable purification and detection. Kozak and Igκ leader sequences mediate efficient translation and secretion. (G4S)3, glycine-serine peptide linker; HMA, human muscle aldolase linker; scFv, single chain variable fragment with variable heavy (VH) and variable light (VL) chain; TAA, tumor associated antigen. (ii) Transgene-encoding virus can be rescued via a reverse genetics system and triggers (iii) BiKE secretion upon infection. BiKEs included in this study either target human carcinoembryonic antigen (CEA) as model tumor antigen or human high molecular weight melanoma-associated antigen (HMWMAA) as a non-relevant control. (B) Replication kinetics of MV-BiKE. Vero producer cells were inoculated with MeVac P BiKE or unmodified MeVac at MOI 0.03. Viral progeny at designated time points were determined via serial dilution titration assay to obtain a multistep growth curve. (C) Direct cytotoxic effect of MV-BiKE. Cells were infected with respective viruses at MOI 0.03 for Vero (i) or at MOI 1 for colorectal cancer cell lines KM12 (ii) and HT 29 (iii). Cell viability was determined via XTT assay and normalized to mock infected control. (D) and (E) BiKE expression kinetics on infected colorectal cancer cells. KM12 and HT 29 cells were inoculated with MeVac P BiKE or MeVac at MOI1. Supernatants and cell lysates were collected at indicated time points. BiKE concentrations were determined via ELISA using anti His6 and anti HA antibodies and purified vBiKEs as standard. (D) Kinetics of BiKE concentrations in culture supematants after infection with MV BiKE. (E) Concentrations of cell associated BiKE in tumor cell lysates and BiKE release into culture supernatants. Error bars indicate SD.

### Figure 4

### Efficacy of MV BiKE immunovirotherapy against bystander colorectal cancer cells.

(A) Schematic outline of in vitro coculture experiments. MV BiKE infected colorectal cancer cells were cocultured with CFSE-labeled, non-infected tumor cells and IL 2 pre stimulated NK cells for 12 h. NK cell degranulation, cytokine expression and killing of CSFE⁺ target cells were analyzed by flow cytometry. (B) and (C) Efficacy of MV BiKE against KM12 and HT 29 colorectal cancer cells. Recombinant MV encoding the tumor-targeting CEAxCD16A BiKE are compared to mock infected control, MV without transgene and MV encoding an HMWMAAxCD16A control BiKE with irrelevant specificity. (B) Exemplary pseudocolor plots for KM12 depict CFSE+ target cell viability based on Zombie Violet labeling of dead cells. (C) Mean bystander target cell death per treatment is shown for n = 10 donors (KM12) and n = 4 donors (HT 29) with individual donors represented by different symbols. Statistical analysis was performed by one way ANOVA with Šidák multiple comparisons test. (D) and (E) NK cell degranulation and cytokine expression in response to MV BiKE therapy. (D) Exemplary histograms for KM12 target cells illustrate CD107a surface levels and intracellular cytokine accumulation. (E) Mean percentages of CD107a⁺, IFNγ⁺ and TNFα⁺ NK cells, respectively, are summarized for n = 4 donors (KM12) and n = 2 donors (HT 29) with individual donors represented by symbols as in (C).

### Figure 5

### NK cell characterization in response to MV or MV-BiKE therapy.

NK cell surface markers and IFNγ release were analyzed after coculture with infected colorectal cancer cells. MV BiKE infections were compared to NK cell only controls, mock or MV infection, IL 2 stimulation, and combination treatment with MV and purified vBiKEs (10 ng/µL). (A) Schematic outline of the experiment. (B) IFNγ concentrations in coculture supernatants were quantified by ELISA. Results are shown for KM12 and HT 29 tumor cells with technical triplicates for n = 2 donors, represented by different symbols. (C) Characterization of NK cell surface markers by flow cytometry. Exemplary histograms from one donor are depicted including Fluorescence Minus One + Isotype (FMO I) controls for each marker. Mean fluorescence intensity (MFI) or the percentage of cells expressing the respective marker are summarized for n = 2 donors. Different symbols represent individual donors. For CD16, three technical replicates are included per donor. For additional data on MV-BiKE conditions compared to mock infected controls, refer to Figure S5.

### Figure 6

### MV BiKE efficacy against patient derived pancreatic cancer cultures.

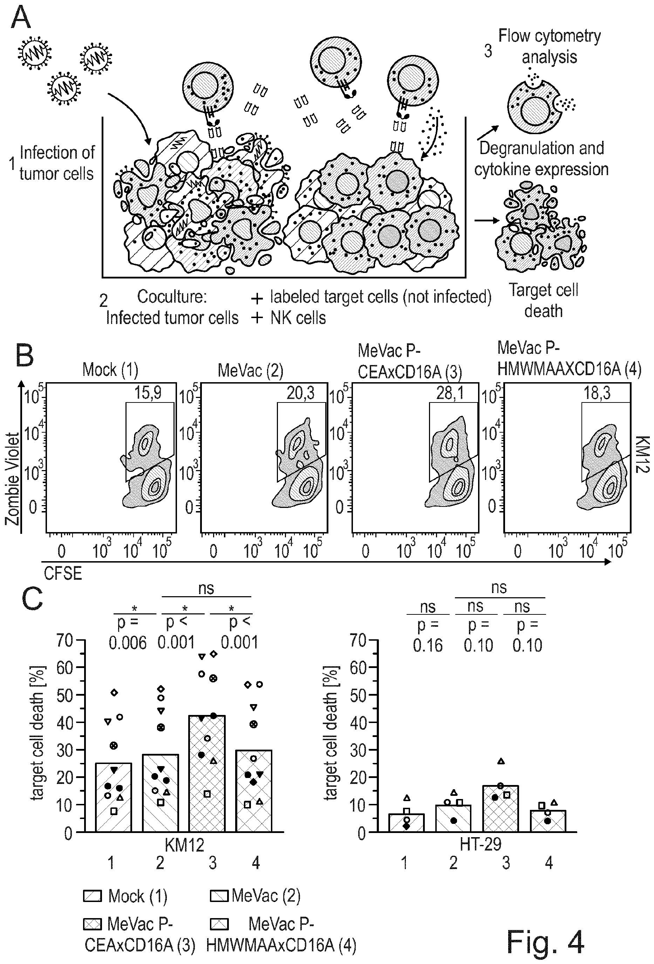

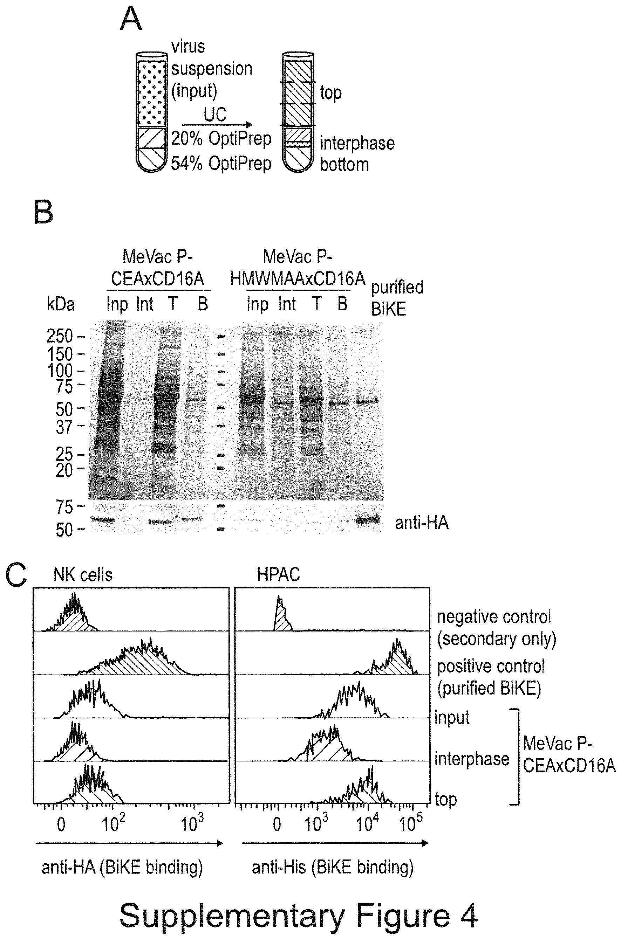

(A) BiKE binding to pancreatic cancer cultures. BiKEs were purified from supernatants of Vero producer cells infected with respective viruses (vBiKEs). Cancer cells were incubated with CEAxCD16A vBiKEs or the HMWMAAxCD16A control. Binding was assessed via flow cytometry based on BiKE labeling with anti His6 antibodies. Histograms for PC43 and PC17 are depicted. (B) and (C) NK cell cytotoxicity upon vBiKE treatment. Allogenic, IL 2 pre stimulated NK cells were co incubated with CFSE labeled PC43 or PC17 cultures (E:T = 5:1) in presence of vBiKEs (5 ng/µL) for 12 h with subsequent analysis of target cell killing via flow cytometry. (B) Pseudocolor plots exemplarily illustrate gating and CFSE⁺ tumor cell viability for PC17 based on Zombie Violet staining of dead cells. (C) Target cell death for n = 2 donors with technical duplicates per donor is shown. (D)-(G) NK cell activity upon infection of patient derived cultures with MV BiKE. Infected cancer cells were cocultured with CFSE-labeled, non-infected target cells and allogenic, IL 2 pre stimulated NK cells for 12 h (compare Figure 4A). NK cell degranulation, cytokine expression, and target cell killing were analyzed by flow cytometry. (D) NK cell degranulation (CD107a) and intracellular IFNγ accumulation in response to MV-BiKE therapy are depicted in exemplary histograms. (E) Mean percentages of CD107a⁺ and IFNγ⁺ NK cells are shown for n = 5 donors (CD107a, left) and n = 3 donors (IFNγ, right) with different symbols representing individual donors. (F) Pseudocolor plots display the viability of non-infected CFSE⁺ target cells in coculture with respective MV BiKE infected cancer cells and NK cells. (G) Mean percentages of target cell death in designated cocultures are summarized for n = 5 donors (PC43, top) and n = 7 donors (PC17, bottom) with donors represented by symbols as in (E).

### SUPPLEMENTARY FIGURES

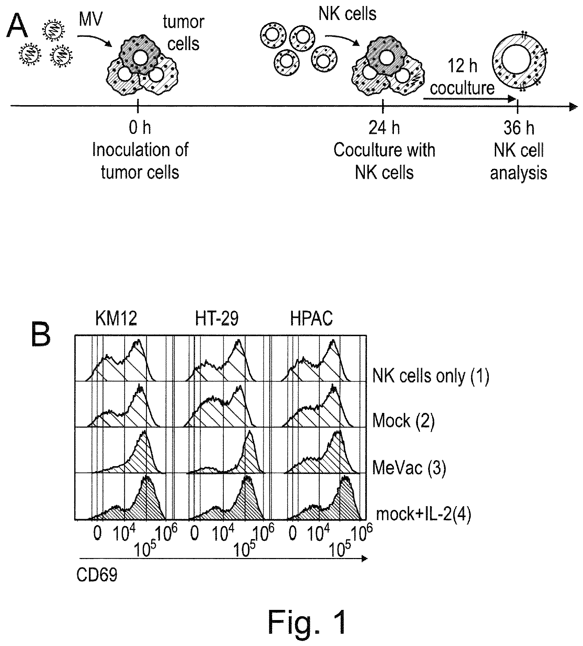

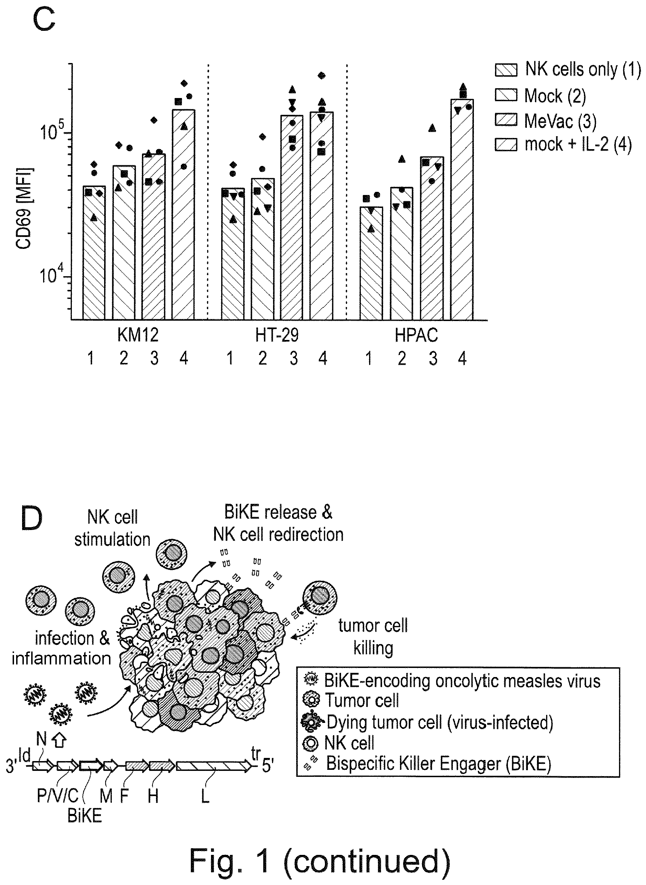

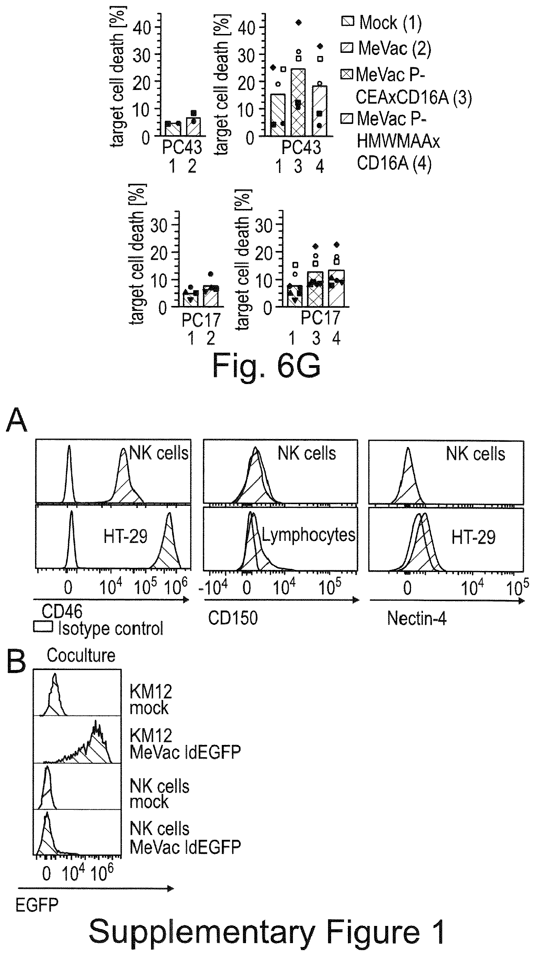

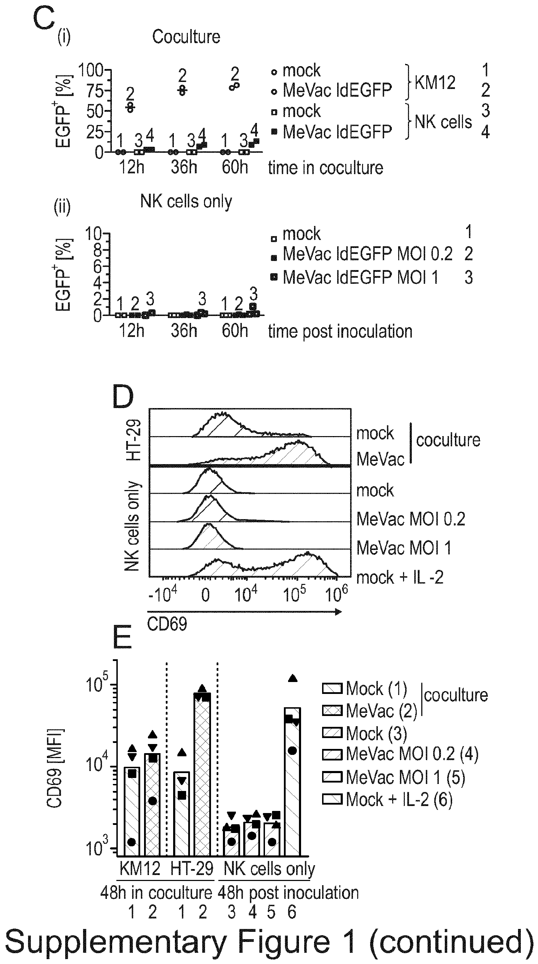

### Supplementary Figure 1 (S1)

### NK cell infection and stimulation in MV therapy

(A) Expression of MV entry receptors CD46, CD150 (SLAM) and Nectin 4 on NK cells purified from healthy donor PBMCs was analyzed by flow cytometry. Lymphocytes, gated from PBMCs based on FSC/SSC signals, and HT 29 tumor cells are depicted as controls. Representative histograms for n = 2 donors are shown. (B) - (E) KM12 tumor cells were inoculated with MeVac ldEGFP or MeVac at MOI1. After 24 h, NK cells were either added to the infected cells, or inoculated directly with MV at MOI 0.2 and MOI 1, respectively. EGFP expression in live (Zombie Violet-) KM12 and NK cells as well as CD69 levels on NK cells were analyzed by flow cytometry at designated time points. (B) Exemplary histograms after 60 h coculture and (C) percentages of EGFP⁺ tumor and NK cells in comparison to mock infected conditions for n ≥ 2 donors are shown. (D) Histograms depict CD69 expression for one exemplary donor after 48 h coculture with HT 29 or 48 h post inoculation for NK cell only conditions. (E) CD69 MFI data for n = 4 donors from two experiments are summarized.

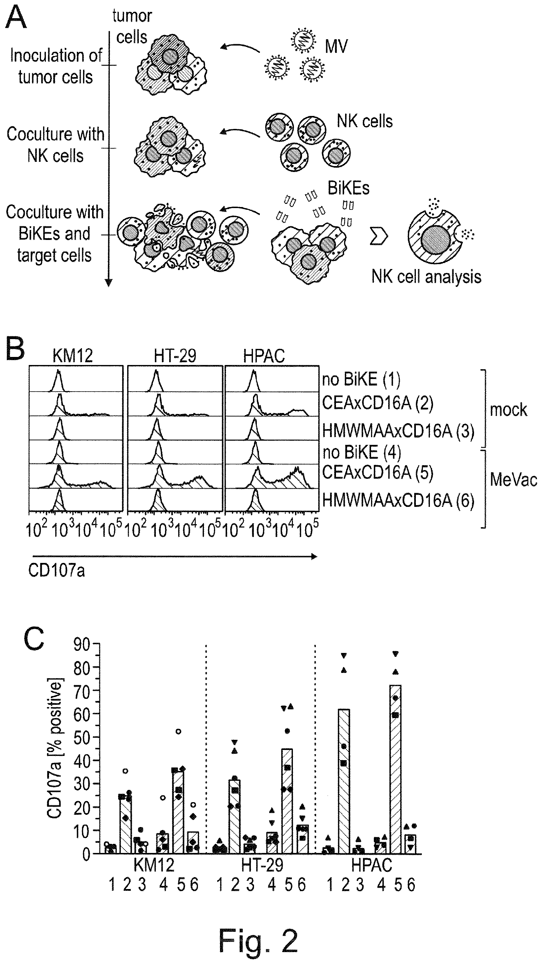

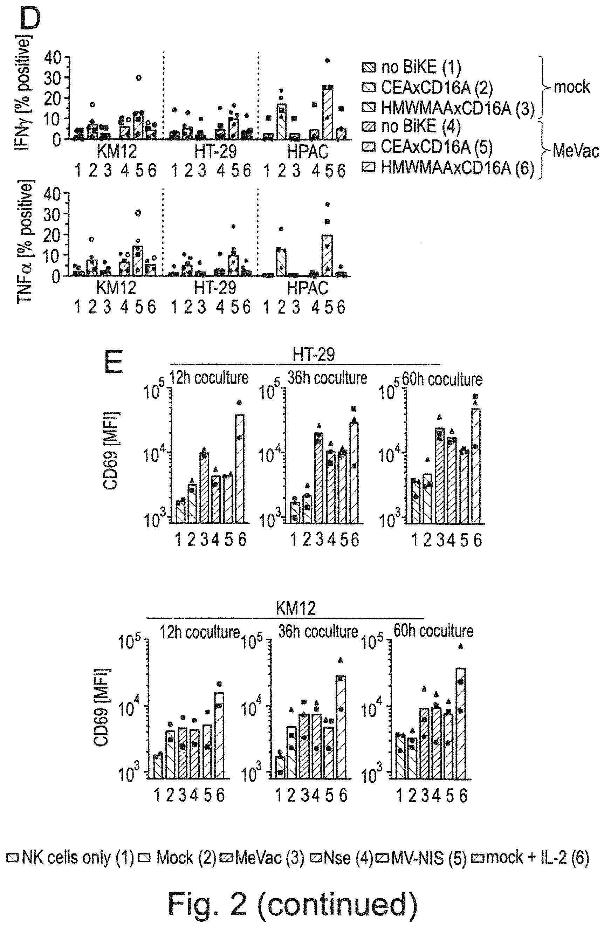

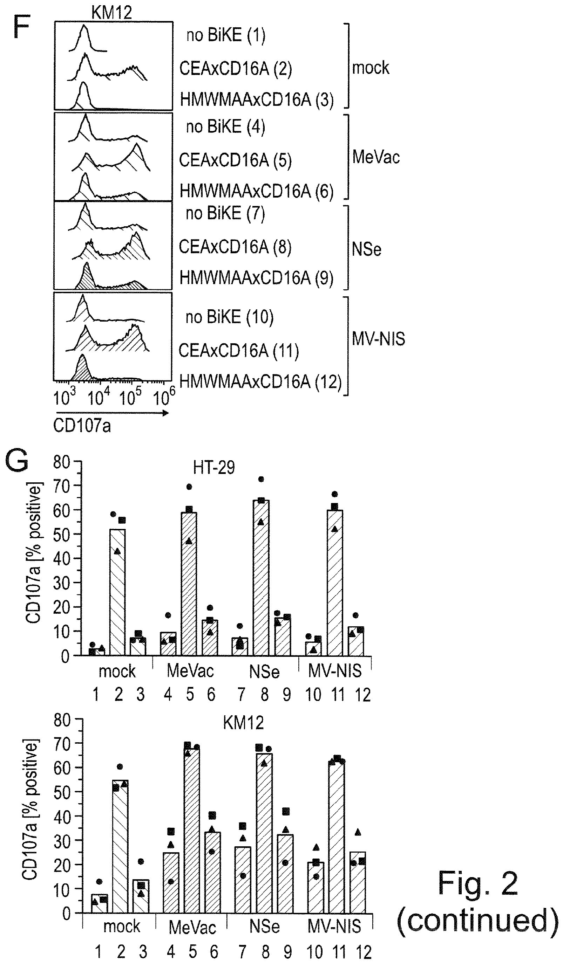

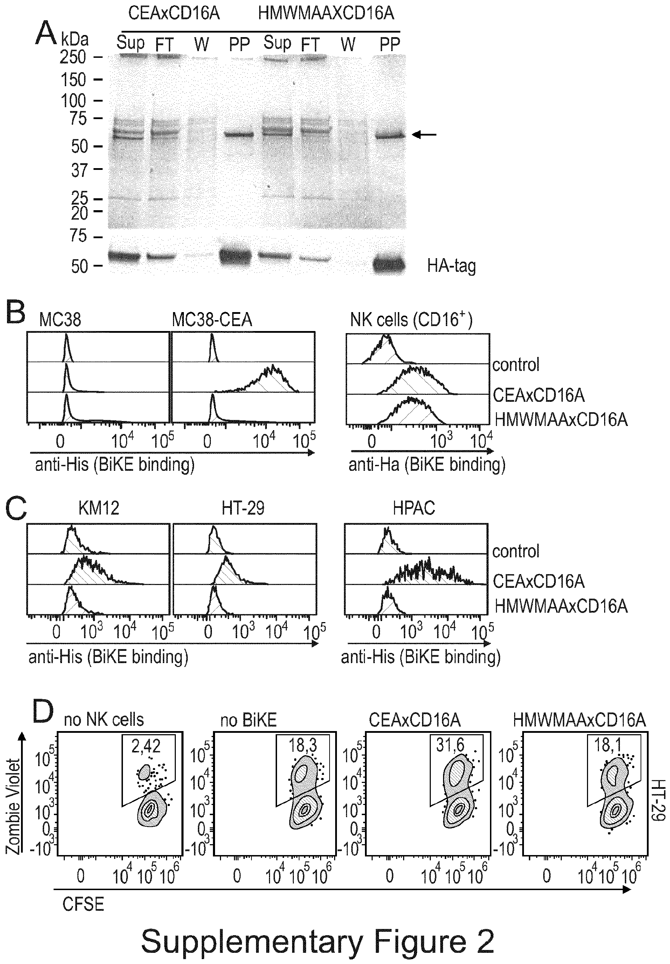

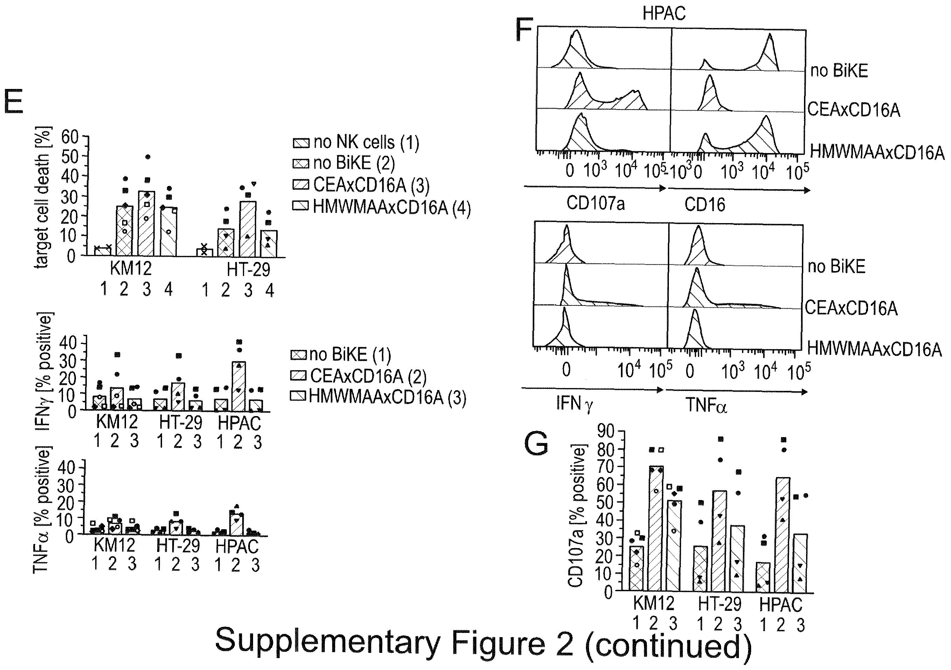

### Supplementary Figure 2 (S2)

### Functionality of MV-encoded bispecific killer engagers

(A) Purification of BiKE proteins. Supernatants of MV-BiKE infected Vero cells cultured in serum-free medium were collected for purification of the respective CEA or HMWMAA-targeting BiKE (vBiKE). Proteins were purified by affinity exchange chromatography via the His6 tag. Fractions were analyzed by polyacrylamide gel electrophoresis. Equal volumes were loaded except for the purified protein samples, which were adjusted to 2 µg total protein. Stain-free imaging of the gel (top) was performed prior to western blotting (bottom). BiKE proteins were detected via anti HA biotin and streptavidin HRP. Sup, supernatant of MV-BiKE infected cells; FT, column flow-through; W, wash fraction; PP, purified protein. Arrow indicates BiKE protein bands (57.5 kDa). (B) BiKE binding to CEA expressing MC38 cells, CD16⁺ NK cells and (C) colorectal cancer (KM12, HT 29) or pancreatic ductal adenocarcinoma (HPAC) cell lines. Cells were incubated with respective vBiKEs and analyzed by flow cytometry. Antibodies specific for the His6 or HA tag were used to detect BiKE binding to CEA on tumor cells or to CD16 on NK cells, respectively. (D) and (E) Efficacy of vBiKEs against colorectal cancer cells. CFSE labeled tumor cells were cocultured with NK cells (E:T = 5:1) and vBiKEs (10 ng/uL). Target cell-specific CEAxCD16A BiKEs are compared to HMWMAAxCD16A BiKEs with irrelevant specificity. Target cell killing was analyzed by flow cytometry based on Zombie Violet staining. (D) Exemplary pseudocolor plots for HT 29 illustrate separation of live and dead CFSE⁺ tumor cell populations. (E) Mean target cell death is shown for n ≥ 4 donors per cell line with different symbols to discriminate individual donors. (F) and (G) NK cell effector functions in response to vBiKE. (F) Exemplary histograms for HPAC target cells illustrate NK cell degranulation (CD107a), CD16 surface levels and intracellular cytokine accumulation (IFNγ and TNFα). (G) Mean percentages of CD107a⁺, IPNγ⁺ and TNFα⁺ NK cells are summarized for n ≥ 4 donors per cell line with symbols as in (E).

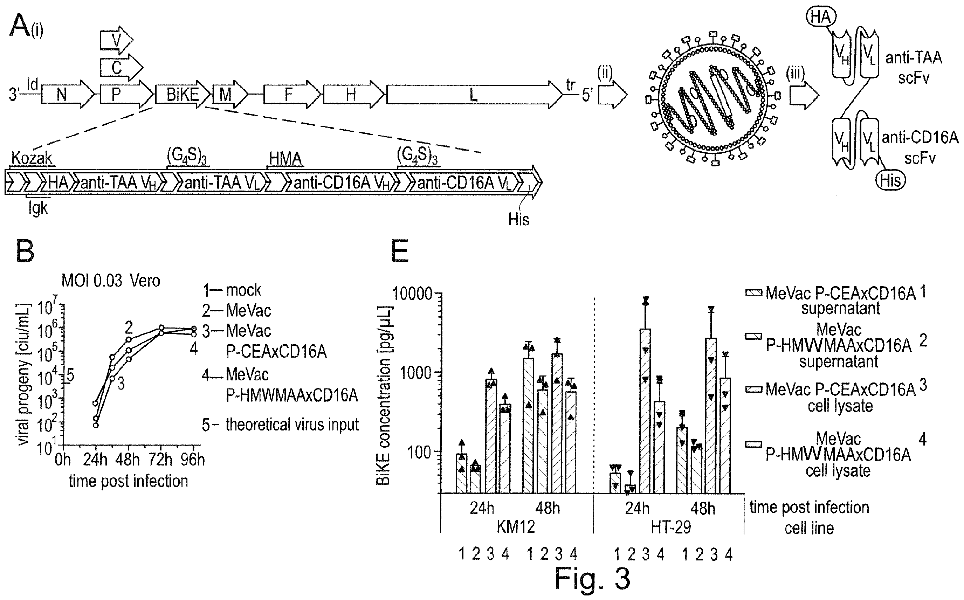

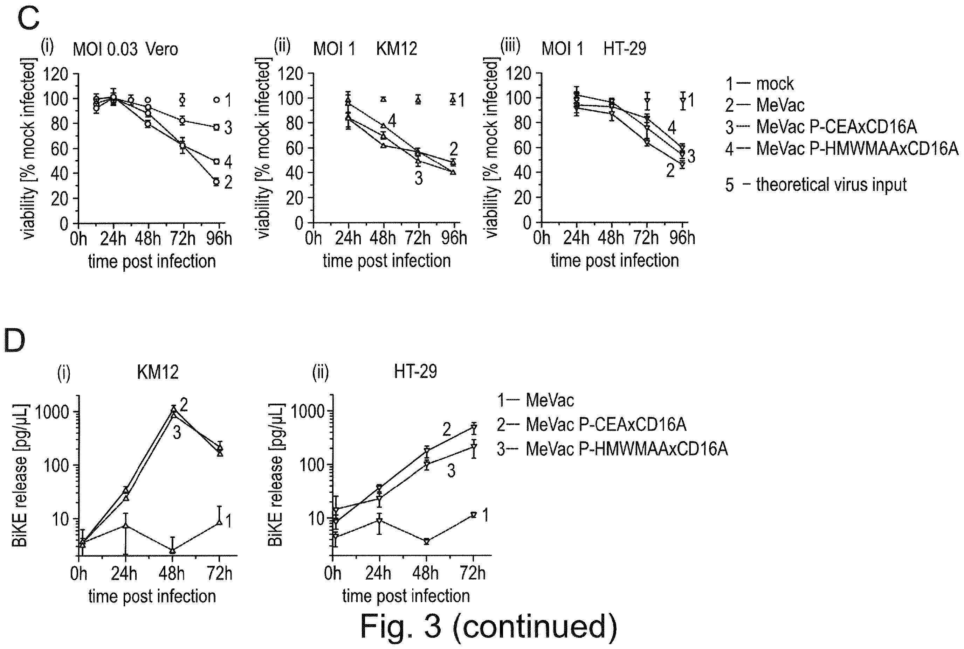

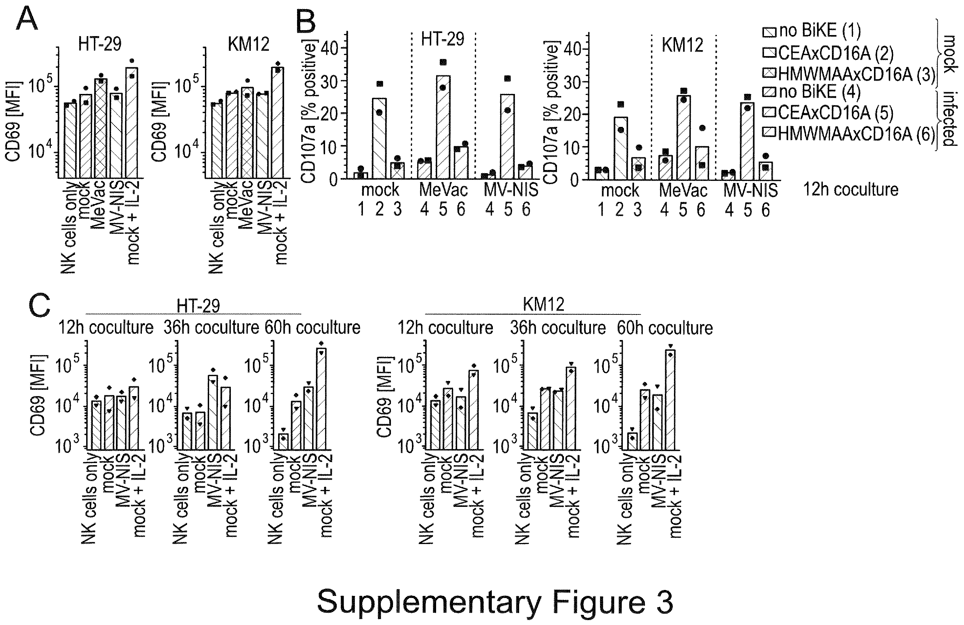

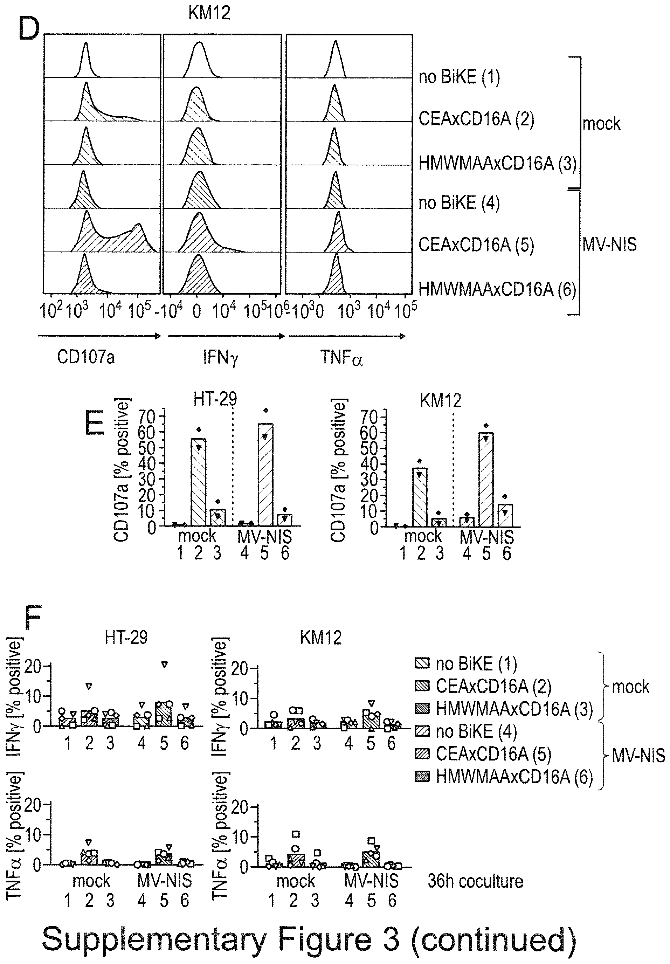

### Supplementary Figure 3 (S3)

### NK cell activation and degranulation in MV + BiKE combination therapy

HT 29 and KM12 colorectal cancer cells were inoculated with lab grade MeVac and commercial MV NIS at MOI1. NK cells were added 24 h post inoculation. Firstly, CD69 expression on NK cells after exposure to the infected cells was analyzed at different time points post inoculation. Secondly, non-infected target cells and vBiKEs were added to the coculture at designated time points and incubated for 4 h until NK cell **degranulation** was analyzed. (A) CD69 MFI on NK cells was analyzed after 12 h co incubation with infected HT 29 or KM12, *i.e.,* 36 h post inoculation. (B) Quantification of NK cell degranulation is shown for vBiKE treatment combined with the exposure to infected HT 29 and KM12. (C) NK cells were co incubated with HT 29 or KM12 cells infected with MV-NIS or subjected to mock infection. CD69 expression was analyzed at multiple time points as indicated. (D) - (F) After 36 h coculture, *i.e*., 60 h post inoculation, vBiKE treatment was performed and the percentages of CD107a⁺, IFNγ⁺, and TNFα⁺ NK cells were determined 4 h after treatment. (D) Exemplary histograms, (E) percentages of NK cells positive for the degranulation marker CD107a for n = 2 donors, and (F) cytokine expression for n = 5 donors are shown.

Data for mock and MeVac conditions displayed in (A) are also included in the summary bar plots in Figure 1C and Figure 2C, respectively. Data in (C) - (E) are from an independent experiment with two donors analogous to Figure 2E - G. In (F), data from both experiments, *i.e*., a total of five donors, are summarized with symbols for individual donors as in Figure 2G and S3E.

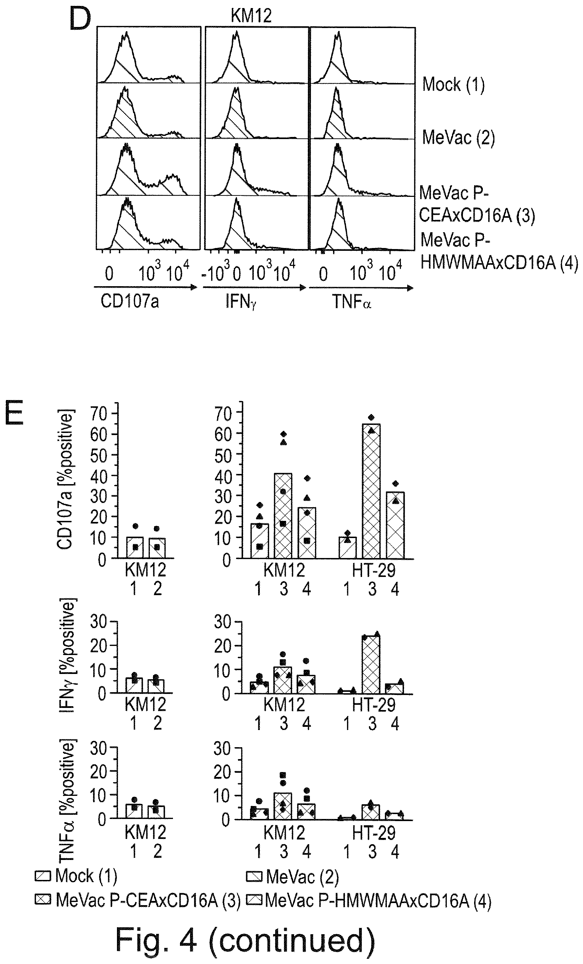

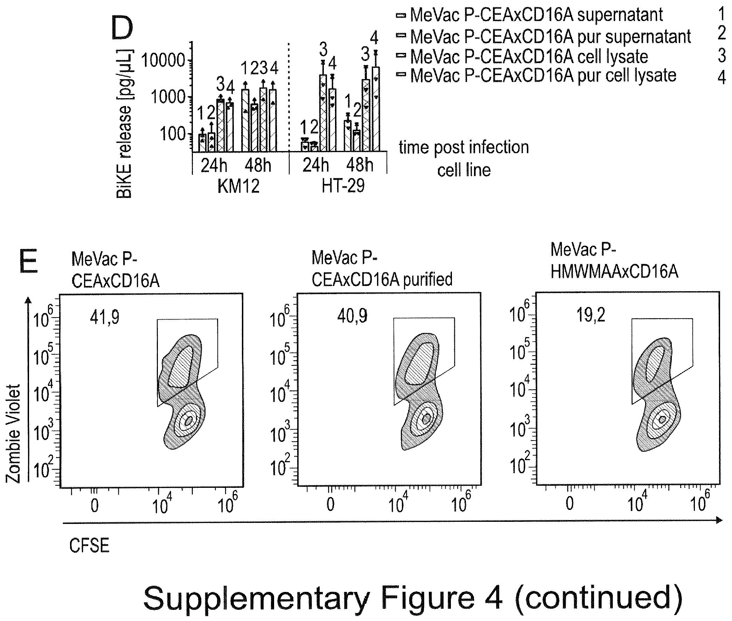

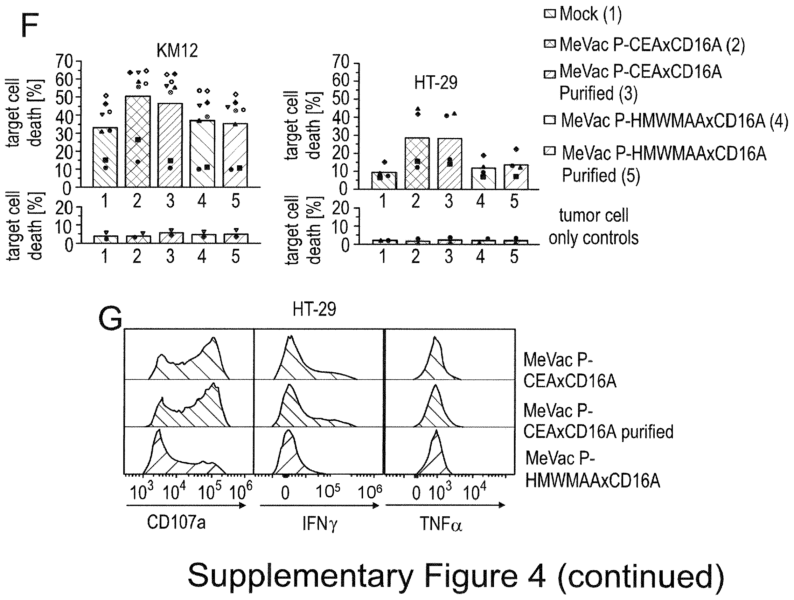

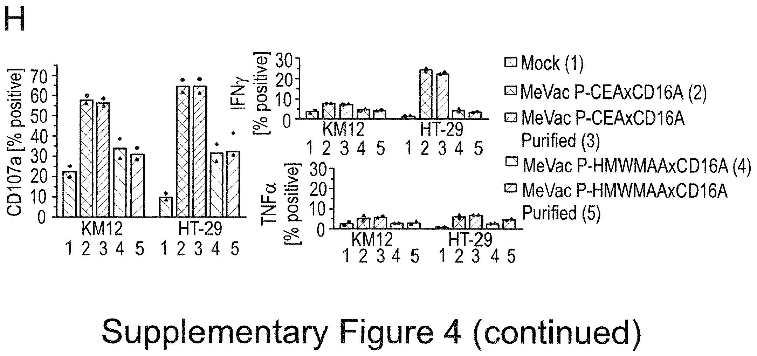

### Supplementary Figure 4 (S4)

### Efficacy of immunovirotherapy with MV BiKE purified via ultracentrifugation.

(A) Schematic representation of MV purification via OptiPrep fractionation ultracentrifugation (UC). The virus suspension was layered onto 54% and 20% OptiPrep phases and, following ultracentrifugation, the interphase containing purified viruses was. In addition, the bottom and top fractions were kept for analysis. (B) Total protein and BiKE levels in respective samples were analyzed by polyacrylamide gel electrophoresis, stain free imaging of the gel (top) and western blot (bottom). For the input virus suspension (Inp), purified virus interphase (Int) and bottom fraction (B), 5 × 104 ciu were loaded per lane. For the top fraction (T), the same sample volume as for the input was loaded for direct comparison. Purified CEAxCD16A vBiKE (1 µg) served as a control. The HA tagged BiKE proteins were detected with anti HA biotin and streptavidin HRP. (C) CEAxCD16A BiKE binding to CD16⁺ NK cells and HPAC tumor cells after incubation with designated fractions from the purification was assessed by flow cytometry. For the input and interphase fractions, volumes corresponding to 5×10⁴ ciu were used for incubation. For the top fraction, the same volume as for the input was applied. BiKE binding to CD16 on NK cells and to CEA on cancer cells was detected with anti HA and anti His6 antibodies, respectively. (D) BiKE expression after infection with purified virus. KM 12 and HT 29 cells were inoculated with conventional lab grade MeVac P CEAxCD16A compared to UC purified virus at MOI1. Supernatants and cell lysates were collected at indicated time points. BiKE concentrations were determined via ELISA with purified vBiKEs as standard. Error bars indicate SD. (E) - (H) Efficacy of purified MV BiKE in comparison to lab grade counterparts. MV BiKE infected colorectal cancer cells were cocultured with CFSE-labeled, non-infected tumor cells and IL 2 pre stimulated NK cells for 12 h. NK cell degranulation, cytokine expression and killing of CSFE⁺ target cells were analyzed by flow cytometry (compare Figure 4A). (E) Exemplary pseudocolor plots for HT 29 depict CFSE+ target cell viability. (F) Mean bystander target cell death per treatment is shown for n = 8 donors (KM12, left) and n = 4 donors (HT 29, right) with individual donors represented by different symbols. Tumor cell only controls without NK effector cells are shown below the respective bar plots. (G) Exemplary histograms depict NK cell degranulation (CD107a) and intracellular cytokine accumulation for HT 29 target cells. (H) Mean percentages of CD107a⁺, IFNγ⁺ and TNFα⁺ NK cells are summarized for n = 2 donors per cell line with symbols for individual donors as in (F).

Data for mock and the non-purified MeVac P-BiKE conditions is also included in the summary bar plots in Figure 4C and displayed again for comparison.

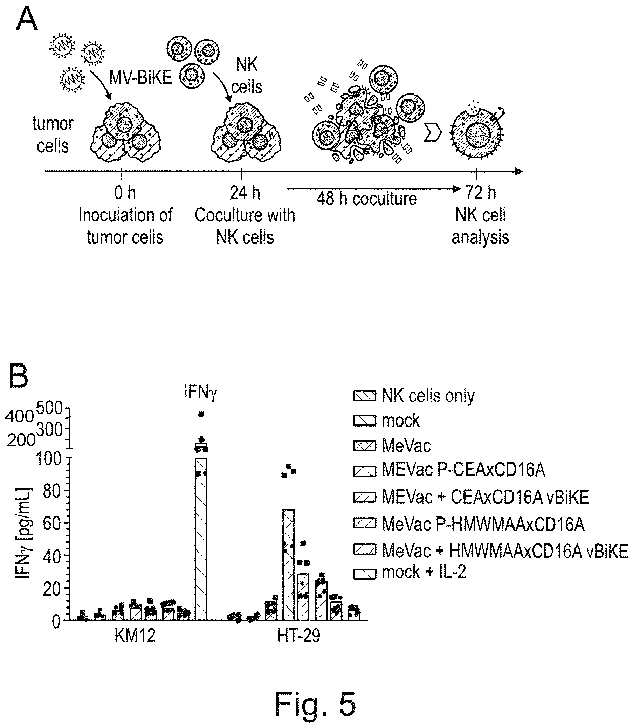

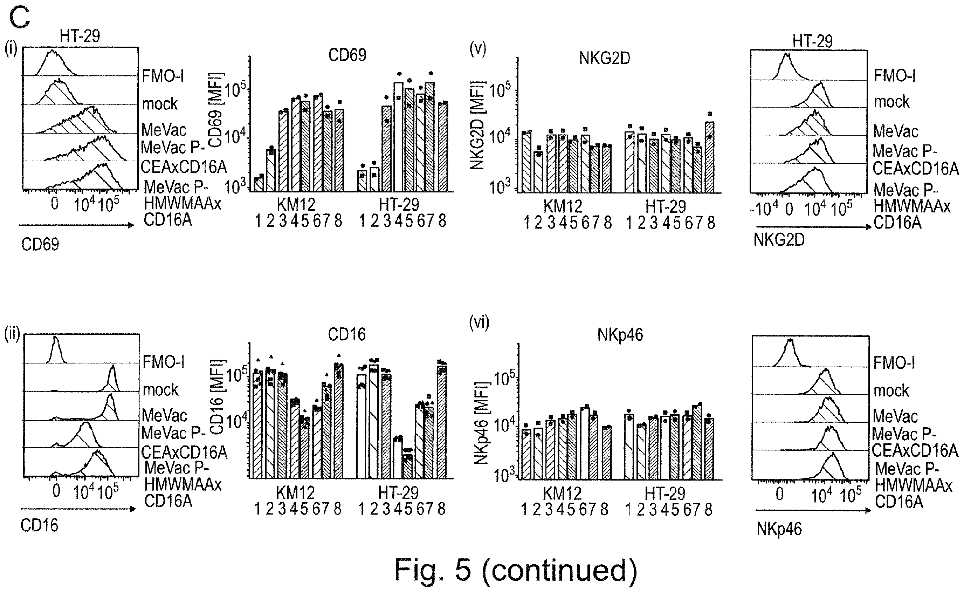

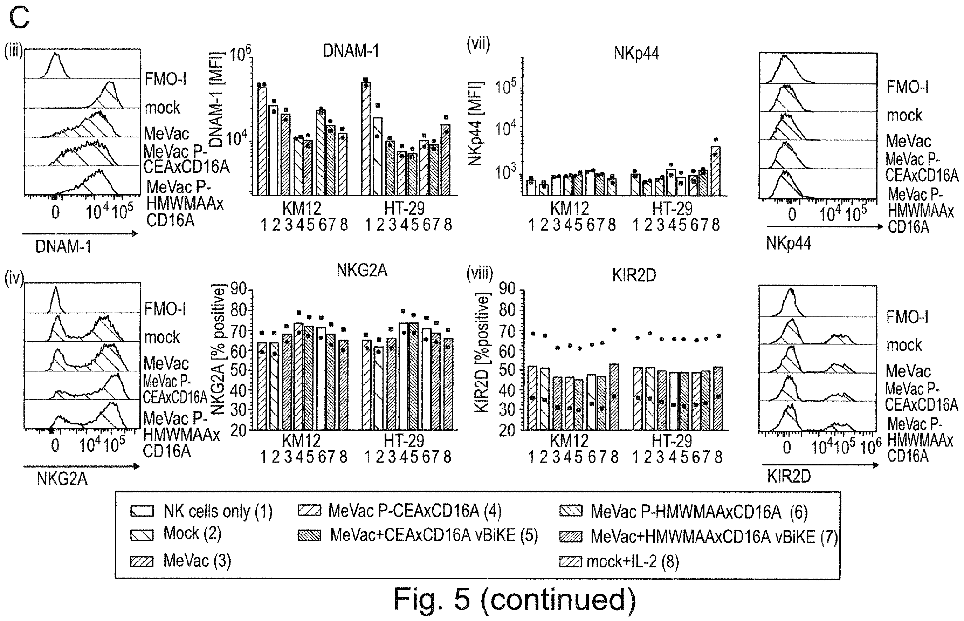

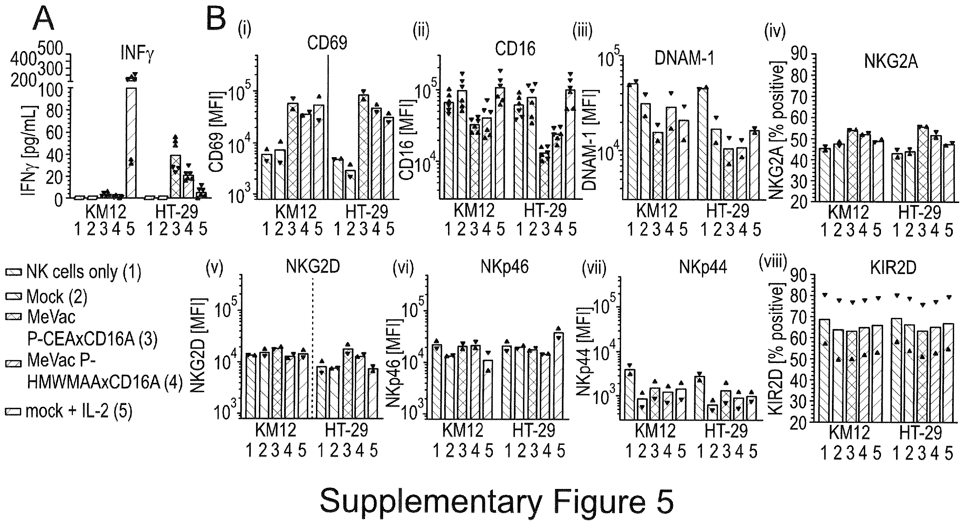

### Supplementary Figure 5 (S5)

### NK cell characterization in response to MV-BiKE therapy.

The experiment depicted in Figure 5 was repeated with n = 2 donors for selected conditions: IFNγ release and NK cell surface markers were analyzed after 48 h coculture with infected colorectal cancer cells (compare Figure 5A). MV BiKE infections were compared to NK cell only controls, mock infection, and IL 2 stimulation. (A) IFNγ concentrations in coculture supernatants were quantified by ELISA. Results are shown for KM12 and HT 29 tumor cells with technical triplicates for n = 2 donors, represented by different symbols. (B) NK cell surface markers were characterized by flow cytometry. Mean fluorescence intensity (MFI) or the percentage of cells expressing the respective marker are summarized for n = 2 donors. Different symbols represent individual donors. For CD16, three technical replicates are included per donor.

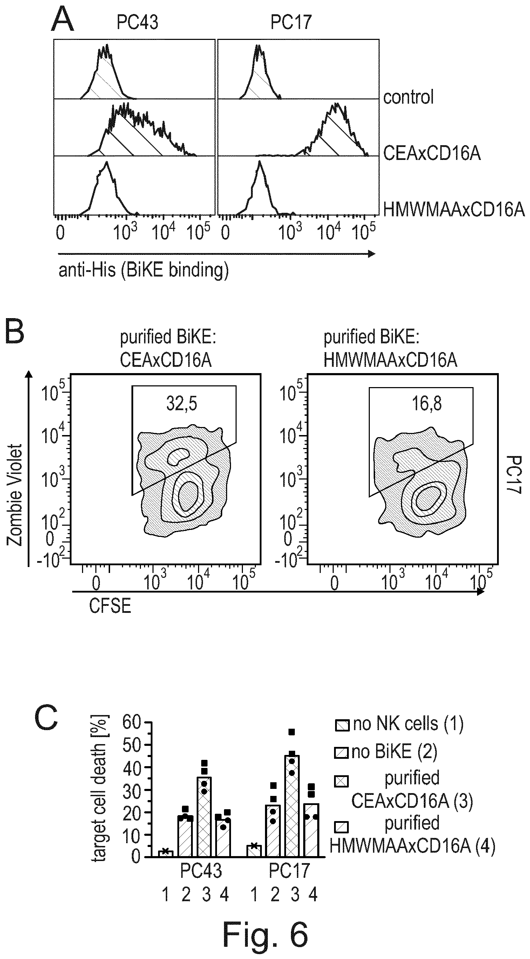

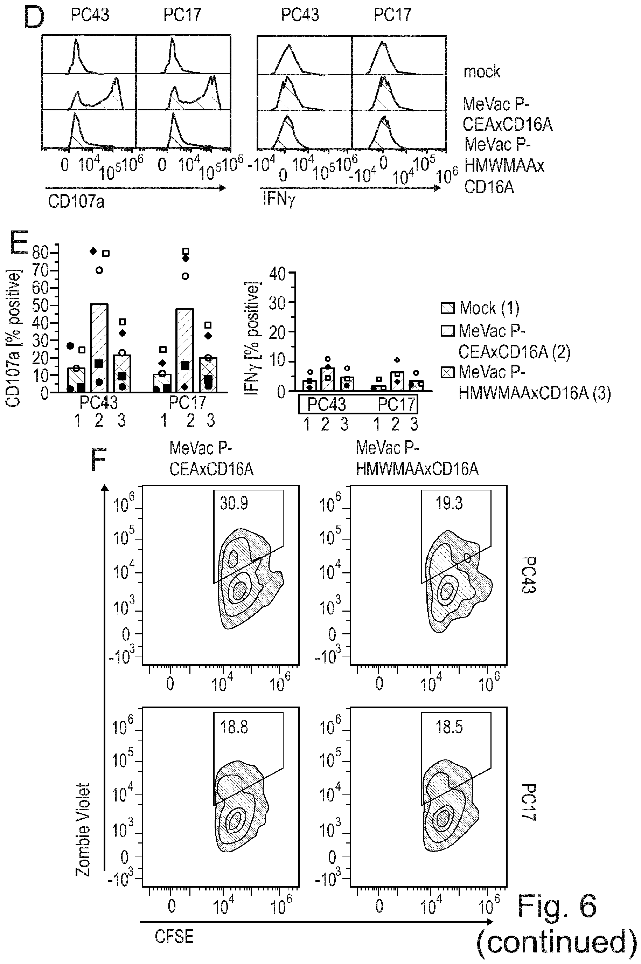

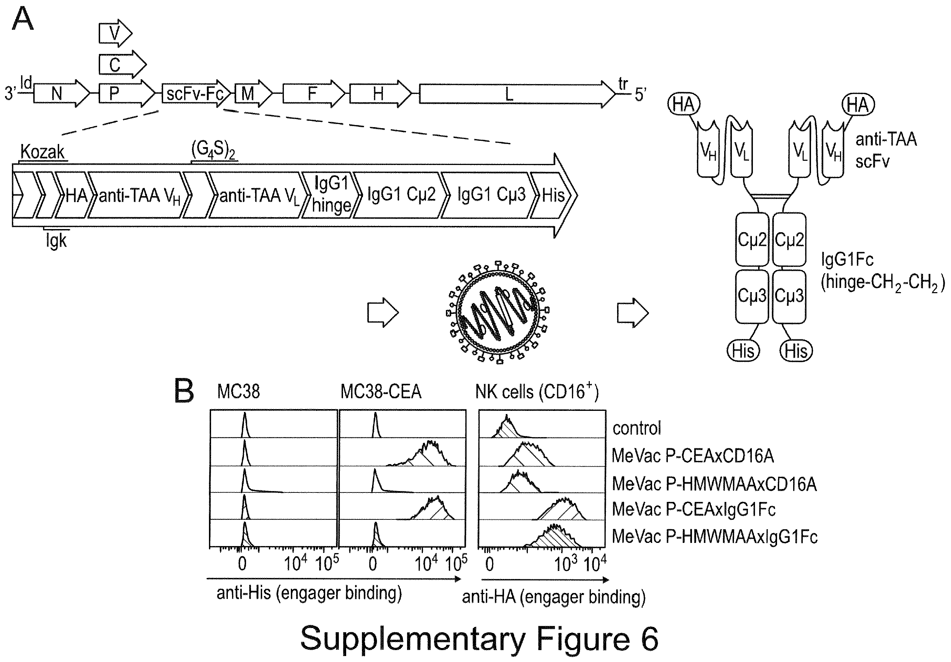

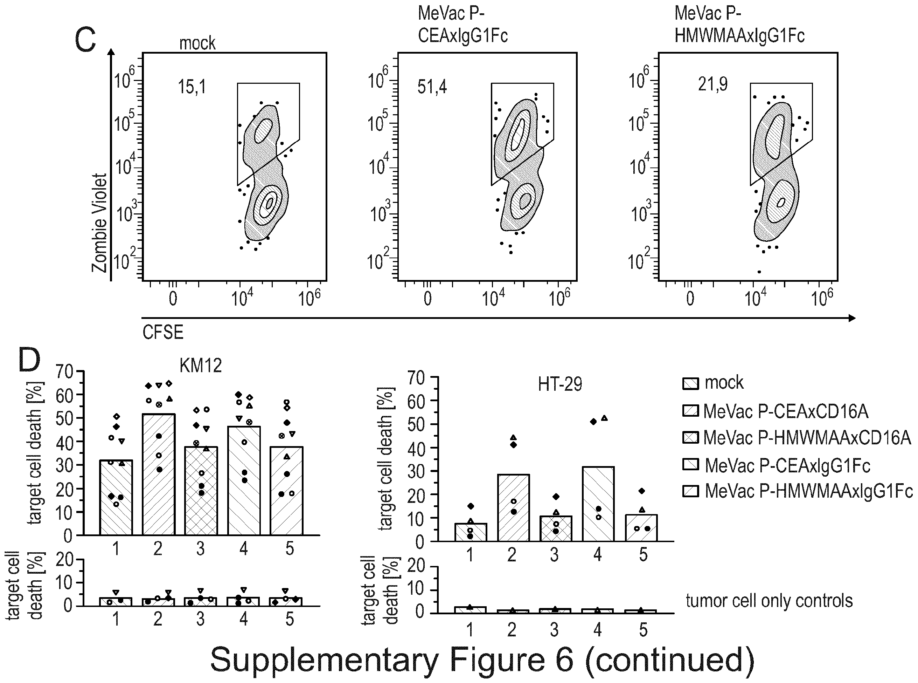

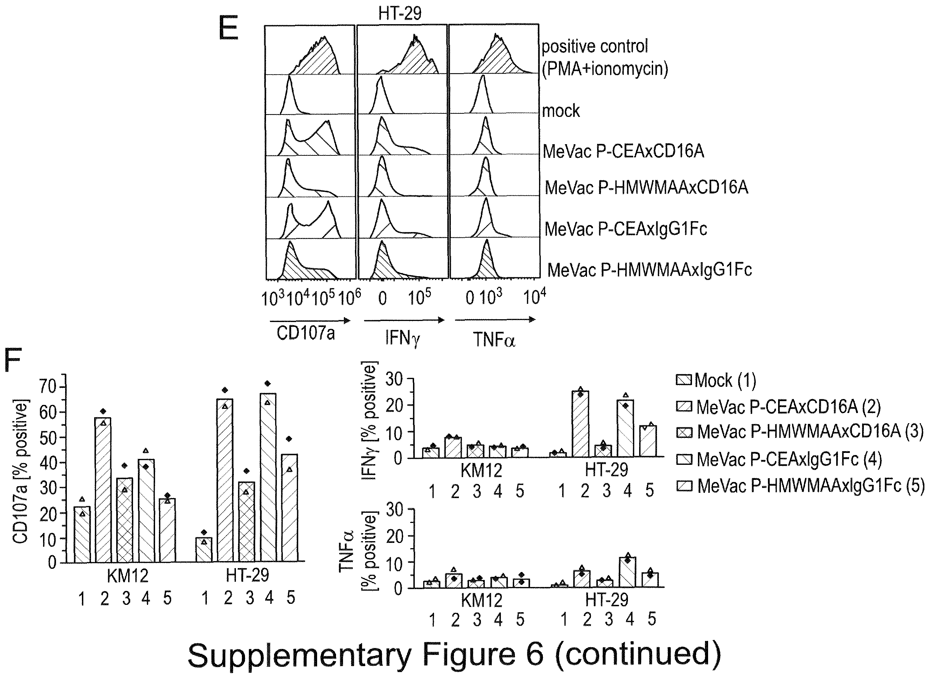

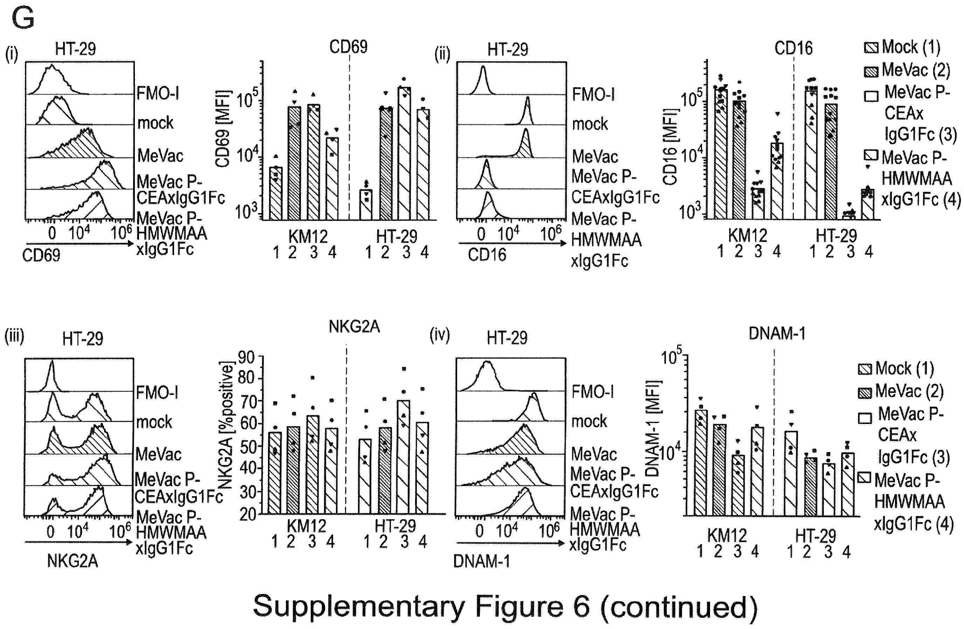

### Supplementary Figure 6 (S6)

### Comparison of MV encoded BiKEs and scFv-Fcs for NK cell engagement.

(A) Schematic of the recombinant MV encoding an scFv Fc protein. Analogous to MV BiKE, the transgene is encoded downstream of the P open reading frame, with Hemagglutinin (HA) and hexa histidine (His6) tags included for detection as well as Kozak and IgK leader sequences added for translation and secretion. The transgene-encoding virus can be rescued via a reverse genetics system and results in release of dimerized scFv-Fc proteins from infected cells. CH, constant heavy chain; (G4S)₃, glycine-serine peptide linker; scFv, single chain variable fragment with variable heavy (VH) and variable light (VL) chain; TAA, tumor associated antigen. (B) Binding of MV-encoded scFv-Fc to tumor and effector cells. Cells were incubated with respective virus suspensions and analyzed by flow cytometry. Antibodies specific for the His6 or HA tag were used to detect BiKE binding to CEA on tumor cells or to Fc receptors on NK cells, respectively. For MC38 and MC38 CEA, controls were stained with anti His6 antibody only. For NK cells, MeVac without transgene with subsequent anti HA antibody staining was used as control. (C) - (F) Efficacy of MV scFv Fc in comparison to MV BiKE and mock infected control. Infected colorectal cancer cells were co incubated with non-infected, CFSE-labeled tumor cells and IL 2 pre stimulated NK cells for 12 h. Killing of CSFE⁺ target cells, NK cell **degranulation** and cytokine expression were analyzed by flow cytometry (compare Figure 4A). (C) Pseudocolor plots for one exemplary donor with HT 29 target cells illustrate CFSE⁺ tumor cell viability. (D) Target cell death is summarized for n = 9 donors (KM12, left) and n = 4 donors (HT 29, right) for each treatment with symbols representing individual donors. Controls without NK effector cells are shown below. (E) Histograms depict NK cell degranulation (CD107a) and intracellular cytokine accumulation (IFNγ, TNFα) for HT 29 target cells treated with CEA targeting MV-BiKE or MV scFv Fc and respective controls. (F) Mean percentages of CD107a⁺, IFNγ⁺ and TNFα⁺ NK cells are shown for n = 2 donors per cell line with donors represented by symbols as in (D). Data for mock and the MeVac P-BiKE conditions is also included in the summary bar plots in Figure 4C and Figure S4 and displayed again for comparison. (G) Characterization of NK cell surface markers in response to MV scFv Fc treatment. Tumor cells were inoculated at MOI 1 one day prior to coculture. Freshly isolated NK cells were co incubated with infected cells for 48 h and surface markers on NK cells were analyzed by flow cytometry. For each marker, exemplary histograms (left) and a summary of either the MFIs or the percentage of cells expressing the respective marker (right) are shown for n = 4 donors. Different symbols represent individual donors. For CD16, three technical replicates are included per donor. FMO I, Fluorescence Minus One + Isotype control.

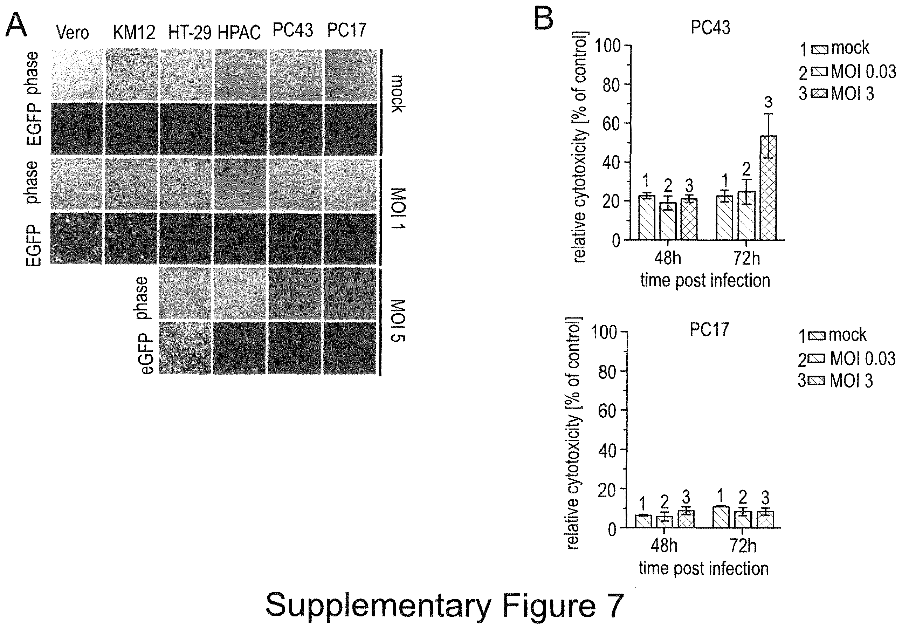

### Supplementary Figure 7 (S7)

### Cancer cell sensitivity towards MV infection.

Sensitivity of patient-derived pancreatic cancer short term cultures (PC43, PC17) towards MV infection in comparison to Vero producer cells and colorectal cancer (KM12, HT 29) or pancreatic adenocarcinoma (HPAC) cell lines. (A) Cells were inoculated with MeVac ldEGFP at indicated MOIs. Images were acquired 36 h post infection. Scale bar, 200 µm (B) Patient-derived cultures were inoculated with highly purified MV NIS at MOI 0.03 and MOI 3, or mock infected with medium only. Relative cytotoxicity was calculated based on LDH release.

### EXAMPLES

### MATERIALS AND METHODS

### Generation of MV-BiKE and MV-scFv-Fc vectors

BiKEs were constructed based on an affinity maturated, humanized anti-hCEA scFv (SM3E) (Graff *et al,* 2004; Bajic *et al,* 2020) and the affinity maturated anti-CD16A scFv (LSIV21) (Reusch *et al,* 2014). This CD16A-targeting scFv is employed in engager formats tested in clinical trials (Bartlett *et al,* 2020; NCT04259450), and Fc receptor binding is only marginally impaired by serum IgG (Wingert *et al,* 2021). Reverse translated nucleotide sequences were codon optimized for expression in *homo sapiens* using the GENEius algorithm (Eurofins Genomics). Variable heavy (V_{H}) and light (V_{L}) chains within each scFv were connected by artificial (Gly₄Ser)₃ peptide linkers. An HMA linker (PSGQAGAAASESLFVSNHAY (SEQ ID NO:7) was inserted between the N-tenninal anti-hCEA scFv and the C-terminal anti-CD16A scFv. Influenza virus hemagglutinin (HA) and hexa-histidine (His₆) tags were fused to the N- and C-terminus of the BiKE sequence, respectively. The BiKE sequence was preceded by a 5' Kozak sequence and an Igκ leader sequence (compare GenBank: AB050084.1). After synthesis of the BiKE cassette (Eurofins Genomics), the 1644 bp transgene fulfilling the *rule of six* was inserted into a Schwarz vaccine strain-derived MV full length genome with an additional transcription unit downstream of the phosphoprotein open reading frame (MeVac P-ATU) (Veinalde *et al,* 2017). To generate HMWMAA-targeting control constructs, the anti-hCEA scFv was replaced by an anti-hHMWMAA scFv (RAFT3) (Kang *et al,* 2000; Kaufmann *et al,* 2013). To obtain scFv-Fc constructs, the anti-CD16A scFv in respective BiKE sequences was replaced by human IgG1Fc (hinge-CH₂-CH₃) (Engeland *et al,* 2014), eliminating the HMA linker. Sequences were validated by Sanger sequencing (Eurofins Genomics).
SM3E V_{H}: QVKLEQSGAEVVKPGASVKLSCKASGFNIKDSYMHWLRQGPGQCLEWIG WIDPENGDTEYAPKFQGKATFTTDTSANTAYLGLSSLRPEDTAVYYCNEGTPTGPYY FDYWGQGTLVTVSS (SEQ ID NO:8)
SM3E V_{L}: ENVLTQSPSSMSVSVGDRVTIACSASSSVPYMHWLQQKPGKSPKLLIYLTS NLASGVPSRFSGSGSGTDYSLTISSVQPEDAATYYCQQRSSYPLTFGCGTKLEIK (SEQ ID NO:9)

### Cell culture

Cell lines, patient-derived cultures and NK cells were cultivated at 37°C and 5% CO₂ in humidified incubators. Cultures were routinely tested to exclude mycoplasma contamination.

### Cell lines

Vero cells were obtained from the American Type Culture Collection (ATCC, CCL-81). KM12 and HT-29 cells were obtained from Christoph Plass, DKFZ Heidelberg. HPAC cells were obtained from Daniel Abate-Daga, Moffitt Cancer Center.

Vero and KM12 cell lines were cultivated in Dulbecco's modified Eagle's medium (DMEM, Thermo Fisher Scientific and PAN Biotech) supplemented with 10% (v/v) fetal calf serum (FCS, PAN Biotech). HT-29 and HPAC cell lines were cultivated in Rosewell Park Memorial Institute (RPMI) 1640 medium (Thermo Fisher Scientific and PAN Biotech) with 10% (v/v) FCS.

### Patient-derived pancreatic cancer cultures

Patient-derived pancreatic cancer cultures PC17 and PC43 were generated and cultivated as described previously (Ehrenberg *et al,* 2019). Tumor samples were obtained with informed consent in accordance with the Declaration of Helsinki. Cultures were subjected to SNP typing and Multiplex Cell Contamination Testing (Multiplexion, Heidelberg, Germany). PC17 and PC43 cultures were grown in DMEM Advanced F12 medium supplemented with 0.6% (w/v) glucose, 2 mM L-glutamine, 2% B27 supplement (1 ×) (all Thermo Fisher Scientific), 12 µg/mL heparin and 5 mM HEPES buffer (both Sigma Aldrich), referred to as CSCN medium. Cytokines, *i.e*., 10 ng/mL rhFGF-basic, 20 ng/mL rhFGF-10, and 20 ng/mL rhNodal (all R&D Systems) were added to the cultures and renewed twice a week.

### NK cells

Human peripheral blood mononuclear cells (PBMCs) were isolated by density gradient centrifugation of blood or buffy coats obtained from healthy donors with informed consent (Ethics Vote 118/2021, Ethics Committee of Witten/Herdecke University). Buffy coats were provided by the Deutsches Rotes Kreuz (DRK)-Blutspendedienst in Mannheim, Germany and DRK-Blutspendedienst West, Zentrum fur Transfusionsmedizin Hagen (Hagen, Germany). Leukocyte filters containing PBMCs from whole blood were donated by BZD Gesellschaft f. Transfusionsmedizin Duisburg mbH (Duisburg, Germany).

NK cells were purified via depletion of other immune cell subsets using the human NK Cell Isolation Kit (Miltenyi Biotec) according to the manufacturer's instructions. NK cells were either directly used for experiments, or cultured at 2 × 10⁶ cells/mL in RPMI medium supplemented with 10% human serum (Sigma Aldrich), 50 U/mL penicillin-streptomycin (Thermo Fisher Scientific), and 100 U/mL IL-2 (Proleukin, Clinigen) (pre-stimulated). Viability and purity of isolated NK cells was confirmed by flow cytometry (Zombie Violet⁻, CD45⁺, CD3⁻, CD19⁻, CD14⁻, and CD56⁺).

### Recombinant measles viruses:

### Virus rescue, propagation, and titration

Recombinant MV were generated via transfection of Vero cells with antigenomic cDNA constructs using the established RNA polymerase II-dependent reverse genetics system (Martin *et al,* 2006) as previously described (Heidbuechel & Engeland, 2019). For virus propagation, Vero cells were inoculated at a multiplicity of infection (MOI) of 0.03 in Opti-MEM (Thermo Fisher Scientific), which was diluted with culture medium 2 h post infection. Cultures were incubated at 37°C and 5% CO₂, syncytia formation was monitored, and virus-containing cells were scraped 40-48 h post infection. Viruses were released by one freeze-thaw cycle, clarification was performed (2500 × g, 5 min) and aliquots of cell-free supernatant were stored at -80°C. Infectious units in viral stocks were quantified by ten-fold serial dilution in octuplicates using 96-well plates with 1.5 × 10⁴ Vero cells per well. Individual syncytia were counted 48 and 72 h post infection and titers calculated as cell infectious units (ciu)/mL.

### Viral infection, replication kinetics and cytotoxicity

Cells were seeded one day prior to inoculation at designated MOIs. Virus suspensions were diluted in Opti-MEM, unless indicated otherwise, and medium only was applied for mock infected controls. Inocula were replaced by respective culture media 2 h post infection.

Phase contrast and fluorescence microscopy images were taken with a Leica DMi8 microscope.

For multi-step growth curves, 1 × 10⁵ Vero cells in 12-well plates were inoculated at MOI 0.03 in triplicates, cells were scraped into the culture medium at designated time points, and pooled per condition. After one freeze-thaw cycle, titers of clarified virus suspensions were determined by serial dilution titration in quadruplicates.

Cell viability was assessed by XTT (2,3-Bis-(2-methoxy 4-nitro-5-sulfophenyl)-2H-tetrazolium-5-carboxanilide) assay. Cells were inoculated at MOI 0.03 (Vero, 1 × 10⁵ cells, 12-well plate) or MOI 1 (KM12 and HT-29, 4.5 × 10⁴ cells, 48-well plate) in triplicates. At designated time points post infection, the Colorimetric Cell Viability Kit III (PromoCell) was used according to the manufacturer's protocol. Viability was normalized to mock infected controls at respective time points.

Cell lysis was assessed by LDH release assay. Patient-derived cultures (PC43 and PC17, 1 × 10⁵ cells, 12-well plate) were inoculated at MOI 0.03 and MOI 3 in triplicates, with virus suspensions diluted in CSCN medium. The inoculum was replaced with fresh CSCN medium supplemented with cytokines 2 h post infection. For 100% release samples, mock infected cells were scraped 24 h post inoculation, underwent two freeze-thaw cycles and were stored at -80°C. Culture supernatants were collected at designated time points, centrifuged (380 × g, 5 min) and stored at -80°C. LDH release into the supernatants was quantified using the CytoTox 96 Non-Radioactive Cytotoxicity Assay kit (Promega). Relative cytotoxicity was calculated by normalization to the 100% release control of the respective culture.

### Virus purification via ultracentrifugation

To reduce protein contamination in clarified crude cell lysates, recombinant viruses were purified via iodixanol fractionation ultracentrifugation with a protocol adapted from Liljeroos *et al* (2011). Thinwall UltraClear 5 mL tubes (Beckman Coulter) were disinfected with 70% (v/v) ethanol and 2 × 4 mL virus suspension per construct were loaded onto 500 µL 20% (w/v) OptiPrep (Axis Shield) in TE buffer (5 mM Tris, 1 mM EDTA, pH 7.4), underlayered with 500 µL 54% (w/v) OptiPrep in TE buffer. Tubes were subjected to ultracentrifugation in an SW50.1 rotor (Beckman Coulter) at 28,000 rounds per minute (rpm) for 2 h at 8°C without brake. The interphase containing purified virus was collected, diluted to a total volume of 3 mL in Opti-MEM and stored at -80°C. The upper and lower fractions were kept for analysis.

### MV-encoded BiKE and scFv-Fc proteins:

### Purification of BiKEs from virus infected cells

Virus-encoded BiKEs were purified from supematants of infected Vero cells (vBiKEs). 6 × 10⁶ cells in 15 cm culture dishes were inoculated with respective MV-BiKEs at MOI 0.03 in OptiPRO SFM serum-free medium (Thermo Fisher Scientific), cultured at 37°C, 5% CO₂ and monitored for syncytia formation. Supernatants were collected, clarified (2500 × g, 5 min, 4°C), and passed through a 0.22 µm pore size filter (Merck) before vBiKEs were purified by affinity exchange chromatography via the His₆ tag. Ni-NTA Spin Columns or Superflow Columns (Qiagen) were used for small and large scale purification, respectively, following the manufacturer's instructions. In brief, cell-free supernatants were applied to equilibrated columns allowing BiKE binding to the Ni-NTA resin. Columns were rinsed with wash buffers containing 10 mM and 20 mM imidazole. Immobilized BiKE proteins were recovered with elution buffer containing 500 mM imidazole. Eluates were washed with DPBS, concentrated in Amicon Ultra-15 Centrifugal Filter Units with Ultracel-10 membranes (Merck), and stored at -80°C. Protein concentration in purified vBiKE aliquots was quantified by BCA assay using the Novagen BCA Protein Assay Kit (Merck) according to the manufacturer's instructions.

### Gel electrophoresis and western blot analysis

BiKE content in fractions obtained from the protein purification procedure or virus purification via ultracentrifugation were analyzed by gel electrophoresis with 4-20% TGX Stain Free Gels (Bio-Rad), stain-free imaging and western blot. BiKEs were detected with anti-HA biotin (clone 3F10, 10 ng/µL, Merck), streptavidin-HRP (0.02 µg/mL, Dianova), and Clarity Western ECL Substrate (Bio-Rad). HRP-specific chemiluminescence was acquired using a ChemiDoc MP Imaging System (Bio-Rad).

### Enzyme-linked immunosorbent assay (ELISA) for BiKE quantification

At designated time points post infection, supernatants from triplicate wells were collected and stored at -80°C. Lysates were harvested by scraping into R1PA Buffer (Sigma Aldrich) supplemented with cOmplete protease inhibitor cocktail (Roche), and pooled per condition. Samples were snap-frozen in liquid nitrogen and stored at -80°C. Thawed lysates were centrifuged (2500 × g, 5 min, 4°C) and diluted with DPBS for analysis to compare lysates and supernatants.

In a sandwich ELISA approach, 96-well plates (Nunc MaxiSorp flat-bottom, Thermo Fisher Scientific) were coated with 100 ng anti-His antibody (clone 13/45/31, Dianova) in 100 µL DPBS per well. After blocking, samples were incubated for 2 h at room temperature. Purified vBiKEs were used as standard. BiKEs were detected with anti-HA biotin (clone 3F10, 100 ng/µL, Merck), streptavidin-HRP (0.1 µg/mL, Dianova), and 1-Step Ultra TMP ELISA Substrate (Thermo Fisher Scientific). Absorbance was measured using an Infinite 200 Pro microplate reader (Tecan) with i-control software (v 2.0.10.0).

### BiKE/scFv-Fc binding assay

BiKE and scFv-Fc binding to NK cells or tumor cells was analyzed by flow cytometry. Proteins bound to tumor cells via the anti-TAA scFv were detected with antibodies targeting the C-terminal His₆ tag. Antibodies targeting the N-terminal HA tag were applied to assess binding to NK cells via the anti-CD 16A (BiKE) or IgG1Fc (scFv-Fc) moiety.
1 × 10⁵ cells were incubated with Zombie Violet fixable viability dye (1:5000, BioLegend) for 15 min in the dark. For NK cells, Human TruStain Fc block (1:40, Biolegend) was added. Cells were washed and incubated with purified vBiKEs (2 µL) or MV-BiKE and MV-scFv-Fc virus suspensions (20 µL) for 30 min on ice in the dark. NK cells were stained with anti-CD 16-BV650 (clone 3G8, 1:100, BioLegend) in parallel. After washing, cells were labeled with anti-HA FITC (clone GG8-1F3.3.1, 1:20, Miltenyi Biotec) or anti-His₆ FITC (clone 13/45/31, 1:20, Dianova). Samples were fixed with 1% PFA in DPBS and analyzed by flow cytometry. Unstained and single stained controls were used to control for unspecific antibody binding.

### Functional in vitro coculture assays:

### Target cell killing assay

Killing of tumor cells by IL-2 pre-stimulated NK cells was assessed using a flow cytometry-based cytotoxicity assay (adapted from Kandarian *et al,* 2017). In principle, non-infected target cells were labeled with 5 µM Celltrace CFSE (Thermo Fisher Scientific) according to the manufacturer's instructions, and killing was quantified after co-incubation with NK cells and either vBiKEs or MV-BiKE-infected tumor cells.

To analyze vBiKE efficacy, 4 × 10⁴ CFSE-labeled cells were cocultured with 2 × 10⁵ NK cells and indicated vBiKE concentrations for 12 in TC-treated U bottom plates with 200 µL RPMI supplemented with 10% FCS.

To analyze MV-BiKE efficacy, 4 × 10³ target cells in TC-treated U bottom plates were infected at MOI 1 (KM12) or MOI 5 (HT-29, patient-derived cultures), and medium only for mock controls. Inocula were replaced by RPMI, 10% FCS and cultures were incubated for 48 h (KM12) or 72 h (HT-29, patient-derived cultures). To set up cocultures, 2 × 10⁴ CFSE-labeled, non-infected target cells (bystander cells) and 2 × 10⁵ NK cells were added to the infected cells and incubated for 12 h. Conditions were performed in duplicates and pooled for analysis.

For quantification of target cell death, supernatants were collected and pooled with adherent cells from respective conditions after detachment with 0.05% Trypsin EDTA. Cells were stained with Zombie Violet fixable viability dye (1:2000, BioLegend). After washing and fixation, samples were analyzed by flow cytometry. Unstained and single stained tumor cell controls were included for gating and at least 5000 CFSE⁺ events were recorded. Samples were gated on CFSE⁺ non-infected tumor cells and the percentage of Zombie Violet⁺ dead cells was determined.

NK cell degranulation and cytokine expression were analyzed in parallel to target cell killing. Cocultures were prepared as outlined above, omitting the CFSE-labeling of non-infected target cells. Positive controls were stimulated with 50 nM PMA (Cayman Chemical) and 1 µM ionomycin (Cayman Chemical). To detect degranulation events, anti-CD 107a antibodies were added directly to the cultures. Protein transport inhibitors GolgiStop (1 µL/mL, BD Biosciences) and GolgiPlug (1 µL/1.5 mL, BD Biosciences) containing monensin and brefeldin A, respectively, were added 1 h after setting up the cocultures. After 12 h incubation, cells were harvested. Surface markers (CD45, CD16, CD56) and intracellular cytokines (IFNγ, TNFα) were labeled for analysis by flow cytometry.

### Analysis of NK cell infection with MV

Expression of MV entry receptors was analyzed on freshly isolated NK cells. Dead cells were excluded from the analysis based on Zombie Violet staining (1:5000, Biolegend), cells were stained for CD46, CD150, or Nectin-4, and respective isotype controls. As positive controls, HT-29 tumor cells and PBMCs were included.

NK cell infection and stimulation were analyzed either in coculture with infected tumor cells or upon direct inoculation of NK cells. 2 × 10⁴ tumor cells in TC-treated 96-well U-bottom plates were infected with MV at MOI 1 or mock infected in 100 µL Opti-MEM. MeVac ldEGFP was used to monitor viral (trans)gene expression after infection, and NK cell stimulation was analyzed using MeVac without transgene. After 12h, the inoculum was replaced by 150 µL RPMI, 10% FCS. Cocultures with 1 × 10⁵ NK cells were set up 24 h post infection. For inoculation of NK cells with MV at MOI 0.2 or MOI 1, virus suspension diluted in Opti-MEM was directly added to 1 × 10⁵ NK cells in TC-treated 96-well U-bottom plates. To replace the inoculum with fresh medium, plates were centrifuged at 800 × g for 2 min, supernatants were discarded, and 150 µL RPMI, 10% FCS was added. After incubation for 12 h to 60 h, either EGFP expression in live tumor (CD45⁻) and NK cells (CD45⁺) or CD69 levels on NK cells (CD45⁺, CD56⁺) were analyzed by flow cytometry, respectively.

### NK cell analysis in MV plus vBiKE combination therapy

NK cell stimulation in the context of MV-infection and NK cell effector functions in response to combination treatments with vBiKEs were addressed in coculture assays. 5 × 10³ tumor cells in TC-treated 96-well U-bottom plates were inoculated with MV at MOI 1 or mock infected in 100 µL Opti-MEM. The inoculum was replaced by 150 µL RPMI, 10% FCS. Cocultures with 2 × 10⁵ NK cells were set up 24 h post infection, including IL-2 (1000 IU/mL) stimulated cocultures and NK cell only controls. After 12 h co-incubation, CD69 expression on NK cells was analyzed by flow cytometry.

BiKE combination treatment was started 36 h post infection, *i.e.* after 12 h coculture. To this end, 2 × 10⁵ non-infected target cells and vBiKEs (10 ng/µL) were added to the cocultures, and NK cell degranulation and cytokine expression was assessed. CD107a labeling antibodies, protein transport inhibitors and PMA/ionomycin for positive controls were applied as described above. After 4 h vBiKE treatment, *i.e*. 40 h post infection, cells were harvested. Surface markers (CD45, CD16, CD56) and intracellular cytokines (IFNγ, TNFα) were labeled and NK cells were analyzed by flow cytometry.

For experiments with multiple analyses of CD69 expression at designated time points, cocultures were set up as described above with technical replicates for each time point. NK cells (1 × 10⁵ cells) were added 24 h post infection and analyzed 36 h, 60 h, and 86 h post infection, *i.e*. after 12 h, 36 h, and 60 h in coculture, respectively. BiKE combination treatment was started 60 h post infection and analyzed 4 h later as described above.

### NK cell characterization in MV-BiKE or MV-scFv-Fc therapy

NK cell surface receptors and IFNγ release were analyzed after exposure to MV, MV-BiKE, and MV-scFv-Fc-infected tumor cells, or to MV-infected cells with additional vBiKE treatment. For cocultures, 2 × 10⁴ tumor cells were inoculated at MOI 1, the inoculum was replaced by RPMI, 10% FCS 2 h post infection, and 1 × 10⁵ NK cells were added 24 h post infection. Purified vBiKEs (10 ng/µL) and IL-2 (1000 IU/mL) were added to designated conditions directly after adding NK cells. Analysis was performed 72 h post infection, *i.e*. after 48 h coculture. IFNγ concentrations in coculture supernatants were quantified by ELISA using the IFN gamma Human Uncoated ELISA Kit (Thermo Fisher Scientific) according to the manufacturer's instructions. NK cells were labeled with respective antibodies (panel 1, NKG2D, NKp46, DNAM-1; panel 2, NKG2A, KIR2D, NKp44; panel 3, CD69) and surface marker levels were analyzed by flow cytometry.

### Flow cytometry analysis

To prepare NK cells for flow cytometry analysis, samples were incubated with Zombie Violet fixable viability dye (1:5000, Biolegend) and Human TruStain Fc block (1:40, Biolegend). NK cells were labeled with antibodies specific for CD45, CD16 and CD56. For NK cell purity controls, stimulation and surface receptor analyses, further antibodies were added as indicated in respective sections. Samples were washed and fixed with 1% PFA in DPBS for analysis, or fixed and permeabilized for subsequent intracellular cytokine staining using the Cytofix/Cytoperm Fixation/Permeabilization Kit (BD Biosciences). At least 10,000 live CD45⁺ CD56⁺ NK cell events were recorded.

Surface marker antibodies: CD45-BV510, CD45-APC/Cy7 (clone HI30, 1:200); CD16-BV650, CD16-FITC, CD16-PerCP/Cy5.5 (clone 3G8, 1:100); CD56-APC/Cy7, CD56-PE (clone 5.1H11, 1:200), CD56-FITC (1:50); CD69-BV785, CD69-APC (clone FN50, 1:100); NKG2D-APC (clone 1D11, 1:200); NKp44-APC (clone P44-9, 1:100); NKp46-PE (clone 9E2, 1:200); DNAM-1-PE/Cy7 (clone 11A8, 1:200), CD107a-BV785, CD107a-Alexa Fluor 488 (clone H4A3, 1:200, added during coculture); CD46-APC (clone TRA-2-10, 1:100) (BioLegend); CD3-V450 (clone UCHT-1, 1:100); CD19-V450 (clone HIB19, 1:100); CD14-V450 (clone MϕP9, 1:100); CD150-PE (clone A12, 1:20) (BD Biosciences); NKG2APE-Vio770 (clone REA110, 1:100); KIR2D-PE (clone NKVFS1, 1:100) (Miltenyi Biotec); Nectin-4-Alexa Fluor 488 (clone 337516, 1:50) (R&D Systems).

Intracellular cytokine antibodies: IFNy-Alexa Fluor 647 (clone 4S.B3, 1:100); TNFα-PE, TNFα-BV510 (clone Mab11, 1:100) (BioLegend); IFNy-Alexa Fluor 647 (clone B27, 1:25) (BD Biosciences).

Compensation was calculated based on single stained compensation beads (OneComp eBeads Compensation Beads, Thermo Fisher Scientific; BD CompBeads, BD Biosciences), and gating was based on respective Fluorescence Minus One plus Isotype (FMO-I) controls. NK cell purity was confirmed after isolation, and NK cells were gated as single cell, live CD45⁺ CD56⁺ events for analysis of coculture assays.

Flow cytometry was performed on a FACS Fortessa instrument with FACS Diva software (v 8.0.1, BD Bioscience), or a CytoFLEX flow cytometer with CytExpert software (v 2.3, Beckman Coulter).

### Statistical analysis and data visualization

Flow cytometry data was analyzed and visualized using FlowJo (v 10.8.0, Tree Star Inc.). Statistical analyses were performed using GraphPad Prism software (v 9.1.2, GraphPad Software). Data for target cell death were analyzed by one-way ANOVA comparing selected pairs of means as indicated, and *p* values were adjusted for multiple comparisons by Šidák's test. P values < 0.05 were considered statistically significant.

Schematics in Figure 1A, Figure 2A, Figure 3A, Figure 4A, Figure 5A, Figure S4A, Figure S6A, and the graphical abstract were created with BioRender.com.

### RESULTS

Natural killer cell activity in oncolytic measles virotherapy and combination treatment with bispecific killer engagers:

### NK cell stimulation by virus-infected tumor cells

To assess natural killer (NK) cell activation by virotherapy, NK cells were cocultured with recombinant measles vaccine strain virus (MV)-infected colorectal cancer and pancreatic adenocarcinoma cells and analyzed the early activation marker CD69 to monitor stimulation (Figure 1A). Compared to NK cell only controls and mock treatment, CD69 expression on NK cells was increased after exposure to MV-infected tumor cells (Figure 1B, C). Highest CD69 levels were observed in IL-2 stimulated positive controls. Analysis of MV entry receptors showed CD46 expression on NK cells (Figure S1 A). However, inoculation of NK cells with EGFP-encoding MV did not result in considerable EGFP transgene expression, and low percentages of EGFP⁺ NK cells were detected in cocultures with MeVac ldEGFP-infected tumor cells (Figure SIB, C). In direct comparison, increased CD69 expression was only found after coculture with infected colorectal cancer cell lines, but not after inoculation of NK cells alone (Figure S1D, E), indicating that exposure to infected cells rather than direct NK cell infection elicits stimulation. It is hypothesized that combination treatment with bispecific killer engagers (BiKEs) can enhance NK cell cytotoxicity against malignant cells in the context of virotherapy.

### Validation of BiKE functionality

BiKEs targeting CD16A on NK cells and carcinoembryonic antigen (CEA) as a model tumor antigen were encoded in recombinant MVs. Constructs targeting high molecular weight melanoma associated antigen (HMWMAA) as a control antigen not expressed in CRC and PDAC were generated accordingly. BiKE proteins were purified from supernatants of MV-BiKE-infected Vero producer cells (vBiKE, Figure S2A) and tested for functionality. Specific binding to the targeted tumor antigen and CD16 on NK cells was confirmed by flow cytometry (Figure S2B). Further, binding to CEA-positive CRC and PDAC cells was shown for the CEA-specific BiKE, but not the control construct (Figure S2C). Cocultures of tumor and effector cells were prepared to monitor target cell killing and NK cell effector functions. Target cell death was increased in presence of the relevant vBiKE, demonstrating effective NK cell engagement and hence functionality of vBiKEs (Figure S2D, E). In controls with either non-relevant BiKE protein or without treatment, baseline killing was observed compared to conditions without effector cells. NK cell degranulation (CD107a expression) as well as IFNγ and TNFα cytokine expression were enhanced upon specific BiKE treatment, whereas CD16 surface levels were decreased (Figure S2F, G). Having validated vBiKE functionality, the combination of MV therapy and BiKE treatment was then assessed.

### NK cell effector functions in combination treatment of MV with BiKEs

To address NK cell responses to BiKE engagement after exposure to virus-infected cells, NK cells were cocultured with MV-infected tumor cells and subsequently added vBiKEs as well as non-infected tumor cells (Figure 2A). MeVac treatment alone resulted in a marginal increase of NK cell degranulation and cytokine expression levels compared to mock conditions. NK cell effector functions were strongly elevated upon combination therapy with the tumor-binding CEAxCD16A vBiKE, but not for the non-binding HMWMAAxCD16A vBIKE. NK cell degranulation and cytokine expression levels upon MV plus vBiKE combination treatment exceeded levels observed after respective vBiKE monotherapy (Figure 2B-D). Thus, combining virotherapy and BiKEs is beneficial.

Recombinant MV generated in the laboratory by standard procedures, *i.e*., clarification of infected cell lysate, may contain both viral gene products and other cellular components that stimulate NK cells. In contrast, clinical virotherapy protocols employ highly purified virus products. MV-NIS is such a measles-derived virotherapeutic that is currently investigated in clinical trials (Msaouel *et al,* 2018; NCT03171493; NCT02962167; NCT02068794; NCT02364713; NCT02700230). Therefore, NK cell responses were compared to lab-grade Schwarz (MeVac) or Edmonston (NSe) vaccine strain stocks and highly purified MV-NIS derived from the Edmonston strain.

The stimulation experiments presented in Figure 1 were extended. NK cell coculture with infected tumor cells for 12 h resulted in slightly increased CD69 expression for MeVac compared to mock infection, as shown before, which was however not observed for MV-NIS (Figure S2A). Subsequent coculture with non-infected target cells and vBiKEs elicited high NK cell degranulation for CEAxCD16A vBiKEs as described above. Compared to vBiKE monotherapy (mock), the percentage of CD107a⁺ NK cells was increased upon MeVac, but not MV-NIS infection (Figure S3B). It was thought that different infection kinetics of these viruses might result in varying NK cell responses and hence monitored CD69 levels at three designated time points post inoculation. For HT-29 cocultures, increased CD69 expression compared to mock controls was observed in MeVac, NSe and MV-NIS-infected cocultures after 36 h and 60 h, *i.e*. at 60 h and 84 h post inoculation, respectively (Figure 2E, S3C). Combination therapy starting after a 36 h coculture period again elicited high NK cell degranulation in presence of the target-specific vBiKE. With this timeline, CD107a⁺ NK cells were increased upon MeVac, NSe, and MV-NIS infection compared to vBiKE monotherapy (Figure 2F, G, S2D, E). Similarly, for KM12 target cells, vBiKE treatment after 36 h coculture triggered stronger NK cell degranulation in the MeVac, NSe, or MV-NIS plus CEAxCD16A conditions compared to mock infection, even though no increase in CD69 expression was observed on NK cells exposed to MV-NIS-infected cells at the 36 h coculture time point (Figure S2D, E). Upon coculture with infected cells, increased NK cell degranulation was also observed in controls without BiKE treatment as well as for the non-binding BiKE. In addition, target-specific vBiKE elicited elevated cytokine expression, and IFNγ accumulation was marginally increased upon MV-NIS compared to mock infection (Figure S3F). In conclusion, NK cells strongly respond to BiKE therapy after exposure to infected tumor cells, and this finding extends to clinically relevant MV-NIS, albeit kinetics of stimulation differ between MV variants.

### Therapeutic efficacy of MV-BiKE in vitro

As opposed to oncolytic virotherapy combined with systemic BiKE treatment, co-delivery of BiKE proteins as therapeutic payload of OVs can enable local transgene expression in the tumor microenvironment. Having shown that MeVac and vBiKE administered separately elicit enhanced NK cell effector functions, MV encoding BiKEs were further characterized to realize this concept (Figure 3A). Specifically, live-attenuated MV Schwarz vaccine strain vectors harboring a BiKE open reading frame (MeVac P-BiKE) were generated.

### Characterization of BiKE-encoding MV for oncolytic combination therapy

MV-BiKE replication kinetics were compared to unmodified MV via titration of viral progeny after infection of Vero producer cells (Figure 3B). The direct cytopathic effect of MV-BiKE on Vero cells and human colorectal cancer cell lines was assessed by cell viability assay (Figure 3C). BiKE-encoding viruses were mildly attenuated compared to unmodified control. KM12 cells were found to be more susceptible to MV-mediated cytolysis compared to HT-29. Kinetics of BiKE release into the culture supernatant consequently differed between both cell lines as determined by ELISA, with *de novo* BiKE production confirmed for both. For KM12, the highest BiKE concentrations of approximately 1 ng/µL were measured 48 h post infection with MeVac P-BiKE viruses, whereas the highest concentrations for HT-29 were measured 72 h post infection (Figure 3D). Concentrations of cell-associated BiKE protein in cell lysates showed different kinetics compared to BiKE released into the supernatant, with higher concentrations in lysates already detected 24 h post infection (Figure 3E).

### MV-BiKE mediated NK cell cytotoxicity

The potential of BiKE co-delivery as a therapeutic MV cargo was assessed in coculture assays of human healthy donor NK cells and MV-BiKE-infected tumor cells. To monitor specific cytotoxicity towards bystander cells as the critical target population, killing of non-infected tumor cells was quantified (Figure 4A). Donor-dependent variability of baseline killing was observed in mock-infected controls, and NK cell cytotoxicity was enhanced upon MeVac treatment alone. Infection with MeVac encoding tumor-specific CEAxCD16A BiKE significantly increased the mean KM12 target cell death compared to MeVac and MeVac P-HMWMAAxCD16A controls, respectively (Figure 4B, C). Similar trends were observed for HT-29 target cells. In controls without effector cells, bystander cells were not affected by direct cytotoxicity of different MV-BiKE constructs (Figure S4F). In line with increased tumor cell death, highest NK cell degranulation and cytokine expression were found in cocultures treated with MeVac P-CEAxCD16A (Figure 4D, E). In summary, MV-BiKE effectively mediated NK cell activation and specific cytotoxicity against cancer cells.

As outlined above, virus preparations obtained from clarified crude cell lysate of infected Vero cells contain BiKE protein and other cellular components. Therefore, MV-BiKE via ultracentrifugation was purified to reduce BiKE concentrations in the inoculum. The efficacy of purified viruses was compared to conventional lab-grade stocks (Figure S4A). Reduced concentration of protein per cell infectious unit and lower BiKE content in particular were confirmed via western blot and BiKE binding assay (Figure S4B, C). BiKE concentrations in supernatants and lysates from infected tumor cells were found to be similar for purified and lab-grade viruses (Figure S4D). Purified MV-BiKE efficiently and triggered specific target cell killing, NK cell degranulation and cytokine accumulation, comparable to conventional MV-BiKE stocks (Figure S4 E-H). This indicates that *de novo* BiKE production from virus-infected cells occurs and mediates MV-BiKE efficacy.

### Characterization of NK cells in response to MV-BiKE

Given the functionality and efficacy of MV-BiKE, NK cells in response to measles virotherapy and MV-BiKE treatment was characterized in more detail. In addition, MV-BiKEs were compared to the combination of MV and vBiKEs administered separately. Cytokine release, CD69 and activating as well as inhibitory NK cell receptors were analyzed after 48 h exposure to infected cells MeVac P-CEAxCD16A infection elicited increased IFNγ levels compared to MeVac and MeVac P-HMWMAAxCD16A controls in HT-29 cocultures (Figure 5B, S5A), in line with enhanced NK cell cytokine expression in MV-BiKE efficacy experiments (compare Figure 4E). Further, MV infection of HT-29 combined with tumor-binding CEAxCD16A vBiKE treatment resulted in higher IFNγ concentrations compared to the non-binding control. For KM12, IFNγ concentrations were close to the detection limit in all conditions apart from the IL-2 stimulated control. The CD69 activation marker was increased on NK cells in all MV-infected conditions (Figure 5C(i), S5B(i)), indicating MV-mediated NK cell stimulation as described above (compare Figures 1, 2. and S1). CD16 Fc receptor levels were decreased both for MV-BiKE and MV + vBiKE treatment, which was most pronounced for conditions with tumor-specific, CEA-targeting BiKEs (Figure 5C(ii), S5B(ii)). Surface expression of the adhesion molecule and activating receptor DNAM-1 was decreased upon MV infection, with strongest reductions found in CEAxCD16A conditions (Figure 5C(iii), S5B(iii)). The NKG2A inhibitory NK cell receptor appeared to be mildly increased in coculture with MV-infected tumor cells compared to mock. CEAxCD16A-encoding viruses or combination therapy with CEAxCD16A vBiKEs were also found to mildly increase NKG2A compared to respective HMWMAA-targeting controls (Figure 5C(iv), S5B(iv)). No consistent effects were observed for the activating receptors NKG2D, NKp46, and NKp44, or the killer immunoglobulin-like receptor (KIR) 2D subtype including activating and inhibitory isoforms (Figure 5C(v-viii), S5B(v-viii)).

### NK cell engagement with scFv-Fc constructs as alternative MV transgene

MVs provide a flexible platform to deliver therapeutic transgenes, so that different payloads can be tested to optimize NK cell stimulation and tumor-directed cytotoxicity. In addition to MV-BiKE, analogous MVs encoding scFv-Fc proteins (MV-scFv-Fc, Figure S6A) were generated. These engagers are composed of an IgG1Fc portion fused to the tumor-targeting scFv, resulting in conventional Fc receptor binding on NK cells and other immune cell subsets such as macrophages. Binding of virus-encoded scFv-Fcs to CEA-expressing tumor and CD16⁺ NK cells was confirmed by flow cytometry, with high signals detected on NK cells compared to MV-BiKE, presumably resulting from the dimeric structure of functional scFv-Fc (Figure S6B). Further, binding to *in vitro* differentiated macrophages was confirmed (data not shown).

In coculture assays, MeVac P-CEAxIgG1Fc infection led to increased bystander target cell death compared to controls with non-relevant scFv-Fc transgene and mock infection (Figure S6C, D). Compared to respective MV-BiKEs, no consistent difference in therapeutic efficacy was observed across two cell lines tested. Infection with relevant MV-scFv-Fc further resulted in increased NK cell degranulation and cytokine expression, particularly on HT-29 target cells (Figure S6E, F). Of note, enhanced NK cell degranulation and IFNγ accumulation upon infection with MV encoding non-relevant HMWMAA-scFv-Fc compared to unmodified MV did not correlate with increased HT-29 target cell death. Characterization of NK cell surface markers in response to MV-scFv-Fc therapy revealed high levels of CD69 in all MV-infected conditions compared to mock, with highest NK cell stimulation in the MeVac P-CEAxIgG1Fc condition. CD16 surface levels were strongly reduced upon exposure to MV-scFv-Fc-infected tumor cells, which was most pronounced for the tumor-binding, CEA-targeting transgene. In contrast to BiKEs, binding of scFv-Fc proteins to NK cells was however found to interfere with subsequent CD16 detection by flow cytometry to a limited extent. The percentages of NKG2A⁺ NK cells were slightly increased and DNAM-1 levels decreased upon MeVac P-CEAxIgG1Fc infection compared to controls. Changes in NK cell surface marker expression hence showed similar trends as observed for BiKE-encoding viruses (compare Figure 5C). Taken together, MV-scFv-Fcs are functional, and *in vitro* efficacy was confirmed to be similar to MV-BiKE in terms of NK cell engagement.

### MV-BiKE efficacy against patient-derived pancreatic cancer cultures

Having demonstrated MV-BiKE efficacy in cell lines, patient-derived pancreatic cancer short-term cultures were used to study our therapeutic approach in a more disease-relevant model (Ehrenberg *et al,* 2019). Among seven cultures screened, two cultures were identified, PC43 and PC17, with strong CEA-specific BiKE binding (Figure 6A). Co-incubation with allogenic healthy donor NK cells resulted in considerable baseline killing, which was enhanced in presence of specific vBiKEs (Figure 6B, C). The cultures showed intermediate to low sensitivity towards MV infection, whereas PC43 was found to be more sensitive than PC17 (Figure S5). In the next step, efficacy of MV-BiKE infection to elicit BiKE-mediated NK cell cytotoxicity towards bystander cells was investigated (compare Figure 4A). For both cultures, NK cell degranulation and slightly increased IFNγ expression were detected for MeVac P-CEAxCD16A compared to controls (Figure 6D, E). However, all infected conditions resulted in similarly enhanced tumor cell killing compared to mock control for PC17, whereas increased target cell death and hence therapeutic efficacy of MV-BiKE against bystander cells was observed for PC43 (Figure 6F, G). Hence, MV-BiKE treatment was effective against PC43, the more permissive culture.

The foregoing experimental results demonstrate that BiKEs can redirect NK cells to tumor cells such that the use of BiKEs along with an oncolytic virus such as measles virus strengthens cytotoxicity against malignant cells, including non-infected bystander cells, and tilts the balance towards anti-tumoral activity by triggering immunogenic cell death and local inflammation.

Treatment of colorectal cancer cell lines with MV-BiKE elicited potent NK cell cytotoxicity against bystander cells, accompanied by NK cell degranulation and increased cytokine expression. Compared to separate administration of MV and BiKE, MV-BiKE yields localized, intra-tumoral BiKE accumulation and reduced systemic exposure. Indeed, BiKE production from infected tumor cells *in vitro* was confirmed.

### SEQUENCE LISTING

SEQ ID NO:1 amino acid sequence of CD16a
SEQ ID NO:2 amino acid sequence of CEA
SEQ ID NO:3 amino acid sequence of BiKE CD16a/CEA
SEQ ID NO:4 nucleotide sequence of BiKE CD16a/CEA
SEQ ID NO:5 nucleotide sequence of MV-BiKE CD16a/CEA
SEQ ID NO:6 HMA linker
SEQ ID NO:7 SM3E V_{H}
SEQ ID NO:8 SM3E V_{L}

### References

Bajic D, Chester K & Neri D (2020) An Antibody-Tumor Necrosis Factor Fusion Protein that Synergizes with Oxaliplatin for Treatment of Colorectal Cancer. Mol Cancer Ther 19: 2554-2563
Bartlett NL, Herrera AF, Domingo-Domenech E, Mehta A, Forero-Torres A, Garcia-Sanz R, Armand P, Devata S, Izquierdo AR, Lossos IS, et al (2020) A phase 1b study of AFM13 in combination with pembrolizumab in patients with relapsed or refractory Hodgkin lymphoma. Blood 136: 2401-2409
Ehrenberg KR, Gao J, Oppel F, Frank S, Kang N, Dieter SM, Herbst F, Möhrmann L, Dubash TD, Schulz ER, et al (2019) Systematic Generation of Patient-Derived Tumor Models in Pancreatic Cancer. Cells 8: E142
Engeland CE, Grossardt C, Veinalde R, Bossow S, Lutz D, Kaufmann JK, Shevchenko I, Umansky V, Nettelbeck DM, Weichert W, et al (2014) CTLA-4 and PD-L1 checkpoint blockade enhances oncolytic measles virus therapy. Mol Ther 22: 1949-1959
Graff CP, Chester K, Begent R & Wittrup KD (2004) Directed evolution of an anti-carcinoembryonic antigen scFv with a 4-day monovalent dissociation half-time at 37°C. Protein Engineering, Design and Selection 17: 293-304
Grossardt C, Engeland CE, Bossow S, Halama N, Zaoui K, Leber MF, Springfeld C, Jaeger D, von Kalle C & Ungerechts G (2013) Granulocyte-macrophage colony-stimulating factor-armed oncolytic measles virus is an effective therapeutic cancer vaccine. Hum Gene Ther 24: 644-654
Heidbuechel JPW & Engeland CE (2019) Paramyxoviruses for Tumor-targeted Immunomodulation: Design and Evaluation Ex Vivo. JoVE (Journal of Visualized Experiments): e58651
Kandarian F, Sunga GM, Arango-Saenz D & Rossetti M (2017) A Flow Cytometry-Based Cytotoxicity Assay for the Assessment of Human NK Cell Activity. JoVE (Journal of Visualized Experiments): e56191
Kang N, Hamilton S, Odili J, Wilson G & Kupsch J (2000) In Vivo Targeting of Malignant Melanoma by 125Iodine- and 99mTechnetium-labeled Single-chain Fv Fragments against High Molecular Weight Melanoma-associated Antigen. Clin Cancer Res 6: 4921-4931
Kaufmann JK, Bossow S, Grossardt C, Sawall S, Kupsch J, Erbs P, Hassel JC, von Kalle C, Enk AH, Nettelbeck DM, et al (2013) Chemovirotherapy of Malignant Melanoma with a Targeted and Armed Oncolytic Measles Virus. Journal of Investigative Dermatology 133: 1034-1042
Liljeroos L, Huiskonen JT, Ora A, Susi P & Butcher SJ (2011) Electron cryotomography of measles virus reveals how matrix protein coats the ribonucleocapsid within intact virions. PNAS 108: 18085-18090
Martin A, Staeheli P & Schneider U (2006) RNA polymerase II-controlled expression of antigenomic RNA enhances the rescue efficacies of two different members of the Mononegavirales independently of the site of viral genome replication. J Virol 80: 5708-5715
Msaouel P, Opyrchal M, Dispenzieri A, Peng KW, Federspiel MJ, Russell SJ & Galanis E (2018) Clinical Trials with Oncolytic Measles Virus: Current Status and Future Prospects. Curr Cancer Drug Targets 18: 177-187
Reusch U, Burkhardt C, Fucek I, Le Gall F, Le Gall M, Hoffmann K, Knackmuss SH, Kiprijanov S, Little M & Zhukovsky EA (2014) A novel tetravalent bispecific TandAb (CD30/CD16A) efficiently recruits NK cells for the lysis of CD30+ tumor cells. MAbs 6: 727-738
Veinalde R, Grossardt C, Hartmann L, Bourgeois-Daigneault M-C, Bell JC, Jäger D, von Kalle C, Ungerechts G & Engeland CE (2017) Oncolytic measles virus encoding interleukin-12 mediates potent antitumor effects through T cell activation. Oncoimmunology 6: e1285992

## Claims

1. A bispecific killer engager (BiKE) molecule comprising at least a first binding domain and a second binding domain, wherein the first binding domain is capable of binding to CD16a and wherein the second binding domain is capable of binding to a tumor antigen.

2. The BiKE molecule according to claim 1, wherein the tumor antigen is selected from the group consisting of androgen receptor (AR), BCL-1, calprotectin, carcinoembryonic antigen (CEA), EGFRs, epithelial cell adhesion molecule (Ep-CAM), epithelial sialomucin, membrane estrogen receptors (mER), FAP, HER2/neu, human high molecular weight melanoma-associated antigen (HMW-MAA), IL-6, MOC-1, MOC-21, MOC-52, melan-A/MART-1, melanoma-associated antigen, mucin, OKT9, progesterone receptor (PGR), prostate specific antigen (PSA), prostate stem cell antigen (PSCA), prostate-specific membrane antigen (PSMA), symaptophysin, VEGFRs, CD19, CD20, CD22, CD30 and CD33, preferably wherein the second binding domain is capable of binding to the tumor antigen CEA.

3. The BiKE molecule according to claim 1 or 2, wherein the first binding domain and the second binding domain are separated by a linker.

4. The BiKE molecule according to any one of claims 1 to 3, wherein the first binding domain and/or the second binding domain is a single chain antibody (scFv), preferably wherein the scFv is a humanized scFv.

5. The BiKE molecule according to any one of claims 1 to 4, comprising the amino acid sequence depicted in SEQ IN NO:3

6. A nucleic acid comprising a nucleotide sequence encoding SEQ ID NO:3

7. The nucleic acid according to claim 6 comprising the nucleotide sequence depicted in SEQ ID NO:4

8. A recombinant virus of the family *Paramyxoviridae,* comprising at least one expressible polynucleotide encoding a bispecific killer engager (BiKE) molecule, said molecule comprising at least a first binding domain and a second binding domain, wherein the first binding domain is capable of binding to CD16a and wherein the second binding domain is capable of binding to a tumor antigen.

9. The recombinant virus of the family *Paramyxoviridae* according to claim 8, wherein the tumor antigen is selected from the group consisting of androgen receptor (AR), BCL-1, calprotectin, carcinoembryonic antigen (CEA), EGFRs, epithelial cell adhesion molecule (Ep-CAM), epithelial sialomucin, membrane estrogen receptors (mER), FAP, HER2/neu, human high molecular weight melanoma-associated antigen (HMW-MAA), IL-6, MOC-1, MOC-21, MOC-52, melan-A/MART-1, melanoma-associated antigen, mucin, OKT9, progesterone receptor (PGR), prostate specific antigen (PSA), prostate stem cell antigen (PSCA), prostate-specific membrane antigen (PSMA), symaptophysin, VEGFRs, CD19, CD20, CD22, CD30 and CD33, preferably wherein the second binding domain is capable of binding to the tumor antigen CEA.

10. The recombinant virus of the family *Paramyxoviridae* according to claim 8 or 9, wherein the first binding domain and the second binding domain are separated by a linker.

11. The recombinant virus of the family *Paramyxoviridae* according to any one of claims 8 to 10, wherein the recombinant virus is a recombinant Morbillivirus virus, preferably wherein the recombinant virus is a recombinant measles virus (MV).

12. The recombinant virus of the family *Paramyxoviridae* according to any one of claims 8 to 11, wherein said expressible polynucleotide encoding the BiKE molecule further comprises a nucleotide sequence encoding a cytokine and/or wherein said expressible polynucleotide encoding the BiKE molecule further comprises a nucleotide sequence encoding a signal peptide for secretion operably linked to the BiKE molecule.

13. A polynucleotide encoding the recombinant virus of the family *Paramyxoviridae* according to any one of claims 8 to 12, preferably comprising the nucleotide sequence depicted in SEQ ID NO:5.

14. A BiKE molecule according to any one of claims 1 to 7 or a nucleic acid encoding the BiKE molecule for use in a method for promoting anti-tumor cytotoxicity in a patient or for treating cancer in a patient, the method comprising administering the BiKE molecule or nucleic acid encoding the BiKE molecule to the patient in combination with administering a virus of the family *Paramyxoviridae* to the patient, wherein the BiKE molecule is administered prior to, simultaneously or after administration of the virus.

15. A recombinant virus of the family *Paramyxoviridae* according to any one of claims 8 to 13 or a nucleic acid encoding the recombinant virus for use in a method for promoting anti-tumor cytotoxicity in a patient or for treating cancer in a patient, the method comprising administering the recombinant virus or nucleic acid encoding the recombinant virus to the patient.
